(19) Europäisches Patentamt
European Patent Office
Office européen des brevets

(11) Veröffentlichungsnummer: **0 252 885**
**A1**

(12) **EUROPÄISCHE PATENTANMELDUNG**

(21) Anmeldenummer: **87810386.0**

(22) Anmeldetag: **06.07.87**

(51) Int. Cl.4: **C 07 D 499/00**
A 61 K 31/43
// C07F9/65, C07F7/18,
C07D205/08, C07D403/12

(30) Priorität: **10.07.86 CH 2787/86**

(43) Veröffentlichungstag der Anmeldung:
**13.01.88 Patentblatt 88/02**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Capraro, Hans-Georg, Dr.**
**Habsburgerstrasse 60**
**CH-4310 Rheinfelden (CH)**

**Kohler, Boris, Dr.**
**Holzmattstrasse 45**
**CH-4102 Binningen (CH)**

**Schneider, Peter, Dr.**
**Bäumliackerstrasse 8**
**CH-4103 Bottmingen (CH)**

Patentansprüche für folgenden Vertragsstaaten: AT + ES + GR.

(54) **Bicyclische Thiaazaverbindungen.**

(57) Verbindungen der Formel

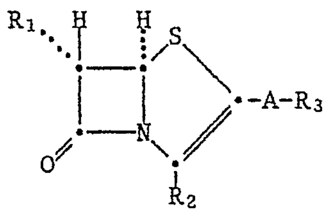

(I),

worin $R_1$ durch Hydroxy oder geschütztes Hydroxy substituiertes Niederalkyl ist, $R_2$ Carboxyl oder funktionell abgewandeltes Carboxyl bedeutet, A für Niederalkylen steht und $R_3$ einen über ein Ringkohlenstoffatom gebundenen, gegebenenfalls partiell ungesättigten Lactamylrest oder Lactonylrest darstellt, optische Isomere von Verbindungen der Formel I, Mischungen dieser optischen Isomere und Salze von Verbindungen der Formel I, die eine salzbildende Gruppe aufweisen, haben antibiotische Wirksamkeit. Die Verbindungen der Formel I werden nach an sich bekannten Verfahren hergestellt.

EP 0 252 885 A1

**Beschreibung**

Bicyclische Thiaazaverbindungen

Die Erfindung betrifft 2-Penem-3-carbonsäure-verbindungen der Formel

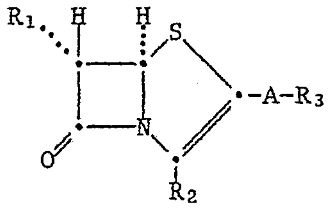

(I),

worin $R_1$ durch Hydroxy oder geschütztes Hydroxy substituiertes Niederalkyl ist, $R_2$ Carboxyl oder funktionell abgewandeltes Carboxyl bedeutet, A für Niederalkylen steht und $R_3$ einen über ein Ringkohlenstoffatom gebundenen, gegebenenfalls partiell ungesätttigten Lactamylrest oder Lactonylrest darstellt, optische Isomere von Verbindungen der Formel I, Mischungen dieser optischen Isomere, Salze von Verbindungen der Formel I, die eine salzbildende Gruppe aufweisen, Verfahren zur Herstellung von Verbindungen der Formel I, pharmazeutische Präparate, die solche Verbindungen enthalten, und ihre Verwendung zur Herstellung von pharmazeutischen Präparaten oder als pharmakologisch wirksame Verbindungen.

Die vor- und nachstehend verwendeten Definitionen haben im Rahmen der vorliegenden Beschreibung die folgenden Bedeutungen:

Funktionell abgewandeltes Carboxyl $R_2$ ist insbesondere unter physiologischen Bedingungen spaltbares verestertes Carboxyl oder geschütztes Carboxyl $R_2'$.

Unter physiologischen Bedingungen spaltbare (d.h. metabolisierbare) veresterte Carboxylgruppen $R_2$ sind aus der Cephalosporin-, Penicillin- und Penemchemie bekannt. Geeignete Gruppen sind in erster Linie Acyloxymethoxycarbonylgruppen, worin Acyl z.B. de Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere $\alpha$-Aminoniederalkanoyloxymethoxycarbonyl, und 4-Crotonolactonyl. Weitere unter physiologischen Bedingungen spaltbare veresterte Carboxylgruppen $R_2$ sind z.B. 5-Indanyloxycarbonyl, Phthalidyloxycarbonyl, 1-Niederalkoxycarbonyloxyniederalkoxycarbonyl, 1-Niederalkoxyniederalkoxycarbonyl oder auch 2-Oxo-1,3-dioxolen-4-ylmethoxycarbonyl, welches in 5-Stellung des Dioxolenringes gegebenenfalls durch Niederalkyl oder Phenyl substituiert ist.

Ein über ein Ringkohlenstoffatom gebundener, gegebenenfalls partiell ungesättigter Lactamylrest oder Lactonylrest $R_3$ ist insbesondere ein monocyclischer 4-bis 6-gliedriger Lactamylrest oder Lactonylrest, in erster Linie der entsprechende Rest einer $\omega$-Amino- bzw. $\omega$-Hydroxy-($C_3$-$C_5$)-niederalkancarbonsäure oder einer $\omega$-Amino-bzw. $\omega$-Hydroxy-($C_4$-$C_5$)-niederalkencarbonsäure, oder ein bicylclischer 5- oder 6-gliedriger Lactamylrest oder Lactonylrest, in erster Linie ein entsprechender von einer $\omega$-Amino- bzw. $\omega$-Hydroxy-($C_4$-$C_5$-niederalkencarbonsäure abgeleiteter Benzolactamylrest oder Benzolactonylrest. Solche Reste $R_3$ sind unsubstituiert oder können z.B. durch gegebenenfalls veräthertes oder verestertes Hydroxy, z.B. Hydroxy, Niederalkoxy, Niederalkanoyloxy oder Halogen, gegebenenfalls veräthertes Mercapto, z.B. Mercapto oder Niederalkylthio, Niederalkyl, durch Hydroxy, verestertes Hydroxy, wie Halogen oder Niederalkanoyloxy, Amino, Niederalkylamine, Diniederalkylamino, Niederalkanoylamino, Carboxyl, Niederalkoxycarbonyl, Cyano, Carbamoyl oder Carbamoyloxy substituiertes Niederalkyl, Niederalkenyl, gegebenenfalls substituiertes Amino, z.B. Amino, Niederalkylamino, Diniederalkylamino der Acylamino, wie Niederalkanoylamino, Carboxyl, verestertes Carboxyl, wie Niederalkoxycarbonyl, Cyano und/oder gegebenenfalls z.B. durch Niederalkyl, Hydroxy, Niederalkoxy, Halogen und/oder Nitro substituiertes Phenyl mono- oder polysubstituiert, in erster Linie mono- oder disubstituiert sein.

Bevorzugte Reste $R_3$ sind solche der Formel

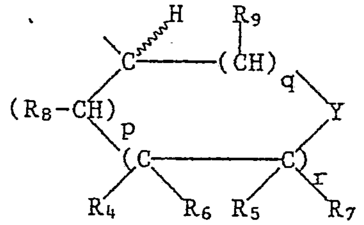

(II),

worin $R_4$, $R_5$, $R_6$ und $R_7$ unabhängig voneinander für Wasserstoff oder für einen der oben als Substituenten von $R_3$ genannten Reste stehen, oder worin $R_4$ gemeinsam mit $R_5$ eine Bindung darstellt

und $R_6$ und $R_7$ unabhängig voneinander für Wasserstoff oder für einen der oben als Substituenten von $R_3$ genannten Reste stehen oder gemeinsam für einen gegebenenfalls durch Hydroxy, Niederalkoxy, Niederalkyl, Halogen und/oder Nitro substituierten 1,4-Butadien-1,4-ylen-Rest stehen, $R_8$ und $R_9$ unabhängig voneinander für Wasserstoff oder für einen der oben als Substituenten von $R_3$ genannten Reste stehen, Y ein Rest der Formel -O-C(=O)- oder $-NR_{10}$ -C(=O)- ist, worin $R_{10}$ für Wasserstoff, Niederalkyl, für einen der oben als Substituenten von $R_3$ genannten substituierten Niederalkyl-Reste, insbesondere durch Amino, Hydroxy, Carboxy oder Cyano substituiertes Niederalkyl, oder für Niederalkenyl steht, r 0 oder 1 ist und p und 9 unabhängig voneinander für eine ganze Zahl von 0 bis 3 stehen, mit der Massgabe, dass wenn r 0 ist, die Summe von p und q 1,2, oder 3 ist, oder wenn r 1 ist, die Summe von p und q 0 oder 1 ist, und mit der weiteren Massgabe, dass das dem Lacton-Ringsauerstoffatom (-O-) bzw. dem Lactam-Ringstickstoffatom ($-NR_{10}$ -) benachbarte Kohlenstoffatom keinen Substituenten aus der Gruppe gegebenenfalls veräthertes oder verestertes Hydroxy, gegebenenfalls veräthertes Mercapto und gegebenenfalls substituiertes Amino trägt.

Entsprechende monocyclische 4-, 5- oder 6-gliedrige gesättigte Lactamyl-Reste $R_3$ können auch als 2-Oxo-azetidinyl, 2-Oxo-pyrrolidinyl und 2-Oxo-piperidinyl, entsprechende monocylische 5-oder 6-gliedrige ungesättigte Reste demgemäss auch als 2-Oxo-dihydropyrrolyl oder 2-Oxo-tetrahydropyridyl bezeichnet werden. Benzoderivate solcher 5- bzw. 6-gliedriger Lactamylreste sind dementsprechend z.B. 2-Oxo-benzo-dihydropyrrolyl oder 2-Oxo-benzotetrahydropyridyl. Monocyclische 4- bis 6-gliedrige, gesättigte Lactonyl-Reste $R_3$ können demgemäss auch als 2-Oxo-oxetanyl, 2-Oxotetrahydrofuryl oder 2-Oxo-tetrahydropyranyl und entsprechende 5-oder 6-gliedrige ungesättigte Reste auch als 2-Oxo-dihyrofuryl bzw. 2-Oxo-dihydropyranyl bezeichnet werden. Bezonderivate solcher 5-bzw. 6-gliedriger Lactonylreste sind dementsprechend z.B. 2-Oxo-benzo-dihydrofuryl und 2-Oxo-benzo-dihydropyranyl. In Resten $R_3$ , die eine Kohlenstoff-Kohlenstoff-Doppelbindung oder einen annelierten Benzoring enthalten, ist die Doppelbindung bzw. der Benzoring vorzugsweise $\alpha,\beta$-ständig zur Lactam- bzw. Lactam-Carbonylgruppe angeordnet.

Die genannten Reste sind über das zur Lactam- bzw. Lactoncarbonyl-gruppe oder insbesondere über das zum Lactam-Ringstickstoffatom bzw. Lacton-Ringsauerstoffatom $\alpha$-ständige Ringkohlenstoffatom, bei 5- und 6-gliedrigen Lactonyl- und Lactamylresten ferner auch über das entsprechende $\beta$-ständige Ringkohlenstoffatom mit dem Rest A verknüpft.

In der vorliegenden Beschreibung bedeutet der im Zusammenhang mit Definitionen von Gruppen bzw. Verbindungen verwendete Ausdruck "nieder", dass die entsprechenden Gruppen bzw. Verbindungen, sofern nicht ausdrücklich anders definiert, 1 bis 7, bevorzugt 1 bis 4, Kohlenstoffatome enthalten.

Durch Hydroxy substituiertes Niederalkyl $R_1$ ist insbesondere in $\alpha$-Stellung zum Penem-Ringgerüst durch Hydroxy substituiertes Niederalkyl und bedeutet z.B. 1-Hydroxyprop-1-yl, 2-Hydroxyprop-2-yl, 1-Hydroxybut-1-yl, 2-Hydroxybut-2-yl und inbesondere Hydroxymethyl und 1-Hydroxyäthyl.

Niederalkanoyloxymethoxycarbonyl ist z.B. Acetoxymethoxycarbonyl oder Pivaloyloxymethoxycarbonyl.

$\alpha$ -Aminoniederalkanoyloxymethoxycarbonyl ist, z.B. Glycyloxymethoxycarbonyl, Valyloxymethoxycarbonyl oder Leucyloxymethoxycarbonyl.

1-Niederalkoxycarbonyloxyniederaloxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolenringes gegebenenfalls durch Niederalkyl oder Phenyl substituiert ist, handelt es sich vor allem um eine 5-Phenyl- und in erster Linie um einen 5-Methyl-2-oxo-1,3-dioxolen-4-ylmethoxy-Gruppe.

Niederalkoxy ist z.B. Methoxy oder Aethoxy, ferner auch n-Propyloxy, Isopropyloxy, n-Butyloxy, Isobutyloxy oder tert.-Butyloxy, sowie n-Pentyloxy, n-Hexyloxy oder n-Heptyloxy.

Niederalkanoyloxy ist z.B Formyloxy, Acetyloxy oder Propionyloxy.

Halogen ist z.B. Fluor, Chlor, Brom oder Jod.

Niederalkylthio ist z.B. Methylthio, Aethylthio, n-Propylthio, lospropylthio oder n-Butylthio.

Niederalkyl ist z.B. n-Propyl, Isopropyl, n-Butyl, Isobutyl, sek.-Butyl oder tert.-Butyl, ferner n-Pentyl, n-Hexyl oder n-Heptyl und insbesondere Methyl oder Aethyl.

Niederalkylamino ist z.B. Methylamino, Aethylamino, n-Propylamino, Isopropylamino oder n-Butylamino, während Dinierderalkylamino z.B. Dimethylamino, Diäthylamino, Di-n-propylamino oder Diisopropylamino bedeutet.

Niederalkanoylamino ist z.B. Acetylamino oder Propionylamino.

Niederalkoxycarbonyl ist z.B. Methoxycarbonyl oder Aethoxycarbonyl.

Niederalkenyl hat z.B. 2 bis 4 Kohlenstoffatome und ist z.B. Vinyl oder Allyl.

Niederalkenyl A ist geradkettiges Niederalkylen, z.B. Methylen, 1,2-Aethylen, 1,3-Propylen oder 1,4-Butylen, oder verzweigtkettiges Niederalkylen, wie durch Niederalkyl, insbesondere Methyl oder Aethyl, mono- oder disubstituiertes geradkettiges Niederalkylen, z.B. Aethyliden ("Methyl-methylen"), 1,2-Propylen, 1,2-Butylen, 1,3-Butylen oder 2-Methyl-1,2-propylen.

Bevorzugt als Rest $R_1$ sind Hydroxymethyl und in erster Linie 1-Hydroxyäthyl.

Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxy-niederalkoxycarbonyl, z.B. 1-Aethoxycarbonyloxyäthoxycarbonyl.

Bevorzugte Lactamyl-Reste $R_3$ sind solche der Formel II (Y steht für die Gruppe $-NR_{10}$ -C(=O)-), worin $R_{10}$ Wasserstoff oder Niederalkyl ist, $R_4$ und $R_6$ Wasserstoff oder gemeinsam eine Bindung darstellen,

$R_6$ und $R_7$ unabhängig voneinander für Wasserstoff, Niederalkyl, durch Hydroxy, Halogen, Niederalkanoyloxy, Amino, Niederalkyamino, Diniederalkylamino, Niederalkanoylamino oder Carbamoyloxy substituiertes Niederalkyl oder gegebenenfalls durch Niederalkyl, Hydroxy, Niederalkoxy und/oder Halogen substituiertes Phenyl stehen, $R_8$ und $R_9$ unabhängig voneinander für Wasserstoff oder Niederalkyl stehen, r 0 oder 1 ist und p und q unabhängig voneinander für eine ganze Zahl von 0 bis 3 stehen, mit der Massgabe, dass wenn r 0 ist, die Summe von p und q 1, oder 3 ist, oder wenn r 1 ist, die Summe von p und q 0 oder 1 ist, und mit der weiteren Massgabe, dass das dem Lactam-Ringstickstoffatom benachbarte Kohlenstoffatom in den Resten der Formel II keinen Substituenten aus der Gruppe Hydroxy, Halogen, Amino, Niederalkylamino, Diniederalkylamino und Niederalkanoylamino trägt. Solche Lactamyl-Reste $R_3$ sind beispielsweise 4-Oxo-azetidin-2-yl, 5-Oxo-pyrrolidin-2-yl, -3-yl oder -4-yl und 6-Oxo-piperidin-2-yl oder -5-yl, wobei diese Reste unsubstituiert oder, wie oben angegeben, substituiert, wie insbesondere am Lactam-Ringstickstoffatom durch Niederalkyl, z.B. Methyl, substituiert sind.

Bevorzugte Lactonyl-Reste $R_3$ sind solche der Formel II (Y steht für die Gruppe -O-C(=O)-), worin $R_4$ und $R_5$ Wasserstoff bedeuten oder gemeinsam eine Bindung darstellen, $R_6$ und $R_7$ unabhängig voneinander für Wasserstoff, Niederalkyl, durch Hydroxy, Halogen, Nideralkanoyloxy, Amino, Niederalkylamino, Diniederalkylamino, Niederalkanoylamino oder Carbamoyloxy substituiertes Niederalkyl, Hydroxy, Halogen, Amino, Niederalkylamino, Diniederalkylamino, Niederalkanoylamino oder gegebenenfalls durch Niederalkyl, Hydroxy, Niederalkoxy und/oder Halogen substituiertes Phenyl stehen oder $R_4$ und $R_5$ gemeinsam eine Bindung darstellen und $R_6$ und $R_7$ gemeinsam für einen gegebenenfalls durch Hydroxy, Niederalkoxy, Niederalkyl und/oder Halogen substituierten 1,4-Butadien-1,4-ylen-Rest stehen, $R_8$ und $R_9$ unabhängig voneinander Wasserstoff, Niederalkyl, Amino oder Hydroxy bedeuten, r 0 ist, die Summe von p und q 2 oder 3 ist, oder r 1, q 0 und p 0 oder 1 ist, mit der Massgabe, dass das dem Lacton-Ringsauerstoffatom benachbarte Kohlenstoffatom in den Resten der Formel II keinen Substituenten aus der Gruppe Hydroxy, Halogen, Amino, Nideralkylamino, Diniederalkylamino und Niederalkanoylamino trägt. Solche Lactonyl-Reste $R_3$ sind insbesondere unsubstituiertes oder, wie oben angegeben, substituiertes 5-Oxo-tetrahydrofuran-2-yl, -3-yl oder -4-yl, 2,5-Dihydro-5-oxo-furan-2-yl und 3-Oxo-phthalan-1-yl.

In bevorzugten Verbindungen der Formel I steht A für Methylen, Aethylen oder 1,2-Propylen.

Die in den Verbindungen der Formel I vorhandenen funktionellen Gruppen, wie Hydroxy-, Carboxy- oder Aminogruppen, insbesondere die Hydroxygruppe im Rest $R_1$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Grupppen vor unerwünschten Kondensationsreaktionen, Substitutionsreaktionen und dergleichen während der Synthese der Verbindung der Formel I aus ihren Vorstufen. Solche Schutzgruppen sind leicht, d.h. ohne dass unerwünschte Nebenreaktionen stattfinden, beispielsweise solvolytisch oder reduktiv, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemi", Band 15/1, Georg Thieme Verlag, Stuttgart, 1974.

In Verbindungen der Formel (I) kann eine Hydroxygruppe im Rest $R_1$, ferner eine im Rest $R_3$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl, Dichloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarboyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbut-2-yl)-silyl ode tert.-Butyl-dimethylsilyl, und 2-Oxa-oder 2-Thiacycloalkyl mit 5 bis 7 Kohlenstoffatomen, z.B. 2-Tetrahydropyranyl oder 2-Tetrahydrofuranyl. Bevorzugte Hydroxyschutzgruppen sind Triniederalkylsilyl, Niederalkenyloxyoxalyl und Niederalkenyloxycabonyl.

Eine Carboxylgruppe $R_2$, ferner auch eine im Rest $R_3$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, ie acidolytischen oder insbesondere basisch oder neutral hydrolytischen Bedingungen, leicht spaltbar ist. Solche veresterten Carboxylgruppen enthalten als veresternde Gruppen in erster Linie in 1-Stellung verzweigte oder in 1- oder 2-Stellung geeignet substituierte Niederalkylgruppen. Geeignete in veresterter Form vorliegende Carboxylgruppen sind unter anderen Niederalkoxycarbonyl, z.B. Methoxycarbonyl, Aethoxycarbonyl, oder tert.-Butoxycarbonyl, gegebenenfalls durch Nitro oder Niederalkoxy, wie Methoxy, substituiertes Benzyloxycarbonyl, z.B. 4-Nitrobenzyloxycarbonyl, oder 4-Methoxybenzyloxycarbonyl, Niederalkanoylmethoxycarbonyl, wie Acetonyloxycarbonyl, Benzoylmethoxycarbonyl, worin die Benzoylgruppe gegebenenfalls, z.B. durch Halogen, wie Brom, substituiert ist, z.B. Phenacyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenylcarbonyl, z.B. Allyloxycarbonyl, oder in 2-Stellung durch Niederalklysulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methylsilyl)-äthoxycarbonyl. Weitere in veresterter Form

vorliegende geschützte Carboxylgruppen sind entsprechende organische Silyloxycarbonylgruppen. In diesen enthält das Siliciumatom vorzugsweise Niederalkyl, insbesondere Methyl oder Aethyl, ferner Niederalkoxy, z.B. Methoxy, als Substituenten. Geeignete Silylgruppen sind in erster Linie Triniederalkylsilylgruppen, insbesondere Trimethylsilyl oder Dimethyl-tert.-butylsilyl. Bevorzugte geschützte Carboxylgruppen sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butylmethylsilyl, substituierte Aethoxycarbonylgruppe.

Eine geschützte Aminogruppe im Rest $R_3$ kann beispielsweise in Form einer leicht spaltbaren Acylamino- oder Silylamingruppe oder als Nitro- oder Azidogruppe vorliegen. In einer entsprechenden Acylaminogruppe ist Acyl beispielswese der Acylrest einer organischen Säure mit z.B. bis zu 18 Kohlenstoffatomen, insbesondere einer gegebenenfalls z.B. durch Halogen oder Phenyl, substituierten Alkancarbonsäure oder insbesondere eines Kohlensäurehalbesters. Solche Acylgruppen sind beispielsweise Halogenniederalkanoyl, wie 2-Halogenacetyl, insbesondere 2,2,2-Trifluor- oder 2,2,2-Trichloracetyl, Niederalkenyloxycarbonyl, z.B. Allyloxycarbonyl, gegebenenfalls in 1- oder 2-Stellung substituiertes Niederalkoxycarbonyl, wie Niederalkoxycarbonyl, z.B. tert.Butoxycarbonyl, gegebenenfalls substituiertes Benzyloxycarbonyl, z.B. 4-Nitrobenzyloxycarbonyl, 2-Halogenniederalkoxylcarbonyl, z.B. 2,2,2-Trichloräthoxycarbonyl oder 2-Bromäthoxycarboyl oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl. Eine Silylaminogruppe ist in erster Linie eine organische Silylaminogruppe, worin das Siliciumatom vorzugsweise Niederalkyl, z.B. Methyl, Aethyl, n-Butyl oder tert.-Butyl, ferner Niederalkoxy, z.B. Methoxy, als Substituenten enthält. Ensprechende Silylgruppen sind in erster Linie Triniederalkylsilyl, insbesondere Trimethylsilyl, ferner Dimethyl-tert.-butylsilyl. Bevorzugte geschützte Aminogruppen sind z.B. Azido, Nitro, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nichttoxische Salze von Verbindungen der Formel I. Solche Salze werden beispielsweise von den in Verbindungen der Formel I vorhandenen sauren Gruppen, z.B. Carboxylgruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organsichen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N'-Dibenzyläthylendiamin, Dibenzylamin oder N-Benzyl-$\beta$-phenäthylamin. Verbindungen der Formel I mit einer basischen Gruppe, z.B. mit einer Aminogruppe, können Säureadditionssalze, z.B. mit anorganischen Säuren wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen. Zur Isolierung oder Reinigung können auch pharmzeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können in den Resten $R_1$, $R_3$ und/oder A zusätzliche Chiralitätszentren besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist ein Rest $R_1$, welcher ein asymmetrisches Kohlenstoffatom besitzt, insbesondere 1-Hydroxyäthyl, die R-Konfiguration auf. Ferner weist das Kohlenstoffatom, mit dem der Rest $R_3$ an die Gruppe A gebunden ist, eine asymmetrische Substituion auf und kann dementsprechend in der R-, S- oder der racemischen R,S-Konfiguration vorliegen. Ferner kann ein verzweigtkettiger Niederalkylenrest A ein Chiralitätszentrum aufweisen. Die Erfindung betrifft demgemäss die reinen Diastereomeren und Gemische der Diastereomeren von Verbindungen der Formel I, welche im Rest $R_1$, $R_3$ und/oder A zusätzliche Chiralitätszentren aufweisen.

Die Erfindung betrifft vor allem Verbindungen der Formel I, worin $R_1$ durch Hydroxy oder geschütztes Hydroxy substituiertes Niederalkyl ist, $R_2$ Carboxyl, unter physiologischen Bedingungne spaltbares verestertes Carboxyl oder geschütztes Carboxyl $R_2'$ darstellt, A Niederalkylen bedeutet und $R_3$ einen über ein Ringkohlenstoffatom gebundenen, monocyclischen 4- bis 6-gliedrigen, von einer $\omega$-Amino-($C_3$-$C_5$)-niederalkancarbonsäure oder $\omega$-Amino-($C_4$-$C_5$)-niederalkencarbonsäure abgeleiteten Lactamylrest, einen 5- oder 6- gliedrigen, von einer $\omega$-Amino-($C_4$-$C_5$)-niederalkencarbonsäure abgeleiteten Benzolactamylrest, einen monocyclischen 4- bis 6-gliedrigen, von einer $\omega$-Hydroxy-($C_3$-$C_5$)-niederalkancarbonsäure oder $\omega$-Hydroxy-($C_4$-$C_5$)-niederalkencarbonsäure abgeleiteten Lactonylrest oder einen 5- oder 6-gliedrigen, von einer $\omega$-Hydroxy-($C_4$-$C_5$)-niederalkencarbonsäure abgeleiteten Benzolactonylrest darstellt, welcher Rest unsubstituiert oder durch Hydroxy, Niederalkoxy, Niederalkanoyloxy, Halogen, Mercapto, Niederalkylthio, Niederalkyl, durch Hydroxy, Halogen, Niederalkonyloxy, Amino, Nideralkylamino, Diniederalkylamino, Niederalkanoylamino, Carboxyl, Niederalkoxycarbonyl, Cyano, Carbamoyl oder Carbamoyloxy substituiertes Niederalkyl, Niederalkenyl, Amino, Niederalkylamino, Dinierderalkylamino, Niederalkanoylamino, Carboxyl, Niederalkoxycarbonyl, Cyano und oder gegebenenfalls durch Niederalkyl, Hydroxy, Niederalkoxy, Halogen und/oder Nitro substituiertes Phenyl substituiert ist, optische Isomere von solchen Verbindungen der Formel I, welche im Rest $R_1$, $R_3$ und/oder A zusätzliche Chiralitätszentren aufweisen,

Mischungen dieser optischen Isomere und Salze von Verbindungen der Formel I, die eine salzbildende Gruppe enthalten.

Die Erfindung betrifft hauptsächlich Verbindungen der Formel I, worin $R_1$ durch Hydroxy substituiertes Niederalkyl ist, $R_2$ Carboxyl oder unter pysiologischen Bedingungen spaltbares verestertes Carboxyl bedeutet, A Niederalkylen ist und $R_3$ einen Rest der Formel

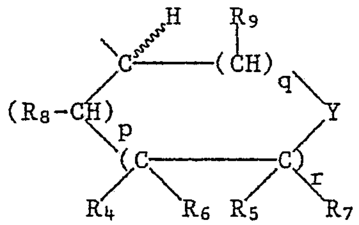

(II),

darstellt, worin Y ein Rest der Formel $-NR_{10}$ -C(=O)- ist und $R_{10}$ Wasserstoff, Niederalkyl, einer der oben als Substituenten von $R_3$ genannten substituierten Niederalkyl-Reste, insbesondere durch Amino, Hydroxy, Carboxy oder Cyano substituiertes Niederalkyl, oder Niederalkenyl ist, $R_4$ und $R_5$ für Wasserstoff stehen oder gemeinsam eine Bindung darstellen, $R_6$ und $R_7$ unabhängig voneinander für Wasserstoff, Niederalkyl, durch Hydroxy, Halogen, Niederalkoxy, Amino, Niederalkylamino, Diniederalkylamino, Niederalkanoylamino oder Carbamoyloxy substitiertes Niederalkyl oder gegebenenfalls durch Niederalkyl, Hydroxy, Niederalkoxy und/oder Halogen substituiertes Phenyl stehen, $R_8$ und $R_9$ unabhängig voneinander für Wasserstoff oder Niederalkyl stehen, r 0 oder 1 ist und p und q unabhängig voneinander für eine ganze Zahl von 0 bis 3 stehen, mit der Massgabe, dass wenn r 0 ist, die Summe von p und q 1, 2 oder 3 ist, oder wenn r 1 ist, die Summe von p und 1 0 oder 1 ist, und mit der weiteren Massgabe, dass das dem Lactam-Ringstickstoffatom benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optische Isomere von solche Verbindungen der Formel I, welche im Reste $R_1$, $R_3$ und/oder A zusätzliche Chiralitätszentren aufweisen, Mischungen dieser optischen Isomere und Salze von Verbindungen der Formel I, die eine salzbildende Gruppe enthalten.

Die Erfindung betrifft ferner hauptsächlich Verbindungen der Formel I, worin $R_1$ durch Hydroxy substituiertes Niederalkyl ist, $R_2$ Carboxyl oder unter physiologischen Bedingungen spaltbares verestertes Carboxyl bedeutet, A Niederalkylen ist und $R_3$ einen Rest der Formel

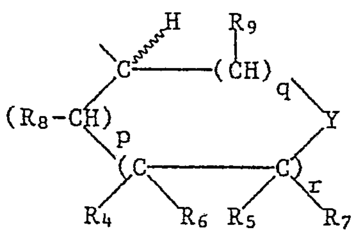

(II),

darstellt, worin Y für die Gruppe -O-C(=O)- steht, $R_4$ und $R_5$ Wasserstoff bedeuten oder gemeinsam eine Bindung darstellen, $R_6$ und $R_7$ unabhängig voneinander für Wasserstoff, Niederalkyl, durch Hydroxy, Halogen, Niederalkanoyloxy, Amino, Niederalkylamino, Diniederalkylamino, Niederalkanoylamino oder Carbamoyloxy substituiertes Niederalkyl, Hydroxy, Halogen, Amino, Niederalkylamino, Diniederalkylamino, Niederalkanoylamino oder gegebenenfalls durch Niederalkyl, Hydroxy, Niederalkoxy und/oder Halogen substituiertes Phenyl stehen oder $R_4$ und $R_5$ gemeinsam eine Bindung darstellen und $R_6$ und $R_7$ gemeinsam für einen gegebenenfalls durch Hydroxy, Niederalkoxy, Niederalkyl und/oder Halogen substituierten 1,4-Butadien1,4-ylen-Rest stehen, $R_8$ und $R_9$ unabhängig voneinander Wasserstoff, Niederalkyl, Amino oder Hydroxy bedeuten, r 0 ist, die Summe von p und q 2 oder 3 ist, oder r 1, q 0 und p 0 oder 1 ist, mit der Massgabe, dass das dem Lacton-Ringsauerstoff benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optische Isomere von solchen Verbindungen der Formel I, welche im Reste $R_1$, $R_3$ und/oder A zusätzliche Chiralitätszentren aufweisen, Mischungen solcher optischen Isomere und Salze von Verbindungen der Formel I, die eine salzbildende Gruppe enthalten.

Die Erfindung betrifft in erster Linie Verbindungen der Formel I, worin $R_1$ in $\alpha$-Stellung durch Hydroxy substituiertes Niederalkyl ist, $R_2$ Carboxyl oder unter physiologischen Bedingungen spaltbares verestertes Carboxyl bedeutet, A Niederalkylen ist, und $R_3$ einen Rest der Formel II darstellt, worin Y die Guppe $-NR_{10}$ -C(=O)- ist und $R_{10}$ Wasserstoff oder Niederalkyl bedeutet, $R_8$ und $R_9$ Wasserstoff bedeuten, r 0 ist, p eine ganze Zahl von 1 bis 3 ist und q 0 oder 1 ist, mit der Massgabe, dass die Summe von p und q höchstens 3 beträgt, optische Isomere von solchen Verbindungen der Formel I, welche im Rest $R_1$, $R_3$ und/oder A zusätzliche Chiralitätszentren aufweisen, Mischungen dieser optischen Isomere und Salze von Verbindungen der Formel I, die eine salzbildende Gruppe enthalten.

Die Erfindung betrifft ferner in erster Linie Verbindungen der Formel I, worin $R_1$ in $\alpha$-Stellung durch Hydroxy substituiertes Niederalkyl ist, $R_2$ Carboxyl oder unter physiologischen Bedingungen spaltbares verestertes Carboxyl bedeutet, A Niederalkylen ist, und $R_3$ einen Rest der Formel II darstellt, worin Y eine Gruppe -O-C(=O)-ist, r 1 ist, p und q 0 sind, $R_4$, $R_5$, $R_6$ und $R_7$ Wasserstoff darstellen oder $R_4$ und $R_5$ gemeinsam eine Bindung darstellen und $R_6$ und $R_7$ unabhängig voneinander für Wasserstoff, Niederalkyl oder durch Halogen, Amino, Diniederalkylamino oder Niederalkanoylamino substituiertes Niederalkyl stehen, oder r 0 ist, p und q jeweils 1 sind und $R_8$ und $R_9$ für Wasserstoff stehen, optische Isomere von solchen Verbindungen der Formel I, welche im Rest $R_1$, $R_3$ und/oder A zusätzliche Chiralitätszentren aufweisen, Mischungen solcher optischen Isomere und Salze von Verbindungen der Formel I, die eine salzbildende Gruppe enthalten.

Die Erfindung betrifft bevorzugt Verbindungen der Formel I, worin $R_1$ Hydroxymethyl oder 1-Hydroxyäthyl ist, $R_2$ Carboxyl oder unter physiologischen Bedingungen spaltbares verestertes Carboxyl bedeutet, A für Methylen oder Aethylen steht und $R_3$ 5-Oxo-tetrahydrofuran-2-yl, gegebenenfalls durch Niederalkyl substituiertes 2,5-Dihydro-5-oxo-furan-2-yl, 5-Oxo-pyrrolidin-2-yl oder 4-Oxo-azetidin-2-yl ist, optische Isomere von Verbindungen der Formel I, Mischungen dieser optischen Isomere und Salze von Verbindungen der Formel I, die eine salzbildende Gruppe aufweisen.

Die Erfindung betrifft in erster Linie die in den Beispielen genannten Verbindungen der Formel I und deren Salze, insbesondere deren pharmazeutisch annehmbare Salze.

Die Verbindungen der vorliegenden Erfindung können nach an sich bekannten Verfahren hergestellt werden.

Die neuen Verbindungen werden z.B. hergestellt, indem man eine Ylid-Verbindung der Formel

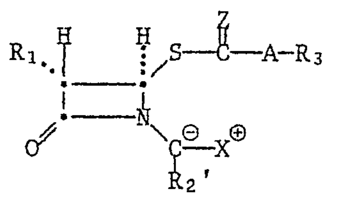

(III),

worin $R_1$, A und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

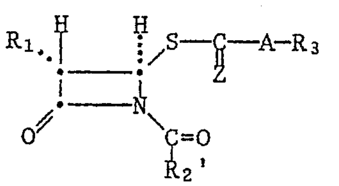

(IV),

worin $R_1$, A und $R_3$ die unter Formel I und $R_2'$ und Z die unter Formel III engegebenen Bedeutungen haben, mit einer organischen Verbindung des dreiwertigen Phosphors behandelt, und, wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine Carboxylgruppe $R_2$ in eine unter physiologischen Bedingungen spaltbare veresterte Carboxylgruppe überführt, und/oder, wenn erwünscht, in einer erhältlichen Verdung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

In den Ausgangsverbindungen der Formeln III und IV sind funktionelle Gruppen, wie eine freie Hydroxygruppe im Rest $R_1$ sowie weitere im Rest $R_3$ enthaltene funktionelle Gruppen, vorzugsweise durch konventionelle Schutzgruppen, z.B. druch die oben genannten, geschützt.

<u>Cyclisierung der Verbindung der Formel III</u>

Die Gruppe $X^{\oplus}$ im Ausgangsmaterial der Formel III ist eine der bei Wittig-Kondensationsteaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

Der Ringschluss kann spontan, d.h. bei der Herstellung der Ausgangsstoffe, oder durch Erwärmen, z.B. in

einem Temperaturbereich von etwa 30°C bis 160°C, vorzugsweise von etwa 50°C bis etwa 100°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder Tetrahydrofuran, einem Carbonsäureamid, z.B. Dimethylformamid, einem Diniederalkylsulfoxide, z.B. Dimethylsulfoxid, oder einem Niederalkanol, z.B. Aethanol, oder in einem Gemisch davon, und, falls notwendig, in einer Inertgas-, z.B. Stickstoffatmosphäre, durchgeführt.

Cyclisierung der Verbindung der Formel IV

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2\ N(R_b)_2$ , worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt von etwa 20° bis etwa 110°C, durchgeführt, wobei man 1 Moläquivalent einer Verbindung der Formel IV mit mindestens 2 Moläquivalenten der Phosphorverbindung umsetzt. Vorzugsweise legt man die Verbindung der Formel IV in einem inerten Lösungsmittel vor und tropft die Phosphorverbindung, vorzugsweise in dem gleichen inerten Lösungsmittel gelöst, hinzu.

In einer bevorzugten Ausführungsform des Verfahrens stellt man das Ausgangsmaterial der Formel IV wie weiter unten angegeben her und setzt es ohne Isolierung aus dem Reaktionsgemisch mit der organischen Verbindung des dreiwertigen Phosphors um, wobei die Endprodukte der Formel I entstehen.

Verfahrensgemäss erhältliche Verbindung der Formel I können in an sich bekannter Weise in andere Verbindungen der Formel I überführt werden.

So können in einer erhältlichen Verbindung der Formel I, worin eine oder mehrere funktionelle Gruppen geschützt sind, diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigestzt werden.

In einer verfahrensgemäss erhältlichen Verbindung der Formel I, worin $R_2$ eine geschützte Carboxylgruppe bedeutet und/oder worin der Rest $R_3$ geschütztes Carboxyl als Substituenten enthält, kann die geschützte Carboxylgruppe in an sich bekannter Weise freigesetzt werden. So kann man tert.-Niederalkoxycarbonyl oder in 2-Stellung durch eine trisubstituierte Silylgruppe substituiertes Niederalkoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoffabgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Tributylzinnhydrid, Dimethylbarbitursäure oder Dimedon, erfolgen. Durch Behandeln mit einem reduzierenden Metall oder Metallsalz, wie oben beschrieben, kann man auch 2-Halogennie-deralkoxycarbonyl (gegebenenfalls nach Umwandlung einer 2-Bromniederalkoxycarbonylgruppe in eine entsprechende 2- Jodniederalkoxycarbonylgruppe) oder Benzoylmethoxycarbonyl in freies Carboxyl umwandeln, wobei Benzoylmethoxycarbonyl durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluoride, in Anwesentheit eines makrocyclischen Polyäthrs ("Kronenäther") oder mit einem Fluorid einer organischen quaternären Base, wie Tetraniederalkylammoniumfluorid, z.B. Tetrabutylammoniumfluoride, in freies Carboxyl überführt werden. Mit einer organischen Silylgruppe, wie Triniederalkylsilyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium-oder Kaliumhydroxid oder Natrium- oder Kaliumcarbonat, in freies Carboxyl übergeführt werden.

Andererseits können auch Verbindungen der Formel I, worin $R_2$ Carboxy, bedeutet, in Verbindungen der

Formel 1 überführt werden, worin $R_2$ eine unter physiologischen Bedingungen spaltbare veresterte Carboxylgruppe darstellt. Solche Ester können z.B. durch Umsetzung eines gegebenenfalls in situ hergestellten Salzes der Säure mit einem reaktionsfähigen Ester eines entsprechenden Alkohols und einer starken anorganischen Säure, wie Schwefelsäure, oder einer starken organischen Sulfonsäure, wie 4-Toluolsulfonsäure, herge stellt werden. Ferner können Säurehalogenide, wie Chloride (hergestellt z.B. durch Behandeln mit Oxalylchlorid), aktivierte Ester, (gebildet z.B. mit N-Hydroxystickstoffverbindungen, wie N-Hydroxysuccinimid) oder gemischte Anhydride (erhalten z.B. mit Halogenameisensäure-niederalkylester, wie Chlorameisensäureäthyl- oder Chlorameinsensäureisobutylester, oder mit Halogenessigsäurehalogeniden, wie Trichloressigsäurechlorid) durch Umsetzen mit geeigneten Alkoholen, gegebenenfalls in Gegenwart einer Base, wie Pyradin, in eine veresterte Carboxylgruppe übergeführte werden. Ferner kann man in Verbindungen der Formel I, die eine in veresterter Form geschützte Carboxylgruppe enthalten, die Carboxylschutzgruppen wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_2$ eine unter physiologischen Bedingungen spaltbare veresterte Carboxylgruppe darstellt.

In verfahrensgemäss erhältlichen Verbindungen der Formel I, worin der Rest $R_1$ und/oder gegebenenfalls der Rest $R_3$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe kann z.B. auch mit Tetrabutylammoniumfluoride und Essigsäure abgespalten werden (unter diesen Bedingungen werden durch trisubstituiertes Silyläthoxy geschützte Carboxygruppen nicht gespalten).

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), 4-Nitrobenzyloxycarbon ylamino durch Behandeln mit einem geeigneten chemischen Reduktionsmittel, wie Zink in Gegenward einer geeigneten Carbonsäure, wie wässriger Essigsäure, oder katalytisch mit Wasserstoff in Gegenwart eines Palladiumkatalysators oder durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis (triphenylphosphin)palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Tributylzinnhydrid oder Dimedon, gespalten werden. Eine mit einer organischen Silylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetall-thiolat, des Thioharnstoffs und anschliessender Solvolyse, wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederakylammoniumfluorid, z.B. Tetraäthylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxide oder Palladium, oder durch Behandeln mit Zink in Gegenwart einer Säure, wie Essigsäure.

In Verbindungen der Formel I kann man weiterhin einen Rest $R_3$ in einen anderen Rest $R_3$ überführen.

So kann beispielsweise in Verbindungen der Formel I, worin der Rest $R_3$ durch eine Carboxylgruppe substituiert ist, diese Carboxylgruppe nach an sich bekannten Verfahren in eine funktionell abgewandelte Carboxylgruppe, wie in eine veresterte Carboxylgruppe, überführt werden. Beispielsweise erhält man durch Umsetzen einer Verbindung der Formel I, worin der Rest $R_3$ durch Carboxyl substituiert ist, mit einem Alkohol, insbesondere einem Niederalkanol, eine Verbindung der Formel I, worin $R_3$ durch verestertes Carboxyl, insbesondere Niederalkoxycarbonyl, substituiert ist, wobei man vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Restern $R_3$ auch in reaktionsfähige funktionelle Derivate, wie gemischte Anhydride, z.B. Säurehalogenide, oder aktivierte Ester, überführen und diese durch Umsetzen mit einem Alkohol, z.B. Niederalkanol, in entsprechend veresterte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall- oder Erdalkalimetallcarbonats, arbeitet.

In Verbindungen der Formel I, worin der Rest $R_3$ durch Amino substituiert ist, kann die Aminogruppe in eine substituierte Aminogruppe, z.B. eine Niederalkylamino-, Diniederalkylamino- oder Niederalk-anoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem

Niederalkylhalogenid oder -sulfonat, in Gegenwart eines basischen Kondensationsmittels, wie eines Hydroxids oder Carbonats eines Alkali- oder Erdalkalimetalls oder einer heteroaromatischen Stickstoffbase, z.B. Pyridin. In analoger Weise kann Amino durch Behandeln mit dem reaktionsfähigen funktionellen Derivat einer Niederalkancarbonsäure, z.B. dem entsprechenden Carbonsäurehalogenid, in Niederalkanoylamino umgewandelt werden. Verbindungen der Formel I mit einem substituierbaren Ringstickstoffatom im Rest $R_3$ [Y in Rest der Formel II ist -NH-C(=O)] können in analoger Weise mit gegebenenfalls substituierten Niederalkyl oder mit Niederalkenylhalogeniden in Gegenwart einer starken Base, z.B. Natriumhydrid, in Verbindungen der Formel I überführt werden, in denen der Rest $R_3$ ein durch gegebenenfalls substituiertes Niederalkyl oder Niederalkenyl substituiertes Ringstickstoffatom enthält.

Salze von Verbindungen der Formel I mit salzbildenen Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der $\alpha$-Aethylcapronsäure, oder mit anorganischen Alkali-oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physikalisch-chemischen Methoden (z.B. fraktioniertes Kristallisieren, Adsorptionschromatographie) trennen und die einzelnen diastereomeren Verbindungen dann in die optisch aktiven Verbindungen spalten.

Bei allen nachträglichen Umwandlungen erhaltener Verbindungen der Formel I werden solche Reakionen bevorzugt, die unter gemässigt alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Staufe abgebrochen wird. Ferner können Ausgangsstoffe in Form von Derivaten verwendet oder in situ, gegebenenfalls under den Reaktionsbedingungen, hergestellt werden.

Die Ausgangsverbindungen der Formeln III und IV können, wie in dem folgenden Reaktionsschema I angegeben, hergestellt werden:

Reaktionsschema I

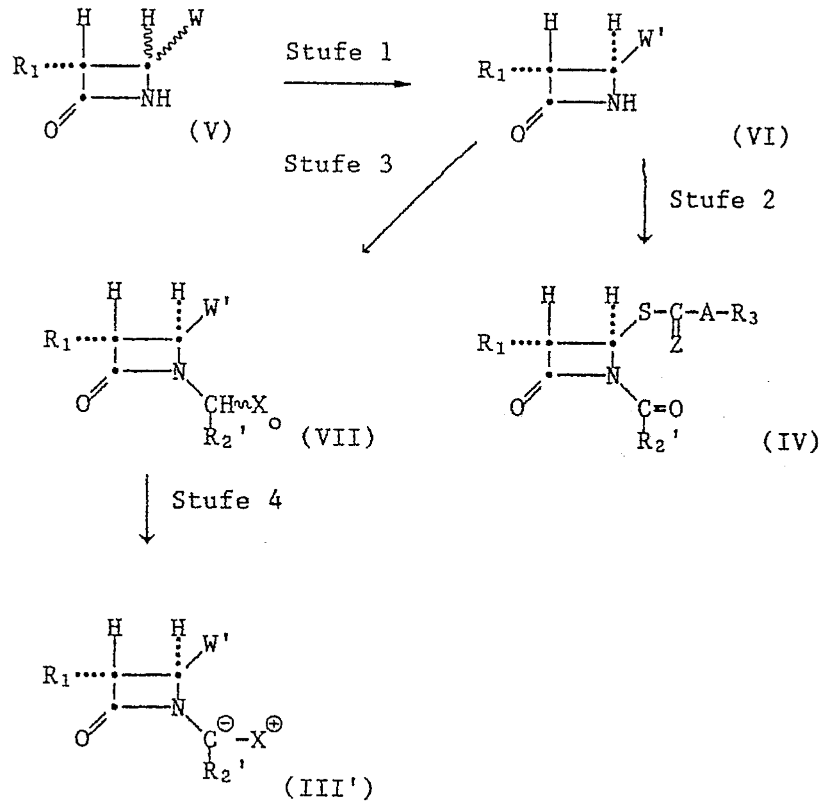


(V) (VI) (VII) (IV) (III')

Stufe 1

Geeignete Ausgangsverbindungen der Formel V, worin W ein durch nucleophile Reaktion leicht austauschbarer Rest, z.B. Niederalkanoyloxy, wie Acetyloxy, oder Sulfonyloxy $R_o$-$SO_2$ -, worin $R_o$ z.B. gegebenenfalls durch Hydroxy substituierten Niederalkyl, wie Methyl, tert.Butyl oder 2-Hydroxyäthyl, ist, sind beispielsweise aus der veröffentlichten Europäischen Patentanmeldung Nr. 82113, der Deutschen Offenlegungsschrift Nr. 3 224 055 und der Deutschen Offenlegungsschrift Nr. 3 013 997 bekannt oder können in dazu analoger Weise hergestellt werden.

In den Verbindungen der Formel VI steht W' für den Rest -S-C(=Z)-A-$R_3$ oder für Triphenylmethylthio oder Niederalkanoylthio.

Eine den Rest -S-C(=Z)-A-$R_3$ einführende Verbindung ist beispielsweise eine Säure der Formel $R_3$ -A-C(=Z)-SH oder insbesondere ein Salz, z.B. ein Alkalimetallsalz, wie das Natrium- oder Kaliumsalz, davon. Die Substitution kann in einem organischen Lösungsmittel, wie in einem Niederalkanol, einem Niederalkancarbonsäureamid, einem cyclischen Aether, oder in einem ähnlichen inerten Lösungsmittel bei Raumtemperatur oder bei etwas erhöhter oder erniedrigter Temperatur, z.B. bei etwa 0° bis etwa 40°C, durchgeführt werden. Die Einführung eines Triphenylmethylthio- oder Niederalkanoylthio-Restes W' erfolgt in analoger Weise durch Umsetzen mit einem Alkalimetallsalz, z.B. dem Natriumsalz, einer Niederalkanthiocarbonsäure, z.B. Thioessigsäure, oder des Triphenylmethylmercaptans.

Säuren der Formel $R_3$ -A-C(=Z)-SH und ihre Salze sind aus der Literatur bekannt oder können in analoger Weise zu den in der Literatur beschriebenen Verfahren hergestellt werden.

Stufe 2

Eine Ausgangsverbindung der Formel (IV) wird erhalten, indem man ein Azetidinon der Formel (VI), in der W' für den Rest -S-C(=Z)-A-$R_3$ steht, mit einer Säure der Formel $R_2$ '-COOH oder insbesondere einem reaktionsfähigen Derivat, wie einem Ester oder Säurehalogenid, z.B. dem Säurechlorid, davon bei einer Temperatur von -50° bis 80°C, bevorzugt bei -20° bis 0°C, in einem inerten Lösungsmittel, wie einem der bei der Umsetzung von Verbindungen der Formel (IV) zu Verbindungen der Formel I genannten, behandelt. Bei Verwendung eines Säurehalogenids arbeitet man vorzugsweise in Gegenwart eines säurebindenden Mittels, wie eines tertiären aliphatischen Amins, eines aromatischen Amins, oder insbesondere eines Alkali-metall- oder Erdalkalimetallcarbonats oder -hydrogencarbonats.

Verbindungen der Formel (VI), worin W' für Triphenylmethylthio oder Niederalkanoylthio steht, können in die

Ausgangsverbindungen der Formel VI, worin W' für den Rest -S-C(=Z)-A-$R_3$ steht, überführt werden, indem man sie in Gegenwart einer Base, Z.B. Pyridin oder Tri-n-butylamin, in einem geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt, und das entstandene Saiz der Formel

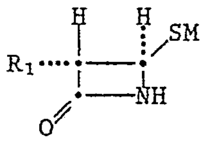

(VI'),

mit einem den Rest $R_3$ -A-C(=Z)- einführenden Acylierungsmittel, z.B. mit der Säure $R_3$ -A-C-(=Z)-OH oder einem reaktionsfähigen funktionellen Derivat, wie einem Säurehalogenid, z.B. dem Chlorid oder Bromid, Azid oder Anhydrid davon, behandelt. Die Acylierung erfolgt,wenn ein reaktionsfähiges funktionelles Derivat der Säure der Formel $R_3$ -A-C(=Z)-OH, z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -5-° bis ca. +60°C, insbesondere bei ca. -30° bis ca. +20°C.

Säuren der Formel $R_3$ -A-C(=Z)-OH und reaktionsfähige funktionelle Derivate davon sind aus der Literatur bekannt oder können in analoger Weise zu den in der Literatur beschriebenen Verfahren hergestellt werden.

Stufe 3

Verbindungen der Formel VII, worin $X_0$ für eine reaktionsfähige veresterte Hydroxygruppe, insbesondere für Halogen, z.B. Chlor oder Brom, steht, werden hergestellt, indem man eine Verbindung der Formel VI mit einer Glyoxylsäure-Verbindung der Formel $R_2$ '-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VII, worin $X_0$ für Hydroxy steht, die Hydroxygruppe in eine reaktionsfähige veresterte Hydroxygruppe umwandelt. Die Verbindungen der Formel VII werden üblicherweise als Gemisch der beiden Isomeren [bezüglich der Gruppierung -CH($R'_2$) ∿ $X_0$ ] erhalten.

Die Anlagerung der Glyoxylsäureester-Verbindung an das Stickstoffatom das Lactamrings in der Verbindung der Formel VI findet bei Raumtemperatur oder, falls notwendig, unter Erwärmen, statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels enfernt wird. Vorzugsweise arbeitet man in Gegenwart eines geeigneten inerten Lösungsmittels oder Lösungsmittelgemisches.

Die Ueberführung einer Hydroxygruppe $X_0$ in eine reaktionsfähige veresterte Hydroxygruppe $X_0$ in einer Verbindung der Formel VII wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base vom Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30°C.

Stufe 4

Das Ausgangsmaterial der Formel (III) wird erhalten, indem man eine Verbindung der Formel (VII) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältlichen Verbindung der Formel (III') worin W' für Triphenylmethylthio oder Niederalkanoylthio steht, in eine Verbindung der Formel (III') überführt, worin W' für den Rest -S-C(=Z)-A-$R_3$ steht.

Die Umsetzung mit der Phosphin- bzw. Phosphit-Verbindung wird vorzugsweise in einem geeigneten inerten Lösungsmittel, wie einem Kohlenwasserstoff, oder einem Aether, oder in einem Lösungsmittelgemisch vorgenommen. Je nach Reaktionsfähigkeit arbeitet man unter Kühlen oder bei erhöhter Temperatur, etwa zwischen -10° und +100°C, bevorzugt bei etwa 20° bis 80°C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

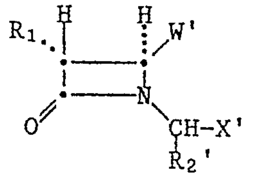

(IIIa),

worin X′ eine Phosphonogruppe oder eine Phosphoniogruppe zusammen mit einem Anion, je nach Bedeutung des Restes $X_0$ (vgl. Formel VII) z.B. Chlorid, bedeutet, in das Ylid-Ausgangsmaterial der Formel III umgewandelt wird.

Die Einführung des Restes $-S-C(=Z)-A-R_3$ in Verbindungen der Formel III′, worin W′ für Niederalkanoylthio oder Triphenylmethylthio steht, kann in analoger Weise erfolgen, wie unter Stufe 2 beschrieben.

Man kann das in Reaktionschema 1 beschriebene Verfahren zur Herstellung der Verbindungen der Formeln (III), (IV), (VI) und (VII), sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat in bekannter Weise, z.B. wie oben beschrieben, die optisch aktiven Verbindungen gemäss der vorliegenden Erfindung isolieren.

Die Erfindung betrifft ebenfalls die neuen Ausgangsverbindungen sowie verfahrensgemäss erhältliche neue Zwischenprodukte, wie diejenigen der Formeln (III), (IV), ferner (VI) und (VII) (W′ steht für den Rest $-S-C(=Z)-A-R_3$ sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen der Formel I gelangt.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_2$ Carboxyl oder eine unter physiologischen Bedingungen spaltbare veresterte Carboxylgruppe darstellt und A und $R_3$ die unter Formel I angegebenen Bedeutungen haben und pharmakologisch annehmbare Salze von solchen Verbindungen mit salzbildenden Gruppen haben antibakterielle Wirkungen. Beispielsweise sind sie in vitro gegen grampositive und gramnegative Kokken, inkl. Enterokokken, z.B. Staphylococcus aureus, Streptococcus pyogenes, Streptococcus pneumoniae und Streptococcus faecalis, und Neisseria sp. in minimalen Konzentrationen von ca. 0,01 bis ca. 64 µg/ml, gegen gramnegative Stäbchenbakterien, wie Enterobacteriaceae, Haemophilus influenzae und Pseudomonas aeruginosa, in minimalen Konzentrationen von ca. 0,05 bis ca. 128 µg/ml und gegen Anaerobier, wie Bacteroides fragilis oder Clostridium sp. in minimalen Konzentrationen von ca. 0,01 bis ca. 0.5 µg/ml wirksam. In vivo, bei der systemischen Infektion der Maus, z.B. durch Staphylococcus aureaus, Streptococcus pyogenes oder Escherichia coli, ergeben sich bei subkutaner Applikation erfindungsgemässer Verbindungen $ED_{50}$ -Werte von ca. 2 bis ca. 15 mg/kg.

Die neuen Verbindungen können als oral oder parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppe in geschützter Form vorliegt, können als Zwischenprodukte zur Herstellung der oben genannten pharmakologisch wirksamen Verbindungen der Formel I verwendet werden.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konzervier-, Stabilisier-, Netz- und/oder Emulgiermittel. Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-,

Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 2 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Beispiel 1:
2-[3S,4R)-3-(tert.Butyldimethylsilyloxymethyl)-4-[(2S)-5-oxo-pyrrolidin-2--yl-acetylthio]-2-oxo-azetidin--1-yl]-2-triphenylphosphoranylidenessigsäureallylester

1,32 g (8,2 mmol) (2S)-5-Oxo-pyrrolidin-2-ylacetylchlorid (hergestellt aus der Säure mit $SOCl_2$ ) wird langsam unter Rühren zu einer Lösung von 4,0 g (5,6 mmol) Silbersalz des 2-[(3S,4R)-3-tert.-Butyldimethylsilyloxymethyl)-4-mercapto-2-oxo-azetidin-1-yl]-2-triphenylphosphoranylidenessigsäureallylesters (Europäische Patentanmeldung Nr. 125207) und 0,85 ml (10,6 mmol) Pyridin in 70 ml $CH_2Cl_2$ getropft (0° unter $N_2$ ). Nach beendigtem Zutropfen rührt man noch 0,5 Stunde bei 0°. Der Niederschlag wird über Hyflo abgenutscht und das Filtrat je zweimal mit ges. $NaHCO_3$ - und Kochsalz-Lösung gewaschen, mit $Na_2SO_4$ getrocknet und eingedampft. Das Rohprodukt wird mittels Flashchromatographie (Kieselgel Merck 70-230 mesh ASTM; Aethylacetat) gereinigt.

$\alpha_D$ (1,12 % Aethanol) 25,6°
IR (3 % $CH_2Cl_2$ ): 3420, 2860-3500, 1756, 1702, 1641, 1438, 1241, 1104, 838 $cm^{-1}$.

Beispiel 2:
(5R,6S)-6-(tert.Butyldimethylsilyloxymethyl)-2-[(2S)-5-oxo-pyrrolidin-2-ylmethyl]-2-penem-3-carbonsäureallylester

1,7 g 2-[(3S,4R)-3-(tert.Butyldimethylsilyloxymethyl)-4[(2S)-5-oxo-pyrrolidin-2-acetylthio]-2-oxo-azetidin-1-yl]-2-triphenylphosphoranylidenessigsäureallylester werden in 500 ml Toluol gelöst und während 1,5 Stunden unter Stickstoff am Rückfluss gerührt. Eindampfen der Lösung und Flashchromatographie (Kieselgel Merck 70-230 mesh ASTM; Toluol/Aethylacetat 1:1) ergeben das reine Endprodukt.

$\alpha_D$ (1,02 % Aethanol) 112,7°;
IR (3 % $CH_2Cl_2$ ): 3420, 2820-3460, 1789, 1702, 1651, 1581, 1314, 1122, 837 $cm^{-1}$.
$^1$H-NMR ($CDCl_3$ ) $\delta$ 5,96 (m,1) 5,86 (s,1), 5,57 (d,1, J=1,4), 5,39 (dd,1, J=1,3 und 17,4), 5,25 (dd,1, J=1,3 und 10,5), 4,72 (ddd,1 J=1,0, 5,0 und 13,0), 3,96 (dd,1, J=4,5 und 11,0), 3,92 (dd,1, J=3,9 und 11,0), 3,90 (m,1), 3,86 (m,1), 3,33 (dd,1, J=5,3 und 13,9), 2,71 (dd,1, J=5,7 und 13,9), 2,35 (m,2), 2,30 (m,1), 1,87 (m,1), 0,88 (s,9), 0,08 (s,6) ppm.

Beispiel 3: (5R,6S)-6-Hydroxymethyl-2-[(2S)-5-oxo-pyrrolidin-2-ylmethyl]-2-penem-3-carbonsäureallylester

770 mg (1,70 mmol) (5R,6S)-6-(tert.Butyldimethylsilyloxymethyl)-2-[(2S)-5-oxo-pyrrolidin-2-ylmethyl]-2-penem-3-carbonsäureallylester werden in 35 ml getrocknetem Tetrahydrofuran gelöst und unter Argonatmosphäre nach Abkühlung auf -78° mit 0,67 ml (11,71 mmol) Essigsäure versetzt. Dann gibt man tropfenweise 3,76 ml (3,76 mmol) einer 1M Tetrabutylammoniumfluorid-Lösung in Tetrahydrofuran dazu, lässt das Gemisch auf Raumtemperatur kommen und rührt 4,5 Stunden bei dieser Temperatur. Es fällt langsam das weisse Produkt aus, das mit kaltem, getrocknetem Tetrahydrofuran und anschliessend mit Aether gewaschen wird. Smp. 134-138°.

$\alpha_D$ (1,00 % Aethanol) 134,0°;
IR (3 % $CH_2Cl_2$ ): 3651, 3420, 2820-3700, 1790, 1705, 1650, 1580, 1315 $cm^{-1}$;
$^1$H-NMR ($CDCl_3$ ) $\delta$ 6,12 (s,1), 5,95 (m,1), 5,65 (d,1, J=1,4), 5,41 (dd,1, J=1,3 und 17,4), 5,28 (dd,1, J-1,3 und 10,5), 4,78 (ddd,1, J=1,0, 5,0 und 13,0), 4,66 (ddd,1, J-1,0, 5,0 und 13,0), 4,01 (m,2), 3,96 (m,1), 3,92 (m,1), 3,30 (dd,1, J=5,3 und 13,9) 2,75 (dd,1 J=5,7 und 13,9), 2,42 (m,1), 2,34 (m,2) 2,30 (m,1), 1,87 (m,1) ppm.

Beispiel 4: (5R,6S)-6-Hydroxymethyl-2-[(2S)-5-oxo-pyrrolidin-2-ylmethyl]-2-penem-3-carbonsäure, Natriumsalz

390 mg (1,16 mmol) (5R,6S)-6-Hydroxymethyl-2-[(2S)-5-oxo-pyrrolidin-2-ylmethyl]-2-penem-3-carbonsäureallylester und 163 mg (1,16 mmol) Dimedon werden in 150 ml Aethanol bei 35° unter Stickstoffatmosphäre gelöst. Nach Zugabe von 183 mg (0,16 mmol) Palladiumtetrakis-(triphenylphosphin) wird die Reaktionsmischung 2,5 Stunden im Dunkeln bei gleicher Temperatur gerührt. Die weisse Suspension wird filtriert und mit kaltem Aethanol, Aethylacetat und Aether gewaschen. Das Produkt wird als Natriumsalz bei pH 7,1 mit Natriumhydrogencarbonatlösung über $XAD_2$ filtriert und danach lyophilisiert.

$\alpha_D$ (0,38 % $H_2O$): 104,5°;
UV ($H_2O$): $\lambda_{max}$ 260, 305 nm;
IR (DMSO-$d_6$ ): 2700-3680, 1770, 1690, 1610, 1581, 1367 $cm^{-1}$;
$^1$H-NMR ($D_2O$) $\delta$ 5,61 (d,1, J=1,4), 4,03 (m,1), 3,99 (m,1), 3,94 (m,2), 3,12 (dd, J-5,5 und 14,3), 3,03 (dd,1, J=5,5 und 14,3), 2,40 (m,2), 2,31 (m,1), 1,91 (m,1) ppm.

Beispiel 5:
2-[(3S,4R)-3-tert.Butyldimethylsilyloxymethyl)-4-[(2R)-5-oxo-pyrrolidin-2-ylacetylthio]-2-oxo-azetidin-1-yl]-2-triphenylphosphoranylidenessigsäureallylester

Analog Beispiel 1 wird aus dem Silbersalz des 2-[(3S,4R)-3-(tert.-Butyldimethylsilyloxymethyl)-4-mercapto-2-oxo-azetidin-1-yl]-2-triphenylphosphoranylidenessigsäureallylesters und (2R)-5-Oxo-pyrrolidin-2-ylacetylchlorid (hergestellt aus der Säure mit $SOCl_2$ ) die Titelverbindung hergestellt.
$\alpha_D$ (0,99 % Aethanol) 32,3°; IR (3 % $CH_2Cl_2$ ): 3420, 2835-3470, 1756, 1704, 1620, 1437, 1242, 1104, 838 $cm^{-1}$.

Beispiel 6:
(5R,6S)-6-(tert.Butyldimethylsilyloxymethyl)-2-[(2R)-5-oxo-pyrrolidin-2-ylmethyl]-2-penem-3-carbonsäureallylester

2-[(3S,4R)-3-(tert.Butyldimethylsilyloxymethyl)-4-[(2R)-5-oxo-pyrrolidin-2-ylacetylthio]-2-oxo-azetidin-1-yl]-2-triphenylphosphoranylidenessigsäureallylester wird in analoger Weise, wie in Beispiel 2 beschrieben, zur Titelverbindung cyclisiert.
Smp. 99-101°.
$\alpha_D$ (0,98 % Aethanol) 85,0°;
IR (3 % $CH_2Cl_2$ ): 3420, 2770-3470, 1789, 1704, 1581, 1315, 1121, 837 $cm^{-1}$;
$^1$H-NMR ($CDCl_3$ ): $\delta$ 5,92 (m,1), 5,77 (s,1), 60 (d,1, J=1,3), 5,43 (dd,1, J=1,3 und 17,4), 5,25 (dd,1, J=1,3 und 11,0), 4,72 (ddd,1, J=1,0, 5,0 und 13,7), 4,59 (ddd,1, J=1,0, 5,0 und 13,7), 3,97 (dd,1, J=5,0 und 10,8), 3,92 (dd,1 J=4,0 und 10,8), 3.90 (m,1), 3,85 (m,1), 3,14 (dd,1, J=6,3 und 14,1), 2,92 (dd,1, J=5,0 und 14,1), 2,37 (m,2), 2,29 (m,1), 1,87 (m,1), 0,89 (s,9), 0,08 (s,3) ppm.

Beispiel 7: (5R,6S)-6-Hydroxymethyl-2-[(2R)-5-oxo-pyrrolidin-2-ylmethyl]-2-penem-3-carbonsäureallylester

Analog Beispiel 3 erhält man ausgehend von (5R,6S)-6-(tert.Butyldimethylsilyloxymethyl)-2-[(2R)-5-oxo-pyrrolidin-2-ylmethyl]-2-penem-3-carbonsäureallylester die Titelverbindung.
$\alpha_D$ (1,04 % Aethanol) 93,4°;
IR (3 % $CH_2Cl_2$ ): 3603, 3419, 2750-3700, 1787, 1703, 1578, 1315 $cm^{-1}$;
$^1$H-NMR ($CDCl_3$ ) $\delta$ 6,50 (s,1), 5,93 (m,1), 5,67 (d,1, J=1,3), 5,43 (dd,1, J=1,3 und 17,4), 5,28 (dd,1, J=1,3 und 11,0), 4,77 (dd,1, J=5,0 und 13,7), 4,66 (dd,1, J=5,0 und 13,7), 4,00 (m,2), 3,94 (m,2), 3,24 (dd,1, J=6,3 und 14,2), 2,83 (dd,1, J=4,8 und 14,2) 2,82 (m,1), 2,38 (m,2), 2,31 (m,1), 1,87 (m,1) ppm.

Beispiel 8: (5R,6S)-6-Hydroxymethyl-2-[(2R)-5-oxo-pyrrolidin-2-ylmethyl]-2-penem-3-carbonsäure, Natriumsalz

Ausgehend von (5R,6S)-6-Hydroxymethyl-2-[(2R)-5-oxo-pyrrolidin-2-ylmethyl]-2-penem-3-carbonsäureallylester erhält man in analoger Weise, wie in Beispiel 4 beschrieben, die Titelverbindung.
$\alpha_D$ (0,28 % $H_2O$) 169,4°;
UV ($H_2O$) $\lambda_{max}$ 259, 305 nm;
IR (DMSO-$d_6$ ): 2700-3650, 1769, 1690, 1610, 1582, 1367 $cm^{-1}$;
$^1$H-NMR ($D_2O$) $\delta$ 5,61 (d,1 J=1,3), 4,03 (m,1), 4,00 (m,1), 3,95 (m,2), 3,38 (dd,1, J=6,2 und 14,3), 2,69 (dd,1, J=4,8 und 14,3), 2,44 (m,1), 2,38 (m,1), 2,31 (m,1), 1,90 (m,1) ppm.

Beispiel 9:
2-[(3S,4R)-3-[(1'R)-1'-Allyloxycarbonyloxyäthyl]-4-[(2S)-5-oxo-pyrrolidin-2-ylacetylthio]-2-oxo-azetidin--1-yl]-2-triphenylphosphoranylidenessigsäureallylester

Analog Beispiel 1 wird aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-5-Oxo-pyrrolidin-2-ylacetylchlorid (hergestellt aus der freien Säure mit $SOCl_2$ ) die Titelverbindung hergestellt.
IR (3 % $CH_2Cl_2$ ): 3420; 2860-3500, 1756, 1701 $cm^{-1}$.

Die Ausgangsverbindung kann wie folgt hergestellt werden:

a) (3S,4R)-3-[(1'R)-1'-Allyloxycarbonyloxyäthyl]-4-triphenylmethylthio-azetidin-2-on

7,22 g Triphenylmethylmercaptan werden in 40 ml Methanol bei 0° suspendiert und über 10 Minuten portionsweise mit insgesamt 1,29 g einer 55%igen Natriumhydrid-Suspension in Oel versetzt. Anschliessend wird eine Emulsion von 6,95 g (3S,4R)-3-[(1'R)-1'-Allyoxycarbonyloxyäthyl]-4-tert.butylsulfonyl-azetidin-2-on (Europäische Patentanmeldung Nr. 126709) in 40 ml Aceton und 40 ml Wasser über 30 Minuten zugetropft. Nach 30 Minuten Rühren bei 0° und 1 Stunde Rühren bei Raumtemperatur wird das Reaktionsgemisch am Rotationsverdampfer eingeengt, mit Methylenchlorid versetzt, und die wässrige Phase wird abgetrennt. Die organische Lösung wird mit Sole gewaschen und über Natriumsulfat getrocknet. Nach Einengen wird die rohe Titelverbindung durch Chromatographie auf Silicagel (Laufmittel Toluol/Aethylacetat 2:3) gereinigt.
DC (Toluol-Aethylacetat 2:3): $R_f$ = 0,59; IR ($CH_2Cl_2$ ): 3390, 1760 $cm^{-1}$.

b)
2-[(3-S,4R)-3-[(1'R)-1'-Allyloxycarbonyloxyäthyl]-4-triphenylmethylthio-2-oxo-azetidin-1-yl]-2-hydroxyessigsäureallylester

7,95 g (3S,4R)-3-[(1'R)-1'-Allyloxycarbonyloxyäthyl]-4-triphenylmethylthio-azetidin-2-on und 4,03 g Glyoxylsäureallylester-äthylhemiacetal in 100 ml abs. Toluol werden mit 40 g Molekularsieb (4Å) versetzt und während 94 Stunden bei 60° gerührt. Nach Abfiltrieren und Einengen am Rotationsverdampfer unter vermindertem Druck wird das Rohprodukt durch Chromatographie an Silicagel gereinigt (Laufmittel Toluol/Aethylacetat 4:1); DC (Silicagel, Toluol/Aethylacetat 4:1); $R_f$ = 0,22 und 0,16; IR ($CH_2Cl_2$): 3521, 1760, 1745 $cm^{-1}$.

c)
2-[(3S,4R)-3-[(1'R)-1'-Allyloxycarbonyloxyäthyl]-4-triphenylmethylthio-2-oxo-azetidin-1-yl]-2-triphenylphosphoranylidenessigsäure-allylester

Zu einer Lösung von 1,0 g 2-[(3S,4R)-3-[(1'R)-1'-Allyloxycarbonyloxyäthyl]-4-triphenylmethylthio-2-oxo-azetidin-1-yl]-2-hydroxyessig-säure-allylester in 10 ml Tetrahydrofuran werden unter Rühren bei -15° nacheinander 182 µl Thionylchlorid und 206 µl Pyridin innert 5 Minuten zugegeben. Die weisse Suspension wird 1 Stunde bei -10° nachgerührt und über Hyflo filtriert. Nach dem Waschen des Rückstandes mit Toluol wird am Rotationsverdampfer eingeengt. Der Rückstand wird in 10 ml Dioxan gelöst, mit 624 mg Triphenylphosphin und 0,257 ml 2,6-Lutidin versetzt und während 46 Stunden bei 80° Badtemperatur gerührt. Das Gemisch wird über Hyflo filtriert und dieser Rückstand mit Toluol nachgewaschen. Die vereinigten Filtrate werden eingedampft. Die Chromatographie des Rückstandes an Silicagel ergibt das reine Produkt (Laufmittel Toluol/Aethylacetat 4:1); DC (Silicagel, Toluol-Aethylacetat 4:1): $R_f$ = 0,20; IR ($CH_2Cl_2$): 1745, 1605 $cm^{-1}$.

d)
2-[(3S,4R)-3-[(1'R)-1'-Allyloxycarbonyloxyäthyl]-4-mercapto-2-oxo-azetidin-1-yl]-2-triphenylphosphoranylidenessigsäure-allylester-Silbersalz

5,31 g 2-[(3S,4R)-3-[(1'R)-1'-Allyloxycarbonyloxyäthyl]-4-triphenylmethylthio-2-oxo-azetidin-1-yl]-2-triphenylphosphoranylidenessigsäure-allylester werden in 63 ml Aether vorgelegt und bei Raumtemperatur mit 50 ml einer 0,5 M wässrigen Silbernitrat-Lösung versetzt. Danach gibt man tropfenweise 14,7 ml eines Gemisches aus 3,6 ml Tributylamin, 0,18 ml Trifluoressigsäure und 25 ml Aether dazu und rührt das Reaktionsgemisch weitere 20 Minuten. Dann wird der Feststoff abgenutscht und mit Aether, Wasser und erneut mit Aether gewaschen. Der Feststoff wird zur Reinigung in 40 ml Aether und 40 ml Wasser aufgeschlämmt, abgenutscht und getrocknet. IR ($CH_2Cl_2$): 1760, 1621 $cm^{-1}$.

Beispiel 10:
(5R,6S)-6-[(1'R)-1'-Allyloxycarbonyloxyäthyl]-2-[(2S)-5-oxo-pyrrolidin-2-ylmethyl]-2-penem-3-carbonsäureallylester

2-[(3S,4R)-3-[(1'R)-1'-Allyloxycarbonyloxyäthyl]-4-[(2S)-5-oxo-pyrrolidin-2-ylacetylthio]-2-oxo-azetidin-1-yl]-1-triphenylphosphoranylidenessigsäureallylester wird in analoger Weise, wie in Beispiel 2 beschrieben, in die Titelverbindung überführt.
IR (3 % $CH_2Cl_2$): 3420, 2820-3460, 1791, 1701 $cm^{-1}$;
$^1H$-NMR ($CDCl_3$) δ 5.92 (m,2), 5,91 (s,1), 5,60 (d,1, J=1,5), 5,41 (dd,1, J=1,3 und 14,2), 5,36 (dd,1, J=1,3 und 14,2), 5,29 (dd,1, J=1,3 und 9,5), 5,26 (dd,1, J=1,3 und 8,9), 5,12 (m,1), 4,78 (ddd, 1, J=1,0, 5,0 und 11,2), 4,68 (ddd,1, J=1,0, 5,0 und 11,2), 4,64 (dd,2, J=1,0 und 11,2), 3,89 (m,1) 3,88 (dd,1, J=1,5 und 7,0), 3,31 (dd,1, J=5,5 und 14,5), 2,72 (dd,1, J=6,2 und 14,5), 2,35 (m,2), 2,33 (m,1), 1,86 (m,1), 1,46 (d,3, J=6,4)

Beispiel 11: (5R,6S)-6-[(1'R)-1'-Hydroxyäthyl]-2-[(2S)-5-oxo-pyrrolidin-2-ylmethyl]-2-penem-3-carbonsäure, Natriumsalz

Analog Beispiel 4 erhält man ausgehend vom (5R,6S)-6-[(1'R)-1'-Allyloxycarbonyloxyäthyl]-2-[(2S)-5-oxo-pyrrolidin-2-ylmethyl]-2-penem-3-carbonsäureallylester die Titelverbindung
$\alpha_D$ (0,164 % $H_2O$) 100,7°;
UV ($10^{-4}$ % $H_2O$) $\lambda_{max}$ = 261; 305 nm;
IR (3 % DMSO-$d_6$): 2800-3650, 1770, 1690, 1611, 1581, 1365 $cm^{-1}$.
$^1H$-NMR ($D_2O$) δ 5,63 (d,1, J=1,5), 4,24 (m,1), 4,03 (m,1), 3,86 (dd,1, J=1,5 und 6,1), 3,09 (dd,1, J=6,3 und 14,2), 3,03 (dd,1, J=5,8 und 14,2), 2,39 (m,2), 2,34 (m,1), 1,92 (m,1), 1,31 (d,3, J=6,4).

Beispiel 12:
2-[(3S,4R)-3-[(1'R)-1'-Allyloxycarbonyloxyäthyl]-4-[(2R)-5-oxo-pyrrolidin-2-ylacetylthio]-2-oxo-azetidin--1-yl]-2-triphenylphosphoranylidenessigsäureallylester

Analog Beispiel 1 wird aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2R)-5-Oxo-pyrrolidin-2-ylacetylchlorid (hergestellt aus der freien Säure mit $SOCl_2$) die Titelverbindung hergestellt.
IR (3 % $CH_2Cl_2$): 3420, 2850-3650, 1752, 1702 $cm^{-1}$.

Beispiel 13:
(5R,6S)-6-[(1'R)-1'-Allyloxycarbonyloxyäthyl]-2-[(2R)-5-oxo-pyrrolidin-2-ylmethyl]-2-penem-3-carbonsäureallylester

2-[(3S,4R)-3-[(1'R)-1'-Allyloxycarbonyloxyäthyl]-4-[(2R)-5-oxo-pyrrolidin-2-ylacetylthio]-2-oxo-azetidin-1-yl]-1-triphenylphosphoranylidenessigsäureallylester wird, wie in Beispiel 2 beschrieben, in die Titelverbin-

dung überführt.
IR (3 % $CH_2$ $Cl_2$ ): 3420, 2800-3500, 1790, 1702 $cm^{-1}$.

Beispiel 14: (5R,6S)-6-[(1'R)-1'-Hydroxyäthyl]-2-[(2R)-5-oxo-pyrrolidin-2-ylmethyl]-2-penem-3-carbonsäure, Natriumsalz

Analog Beispiel 4 erhält man durch Abspaltung aller Schutzgruppen aus (5R,6S)-6-[(1'R)-1'-Allyloxycarbonyloxyäthyl]-2-[(2R)-5-oxo-pyrrolidin-2-ylmethyl]-2-penem-3-carbonsäureallylester die Titelverbindung.
$\alpha_D$ (0,199 % $H_2O$) 163,5°;
UV ($10^{-4}$ % $H_2O$) $\lambda_{max}$ = 261; 305 nm;
IR (3 % DMSO-$d_6$ ): 2800-3650, 1769, 1690, 1610, 1581, 1367 $cm^{-1}$.
$^1$H-NMR ($D_2O$) $\delta$ 5,62 (d,1 J=1,4), 4,25 (m,1), 4,03 (m,1), 3,87 (dd,1, J=1,4 und 6,0), 3,37 (dd,1, J=6,8 und 14,4), 2,71 (dd,1, J=5,3 und 14,4), 2,38 (m,2), 2,34 (m,1), 1,91 (m,1), 1,31 (d,3, J=6,5).

Beispiel 15:
2-[(3S,4R)-3-(tert.Butyldimethylsilyloxymethyl)-4-[(2S)-1-methyl-5-oxo-pyrrolidin-2-ylacetylthio]-2-oxo-azetidin-1-yl]-2-triphenylphosphoranylidenessigsäureallylester.

Analog Beispiel 1 wird aus dem Silbersalz des 2-[(3S,4R)-3-(tert.-Butyldimethylsilyloxymethyl)-4-mercapto-2-oxo-azetidin-1-yl]-2-triphenylphosphoranylidenessigsäureallylesters und (2S)-1-Methyl-5-oxo-pyrrolidin-2-ylacetylchlorid (hergestellt aus der Säure mit $SOCl_2$ ) die Titelverbindung hergestellt.
IR (3 % $CH_2$ $Cl_2$ ): 2850-3510, 1756, 1702, 838 $cm^{-1}$.

Beispiel 16:
(5R,6S)-6-(tert.Butyldimethylsilyloxymethyl)-2-[(2S)-1-methyl-5-oxo-pyrrolidin-2-ylmethyl]-2-penem-3-carbonsäureallylester

Analog Beispiel 2 wird 2-[(3S,4R)-3-(tert.Butyldimethylsilyloxymethyl)-4-[(2S)-1-methyl-5-oxo-pyrrolidin-2-ylacetylthio]-2-oxo-azetidin-1-yl]-2-triphenylphosphoranylidenessigsäureallylester zur Titelvervingung cyclisiert.
IR (3 % $CH_2$ $Cl_2$ ): 2820-3550, 1795, 1710, 838 $cm^{-1}$.

Beispiel 17:
(5R,6S)-6-Hydroxymethyl-2-[(2S)-1-methyl-5-oxo-pyrrolidin-2-ylmethyl]-2-penem-3-carbonsäureallylester

(5R,6S)-6-(tert.Butyldimethylsilyloxymethyl)-2-[(2S)-1-methyl-5-oxo-pyrrolidin-2-ylmethyl]-2-penem-3-carbonsäureallylester wird analog Beispiel 3 zur Titelverbindung umgesetzt.
IR (3 % $CH_2$ $Cl_2$ ): 2750-3550, 1795, 1710, 1685 $cm^{-1}$.

Beispiel 18: (5R,6S)-6-Hydroxymethyl-2-[(2S)-1-methyl-5-oxo-pyrrolidin-2-ylmethyl]-2-penem-3-carbonsäure, Natriumsalz

Analog Beispiel 4 erhält man aus (5R,6S)-6-Hydroxymethyl-2-[(2S)-1-methyl-5-oxo-pyrrolidin-2-ylmethyl]-2-penem-3-carbonsäureallylester die Titelverbindung.
UV ($10^{-4}$ % $H_2O$): $\lambda_{max}$ = 260; 305 nm;
IR (3 % DMSO-$d_6$ ): 2800-3650, 1769, 1680 $cm^{-1}$.

Beispiel 19:
2-[(3S,4R)-3-(tert.Butyldimethylsilyloxymethyl)-4-[(2R)-1-methyl-5-oxo-pyrrolidin-2-ylacetylthio]-2-oxo-azetidin-1-yl]-2-triphenylphosphoranylidenessigsäureallylester

Analog Beispiel 1 wrid aus dem Silbersalz des 2-[(3S,4R)-3-(tert.-Butyldimethylsilyloxymethyl)-4-mercapto-2-oxo-azetidin-1-yl]-2-triphenylphosphoranylidenessigsäureallylesters und (2R)-1-Methyl-5-oxo-pyrrolidin-2-ylacetylchlorid (hergestellt aus der freien Säure mit $SOCl_2$ ) die Titelverbindung hergestellt.
IR (3 % $CH_2$ $Cl_2$ ): 2800-3550, 1760, 1685, 837 $cm^{-1}$.

Beispiel 20:
(5R,6S)-6-(tert.Butyldimethylsilyloxymethyl)-2-[(2R)-1-methyl-5-oxo-pyrrolidin-2-ylmethyl]-2-penem-3-carbonsäureallylester

Analog Beispiel 2 wird 2-[(3S,4R)-3-(tert.Butyldimethylsilyloxymethyl)-4-[(2R)-1-methyl-5-oxo-pyrrolidin-2-ylacetylthio]-2-oxo-azetidin-1-yl]-2-triphenylphosphoranylidenessigsäureallylester zur Titelverbindung cyclisiert.
IR (3 % $CH_2$ $Cl_2$ ): 2750-3600, 1790, 1706, 837 $cm^{-1}$.

Beispiel 21:
(5R,6S)-6-Hydroxymethyl-2-[(2R)-1-methyl-5-oxo-pyrrolidin-2-ylmethyl]-2-penem-3-carbonsäureallylester

Analog Beispiel 3 wird (5R,6S)-6-(tert.Butyldimethylsilyloxymethyl)-2-[(2R)-1-methyl-5-oxo-pyrrolidin-2-ylmethyl]-2-penem-3-carbonsäureallylester in die Titelverbindung überführt.
IR (3 % $CH_2$ $CL_2$ ): 2750-3600, 1790, 1685 $cm^{-1}$.

Beispiel 22: (5R,6S)-6-Hydroxymethyl-2-[(2R)-1-methyl-5-oxo-pyrrolidin-2-ylmethyl]-2-penem-3-carbonsäure, Natriumsalz

Analog Beispiel 4 erhält man aus (5R,6S)-6-Hydroxymethyl-2-[(2R)-1-methyl-5-oxo-pyrrolidin-2-ylmethyl]-2-penem-3-carbonsäureallylester die Titelverbindung.
UV ($10^{-4}$ % $H_2O$) $\lambda_{max}$ = 262; 303 nm;
IR (3 % DMSO-$d_6$): 2800-3650, 1790, 1680 cm $^{-1}$.

Beispiel 23: Analog Beispiel 1 werden die folgenden Verbindungen hergestellt:

Aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-Methyl-5-oxo-pyrrolidin-2-ylacetylchlorid (hergestellt aus der Säure mit $SOCl_2$):
2-[(3S,4R)-3-[(1'R)-1'-Allyloxycarbonyloxyäthyl]-4-[(2S)-1-methyl-5-oxo-pyrrolidin-2-ylacetylthio]-2-oxo-azetidin-1-yl]-2-triphenylphosphoranylidenessigsäureallylester,
IR (4 % $CH_2Cl_2$): 3670, 2800-3550, 1755, 1688, 1620, 1242, 1059, 1000, 825 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2R)-1-Methyl-5-oxo-pyrrolidin-2-ylacetylchlorid (hergestellt aus der freien Säure mit $SOCl_2$): 2-[(3S,4R)-3-[(1'R)-1'-Allyloxycarbonyloxyäthyl]-4-[(2R)-1-methyl-5-oxo-pyrrolidin-2-ylacetylthio]-2-oxo-azetidin-1-yl]-2-triphenylphosphoranylidenessigsäureallylester,
IR (3 % $CH_2Cl_2$): 3680, 2800-3600, 1760, 1750, 1685, 1620, 1435, 1250, 1110, 685 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (2S)-6-Oxo-piperidin-2-ylacetylchlorid:
2-[(3S,4R)-3-(tert.Butyldimethylsilyloxymethyl)-4-[(2S)-6-oxo-piperidin-2-ylacetylthio]-2-oxo-azetidin-1-yl]-2-triphenylphosphoranylidenessigsäureallylester,
IR (3 % $CH_2Cl_2$): 3422, 2820-3100, 1756, 1680, 839 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (2R)-6-Oxo-piperidin-2-ylacetylchlorid:
2-[(3S,4R)-3-(tert.Butyldimethylsilyloxymethyl)-4-[(2R)-6-oxo-piperidin-2-ylacetylthio]-2-oxo-azetidin-1-yl]-2-triphenylphosphoranylidenessigsäureallylester,
IR (3 % $CH_2Cl_2$): 3421, 2850-3450, 1756, 1681, 837 cm $^{-1}$;
aus dem Silbersalz des 2-[(3S,4R)-3-[(1'R)-1'-Allyloxycarbonyloxyäthyl-4-mercapto-2-oxo-azetidin-1-yl]-2-triphenylphosphoranylidenessigsäureallylesters und (2S)-6-Oxo-piperidin-2-ylacetylchlorid:
2-[(3S,4R)-3-[(1'R)-1'-Allyloxycarbonyloxyäthyl]-4-[(2S)-6-oxo-piperidin-2-ylacetylthio]-2-oxo-azetidin--1-yl]-2-triphenylphosphoranylidenessigsäureallylester,
IR (3 % $CH_2Cl_2$): 3381, 2850-3200, 1757, 1660, 1240, 1110, 950 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2R)-6-Oxo-piperidin-2-ylacetylchlorid:
2-[(3S,4R)-3-[(1'R)-1'-Allyloxycarbonyloxyäthyl]-4-[(2R)-6-oxo-piperidin-2-ylacetylthio]-2-oxo-azetidin--1-yl]-2-triphenylphosphoranylidenessigsäureallylester,
IR ($CH_2Cl_2$): 3422, 2890-3420, 1756, 1681 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (2S)-4-Oxo-azetidin-2-ylacetylchlorid:
2-[(3S,4R)-3-(tert.Butyldimethylsilyloxymethyl)-4-[(2S)-4-oxo-azetidin-2-ylacetylthio]-2-oxo-azetidin-1-yl]-2-triphenylphosphoranylidenessigsäureallylester,
IR (3 % $CH_2Cl_2$): 3620, 2800-3600, 1762, 839 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (2R)-4-Oxo-azetidin-2-ylacetylchlorid:
2-[(3S,4R)-3-(tert.Butyldimethylsilyloxymethyl)-4-[(2R)-4-oxo-azetidin-2-ylacetylthio]-2-oxo-azetidin-1-yl]-2-triphenylphosphoranylidenessigsäureallylester,
IR (3 % $CH_2Cl_2$): 3620, 2830-3580, 1756, 840 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-4-Oxo-azetidin-2-ylacetylchlorid:
2-[(3S,4R)-3-[(1'R)-1'-Allyloxycarbonyloxyäthyl]-4-[(2S)-4-oxo-azetidin-2-ylacetylthio]-2-oxo-azetidin-1-yl]-2-triphenylphosphoranylidenessigsäureallylester
IR (3 % $CH_2Cl_2$): 3620, 2800-3600, 1762, 1679, 1621, 1439, 1375, 1245, 1110, 630 cm $^{-1}$;
aus dem Silbersalz des 2-[(3S,4R)-3-[(1'R)-1'-Allyoxycarbonyloxyäthyl]-4-mercapto-2-oxo-azetidin-1-yl]-2-triphenylphosphoranylidenessigsäureallylesters und (2R)-4-Oxo-azetidin-2-ylacetylchorid:
2-[(3S,4R)-3-[(1'R)-1'-Allyloxycarbonyloxyäthyl]-4-[(2R)-4-oxo-azetidin-2-ylacetylthio]-2-oxo-azetidin-1-yl]-2-triphenylphosphoranyliden-essigsäureallylester,
IR (3 % $CH_2Cl_2$): 3620, 2800-3600, 1756, 842 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessig-säureallylesters und 3-[(2S)-5-Oxo-pyrrolidin-2-yl]-propionylchlorid:
2-[(3S,4R)-3-(tert.Butyldimethylsilyloxymethyl)-4-[3-[(2S)-5-oxo-pyrrolidin-2-yl]-propionylthio]-2-oxo-azetidin-1-yl]-2-triphenylphosphoranylidenessigsäureallylester,
IR (3 % $CH_2Cl_2$): 3421, 2850-3510, 1756, 1695, 838 cm $^{-1}$;
aus dem Silbersalz des 2-[(3S,4R)-2-[(1'R)-1'-Allyloxycarbonyloxyäthyl]-4-mercapto-2-oxo-azetidin-1-yl]-2-triphenylphosphoranylidenessigsäureallylesters und 3-[(2S)-5-Oxo-pyrrolidin-2-yl]-propionylchlorid: 2-[(3S,4R)-3-[(1'R)-1'-Allyloxycarbonyloxyäthyl]-4-[3-[(2S)-5-oxo-pyrrolidin-2-yl]-propionylthio]-2-oxo-azetidin-1-yl]-2-triphenylphosphoranylidenessigsäureallylester,
IR (3 % $CH_2Cl_2$): 3420, 2800-3500, 1755, 1695, 1620, 1438, 1245, 1110 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (3S)-2-Oxo-pyrolidin-3-ylacetylchlorid: 2-[(3S,4R)-3-(tert.Butyldimethylsilyloxymethyl)-4-[(3S)-2-oxo-pyrrolidin-3-ylacetylthio]-2-oxo-azetidin-1-yl]-2-triphenylphosphoranylidenessigsäureallylester,
IR (3 % $CH_2Cl_2$): 3421, 2860-3520, 1756, 1704, 840 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (3S)-2-Oxo-pyrrolidin-3-ylacetylchlorid: 2-[(3S,4R)-3-[(1'R)-1'-Allyloxycarbonyloxyäthyl]-4-[(3S)-2-oxo-pyrrolidin-3-ylacetylthio]-2-oxo-azetidin--1-yl]-2-triphenylphosphor anylidenessigsäureallylester,
IR (3 % $CH_2Cl_2$): 3418, 2850-3505, 1756, 1704 cm $^{-1}$;
aus dem Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und (4S)-2-Oxo-pyrrolidin-4-ylacetylchlorid: 2-[(3S,4R)-3-(tert.Butyldimethylsilyloxymethyl)-4-(4S)-2-oxo-pyrrolidin-4-ylacetylthio]-2-oxo-azetidin-1-yl]-2-triphenylphosphoranylidenessigsäureallylester,
IR (3 % $CH_2Cl_2$): 3420, 2855-3515, 1754, 1700, 838 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (4S)-2-Oxo-pyrrolidin-4-ylacetylchlorid: 2-[(3S,4R)-3-[(1'R)-1'-Allyloxycarbonyloxyäthyl]-4-[(4S)-2-oxo-pyrrolidin-4-ylacetylthio]-2-oxo-azetidin--1-yl]-2-triphenylphosphoranylidenessigsäureallylester,
IR (3 % $CH_2Cl_2$): 3422, 2860-3490, 1758, 1704 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-Allyl-5-oxo-pyrrolidin-2-yl-acetylchlorid: 2-[3S,4R)-3-[(1'R)-1'-Allyloxycarbonyloxyäthyl]-4-[(2S)-1-allyl-5-oxo-pyrrolidin-2-ylacetylthio]-2-oxo-azetidinyl]-2-triphenylphosphoranylidenessigsäureallylester,
IR (4 % $CH_2Cl_2$): 2800-3140, 1760, 1689, 1620, 1440, 1245, 1110, 1000 cm $^{-1}$;
aus dem Silbersalz des 2-[(3S,4R)-3-[(1'R)-1'-Allyloxycarbonyloxyäthyl-4-mercapto-2-oxo-azetidin-1-yl]-2-triphenylphosphoranylidenessigsäureallylesters und (2S)-2,5-Dihydro-5-oxo-pyrrol-2-ylacetylchlorid: 2-[(3S,4R)-2-[(1'R)-1'-Allyloxycarbonyloxyäthyl]-4-[(2S)-2,5-dihydro-5-oxo-pyrrol-2-ylacetylthio]-2-oxo-azetidin-1-yl]-2-triphenylphosphoranylidenessigsäureallylester,
IR (5 % $CH_2Cl_2$): 3424, 2820-3550, 1758, 1715, 1110 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-(2-Allyloxycarbonyläthyl)-5-oxo-pyrrolidin-2-ylacetylchlorid: 2-[(3S,4R)-3-[(1'R)-1'-Allyloxycarbonyloxyäthyl]-4-[(2S)-1-(2-allyloxycarbonyläthyl)-5-oxo-pyrrolidin-2-ylacetylthio]-2-oxo-azetidin-1-yl]-2-triphenylphosphoranylidenessigsäureallylester,
IR (3 % $CH_2Cl_2$): 2800-3150, 1760, 1740, 1685, 1620, 1445, 1370, 1240, 1110, 1000 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-(2-Allyloxycarbonylaminoäthyl)-5-oxo-pyrrolidin-2-yl-acetylchlorid: 2-[(3S,4R)-3-[(1'R)-1'-Allyloxycarbonyloxyäthyl]-4-[(2S)-1-(2-allyloxycarbonylaminoäthyl)-5-oxo-pyrrolidin-2-ylacetylthio]-2-oxo-azetidin-1-yl]-2-triphenylphosphoranylidenessigsäureallylester,
IR (3 % $CH_2Cl_2$): 3650, 2800-3150, 1760, 1686, 1620, 1110, 1000 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-(2-Allyloxycarbonyloxyäthyl)-5-oxo-pyrrolidin-2-ylacetylchlorid: 2-[(3S,4R)-3-[(1'R)-1'-Allyloxycarbonyloxyäthyl]-4-[(2S)-1-(2-allyloxycarbonyloxyäthyl)-5-oxo-pyrrolidin-2-ylacetylthio]-2-oxo-azetidin-1-yl]-2-triphenylphosphoranylidenessigsäureallylester,
IR (5 % $CH_2Cl_2$): 2800-3200, 1760, 1687, 1620, 1110 cm $^{-1}$;
aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-(2-Cyanoäthyl)-5-oxo-pyrrolidin-2-yl-acetylchlorid: 2[(3S,4R)-3-[(1'R)-1'-Allyloxycarbonyloxyäthyl]-4-[(2S)-1-(2-cyanoäthyl)-5-oxo-pyrrolidin-2-ylacetylthio]-2-oxo-azetidin-1-yl]-2-triphenylphosphoranylidenessigsäureallylester, IR (3 % $CH_2Cl_2$): 2800-3180, 2252, 1761, 1685, 1620, 1110 cm $^{-1}$.

Beispiel 24: Analog Beispiel 2 werden die folgenden Verbindungen hergestellt:
Aus 2-[(3S,4R)-3-[(1'R)-1'-Allyloxycarbonyloxyäthyl]-4-[(2S)-1-methyl-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-1-methyl-5-oxo-pyrrolidin-2-yl-methyl]-2-penem-3-carbonsäureallylester,
$\alpha_D$ (0,464 % Aethanol): 48,9°;
IR (4 % $CH_2Cl_2$): 3680, 2800-3550, 1795, 1740, 1710, 1685, 1580, 1375, 1330, 1245 cm $^{-1}$;
aus 2-[(3S,4R)-3-[(1'R)-1'-Allyloxycarbonyloxyäthyl]-4-[(2R)-1-methyl-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2R)-1-methyl-5-oxo-pyrrolidin-2-ylmethyl]-2-penem-3-carbonsäureallylester,
$\alpha_D$ (0,755 % Aethanol): 157,4°;

IR (3 % $CH_2$ $Cl_2$ ): 3680, 2750-3600, 1790, 1745, 1705, 1685, 1310, 1245 cm $^{-1}$;
aus 2-[(3S,4R)-3-(tert.Butyldimethylsilyloxymethyl)-4-[(2S)-6-oxo-piperidin-2-ylacetylthio]-2-oxo-azetidin-1-yl]-2-triphenylphosphoranylidenessigsäureallylester der (5R,6S)-6-(tert.Butyldimethylsilyloxymethyl)-2-[(2S)-6-oxo-piperidin-2-ylmethyl]-2-penem-3-carbonsäureallylester,
IR (3 % $CH_2$ $Cl_2$ ): 3680, 2800-3550, 1795, 1740, 1685 cm $^{-1}$;
aus 2-[(3S,4R)-3-(tert.Butyldimethylsilyloxymethyl)-4-[(2R)-6-oxo-piperidin-2-ylacetylthio]-2-oxo-azetidin-1-yl]-2-triphenylphosphoranylidenessigsäureallylester der (5R,6S)-6-(tert.Butyldimethylsilyloxymethyl)-2-[(2R)-6-oxo-piperidin-2-ylmethyl]-2-penem-3-carbonsäureallylester,
IR (3 % $CH_2$ $Cl_2$ ): 3680, 2750-3600, 1789, 1685, 836 cm $^{-1}$;
aus 2-[(3S,4R)-3-[(1'R)-1'-Allyloxycarbonyloxyäthyl]-4-[(2S)-6-oxo-piperidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-6-oxo-piperidin-2-ylmethyl]-2-penem-3-carbonsäureallylester,
IR (3 % $CH_2$ $Cl_2$ ): 3381, 2850-3400, 1790, 1755, 1710, 1580, 1310, 1250, 1175, 790 cm $^{-1}$;
aus 2-[(3S,4R)-3-[(1'R)-1'-Allyloxycarbonyloxyäthyl]-4-[(2R)-6-oxo-piperidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2R)-6-oxo-piperidin-2-ylmethyl]-2-penem-3-carbonsäureallylester,
IR (3 % $CH_2$ $Cl_2$ ): 3421, 2850-3400, 1788, 1680 cm $^{-1}$;
aus 2-[(3S,4R)-3-(tert.Butyldimethylsilyloxymethyl)-4-[(2S)-4-oxo-azetidin-2-ylacetylthio]-2-oxo-azetidin-1-yl]-2-triphenylphosphoranylidenessigsäureallylester der (5R,6S)-6-(tert.Butyldimethylsilyloxymethyl)-2-[(2S)-4-oxo-azetidin-2-ylmethyl]-2-penem-3-carbonsäureallylester,
IR (3 % $CH_2$ $Cl_2$ ): 3500, 2830-3500, 1789, 1750, 839 cm $^{-1}$;
aus 2-[(3S,4R)-3-(tert.Butyldimethylsilyloxymethyl)-4-[(2R)-4-oxo-azetidin-2-ylacetylthio]-2-oxo-azetidin-1-yl]-2-triphenylphosphoranylidenessigsäureallylester der (5R,6S)-6-(tert.Butyldimethylsilyloxymethyl)-2-[(2R)-4-oxo-azetidin-2-ylmethyl]-2-penem-3-carbonsäureallylester,
IR (3 % $CH_2$ $Cl_2$ ): 3500, 2830-3500, 1790, 1753, 840 cm $^{-1}$;
aus 2-[(3S,4R)-3-[(1'R)-1'-Allyloxycarbonyloxyäthyl]-4-[(2S)-4-oxo-azetidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-4-oxo-azetidin-2-ylmethyl]-2-penem-3-carbonsäureallylester,
IR (5 % $CH_2$ $Cl_2$ ): 3500, 2830, 1790, 1768, 1750, 1710, 1680, 1365, 1315, 1245 cm $^{-1}$;
aus 2-[(3S,4R)-3-[(1'R)-1'-Allyloxycarbonyloxyäthyl]-4-[(2R)-4-oxo-azetidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2R)-4-oxo-azetidin-2-ylmethyl]-2-penem-3-carbonsäureallylester,
IR (3 % $CH_2$ $Cl_2$ ): 3500, 2830-3500, 1787, 1755, 839 cm $^{-1}$;
aus 2-[(3S,4R)-3-(tert.Butyldimethylsilyloxymethyl)-4-[(3-(2S)-5-oxo-pyrrolidin-2-yl]-propionylthio]-2-oxo-azetidin-1-yl]-2-triphenylphosphoranylidenessigsäureallylester der (5R,6S)-6-(tert.-Butyldimethylsilyloxymethyl)-2-[2-(2S)-5-oxo-pyrrolidin-2-yl]-äthyl]-2-penem-3-carbonsäureallylester,
IR (3 % $CH_2$ $Cl_2$ ): 3421, 2820-3310, 1790, 1705 cm $^{-1}$;
aus 2-[(3S,4R)-3-[(1'R)-1'-Allyloxycarbonyloxyäthyl]-4-[3-(2S)-5-oxo-pyrrolidin-2-yl]-propionylthio]-2-oxo-azetidin-1-yl]-2-triphenylphosphoranylidenessigsäureallylester der (5R,6S)-6-[(1'R)-1'-Allyloxycarbonyloxyäthyl]-2-[2-(2S)-5-oxo-pyrrolidin-2-yl]-äthyl]-2-penem-3-carbonsäureallylester,
IR (5 % $CH_2$ $Cl_2$ ): 3415, 2800-3300, 1790, 1749, 1700, 1650, 1580, 1375, 1315, 1248 cm $^{-1}$;
aus 2-[(3S,4R)-3-(tert.Butyldimethylsilyloxymethyl)-4-[(3S)-2-oxo-pyrrolidin-3-ylacetylthio]-2-oxo-azetidin-1-yl]-2-triphenylphosphoranylidenessigsäureallylester der (5R,6S)-6-(tert.Butyldimethylsilyloxymethyl)-2-[(3S)-2-oxo-pyrrolidin-3-ylmethyl]-2-penem-3-carbonsäureallylester,
IR (3 % $CH_2$ $Cl_2$ ): 3420, 2820-3315, 1790, 836 cm $^{-1}$;
aus 2-[(3S,4R)-3-[(1'R)-1'-Allyloxycarbonyloxyäthyl]-4-[(3S)-2-oxo-pyrrolidin-3-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(3S)-2-oxo-pyrrolidin-3-ylmethyl]-2-penem-3-carbonsäureallylester,
IR (3 % $CH_2$ $Cl_2$ ): 3422, 2815-3250, 1791, 1705 cm $^{-1}$;
aus 2-[(3S,4R)-3-(tert.Butyldimethylsilyloxymethyl)-4-[(4S)-2-oxo-pyrrolidin-4-ylacetylthio]-2-oxo-azetidin-1-yl]-2-triphenylphosphoranylidenessigsäureallylester der (5R,6S)-6-(tert.Butyldimethylsilyloxymethyl)-2-[(4S)-2-oxo-pyrrolidin-4-ylmethyl]-2-penem-3-carbonsäureallylester,
IR (3 % $CH_2$ $Cl_2$ ): 3410, 2820-3300, 1787, 1707, 836 cm $^{-1}$;
aus 2-[(3S,4R)-3-[(1'R)-1'-Allyloxycarbonyloxyäthyl]-4-[(4S)-2-oxo-pyrrolidin-4-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(4S)-2-oxo-pyrrolidin-4-ylmethyl]-2-penem-3-carbonsäureallylester,
IR (3 % $CH_2$ $Cl_2$ ): 3415, 2820-3315, 1788, 1705 cm $^{-1}$;
aus 2-[(3S,4R)-3-[(1'R)-1'-Allyloxycarbonyloxyäthyl]-4-[(2S)-1-allyl-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-1-allyl-5-oxo-pyrrolidin-2-yl-methyl]-2-penem-3-carbonsäureallylester,
IR (5 % $CH_2$ $Cl_2$ ): 2800-3140, 1790, 1745, 1710, 1690, 1650, 1580, 1370, 1315, 1245, 1175, 940 cm $^{-1}$;
aus 2-[(3S,4R)-3-[(1'R)-1'-Allyloxycarbonyloxyäthyl]-4-[(2S)-2,5-dihydro-5-oxo-pyrrol-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-4-[(2S)-2,5-dihydro-5-oxo-pyrrol-2-yl-methyl]-2-penem-3-carbonsäureallylester,
IR (5 % $CH_2$ $Cl_2$ ): 3420, 2820-3550, 1789, 1715, 1170 cm $^{-1}$;

aus 2-[(3S,4R)-3-[(1'R)-1'-Allyloxycarbonyloxyäthyl]-4-[(2S)-1-(2-allyloxycarbonyläthyl)-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonloxyäthyl]-2-(2S)-1-(2-allyloxycarbonyläthyl)-5-oxo-pyrrolidin-2-ylmethyl]-2-penem-3-carbonsäureallylester,
IR (3 % $CH_2$ $Cl_2$ ): 2800-3150, 1790, 1740, 1700, 1685, 1575, 1445, 1365, 1310, 1240, 1200 1170 cm $^{-1}$;
aus 2-[(3S,4R)-3-[(1'R)-1'-Allyloxycarbonyloxyäthyl]-4-[(2S)-1-(2-allyloxycarbonylaminoäthyl)-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-1-(2-allyloxycarbonylaminoäthyl)-5-oxo-pyrrolidin-2-ylmethyl]-2-penem-3-carbonsäureallylester,
IR (5 % $CH_2$ $Cl_2$ ): 3650, 2800-3140, 1790, 1743, 1705, 1690, 1173 cm $^{-1}$;
aus 2-[(3S,4R)-3-[(1'R)-1'-Allyloxycarbonyloxyäthyl]-4-[(2S)-1-(2-allyloxycarbonyloxyäthyl)-5-oxo-pyrrolidin-2-ylacetylthio]-2-oxo-azetidin-1-yl]-2-triphenylphosphoranylidenessigsäureallylester, der (5R,6S)-6-[(1'R)-1'-Allyloxycarbonyloxyäthyl]-2-[(2S)-1-(2-allyloxycarbonyloxyäthyl)-5-oxo-pyrrolidin-2-ylmethyl]-2-penem-3-carbonsäureallylester,
IR (4 % $CH_2$ $Cl_2$ ): 2800-3180, 1790, 1742, 1705, 1690, 1172 cm $^{-1}$;
aus 2-[(3S,4R)-3-[(1'R)-1'-Allyloxycarbonyloxyäthyl]-4-[(2S)-1-(2-cyanoäthyl)-5-oxo-pyrrolidin-2-ylacetylthio]-2-oxo-azetidin-1-yl]-2-triphenylphosphoranylidenessigsäureallylester der (5R,6S)-6-[(1'R)-1'-Allyloxycarbonyloxyäthyl]-2-[(2S)-1-(2-cyanoäthyl)-5-oxo-pyrrolidin-2-ylmethyl]-2-penem-3-carbonsäureallylester,
IR (5 % $CH_2$ $Cl_2$ ): 2800-3150, 2250, 1790, 1742, 1701, 1685, 1620, 1110 cm $^{-1}$.

Beispiel 25:Analog Beispiel 3 werden die folgenden Verbindungen hergestellt:
aus (5R,6S)-6-(tert.Butyldimethylsilyloxymethyl)-2-[(2S)-6-oxo-piperidin-2-ylmethyl]-2-penem-3-carbonsäureallylester der (5R,6S)-6-Hydroxymethyl-2-[(2S)-6-oxo-piperidin-2-ylmethyl]-2-penem-3-carbonsäureallylester,
IR (3 % $CH_2$ $Cl_2$ ): 2750-3600, 1780, 1685 cm $^{-1}$;
aus (5R,6S)-6-(tert.Butyldimethylsilyloxymethyl)-2-[(2R)-6-oxo-piperidin-2-ylmethyl]-2-penem-3-carbonsäureallylester der (5R,6S)-6-Hydroxymethyl-2-[(2R)-6-oxo-piperidin-2-ylmethyl]-2-penem-3-carbonsäureallylester,
IR (3 % $CH_2$ $Cl_2$ ): 2800-3550, 1778, 1680 cm $^{-1}$;
aus (5R,6S)-6-(tert.Butyldimethylsilyloxymethyl)-2-[(2S)-4-oxo-azetidin-2-ylmethyl]-2-penem-3-carbonsäureallylester der (5R,6S)-6-Hydroxymethyl-2-[(2S)-4-oxo-azetidin-2-ylmethyl]-2-penem-3-carbonsaäureallylester,
IR (3 % $CH_2$ $Cl_2$ ): 2750-3550, 1790, 1750 cm $^{-1}$;
aus (5R,6S)-6-(tert.Butyldimethylsilyloxymethyl)-2-[(2R)-4-oxo-azetidin-2-ylmethyl]-2-penem-3-carbonsäureallylester der (5R,6S)-6-Hydroxymethyl-2-[(2R)-4-oxo-azetidin-2-ylmethyl]-2-penem-3-carbonsäureallylester,
IR (3 % $CH_2$ $Cl_2$ ): 2755-3580, 1787, 1755 cm $^{-1}$;
aus (5R,6S)-6-[tert.Butyldimethylsilyloxymethyl)-2-[2-(2S)-5-oxo-pyrrolidin-2-yl]-äthyl]-2-penem-3-carbonsäureallylester der (5R,6S)-6-Hydroxymethyl-2-[2-(2S)-5-oxo-pyrrolidin-2-yl]-äthyl]-2-penem-3-carbonsäureallylester,
IR (3 % $CH_2$ $Cl_2$ ): 3415, 2800-3300, 1790, 1697 cm $^{-1}$;
aus (5R,6S)-6-(tert.Butyldimethylsilyloxymethyl)-2-[(3S)-2-oxo-pyrrolidin-3-ylmethyl]-2-penem-3-carbonsäureallylester der (5R,6S)-6-Hydroxymethyl-2-[(3S)-2-oxo-pyrrolidin-3-ylmethyl]-2-penem-3-carbonsäureallylester,
IR (3 % $CH_2$ $Cl_2$ ): 2760-3540, 1780, 1700 cm $^{-1}$;
aus (5R,6S)-6-(tert.Butyldimethylsilyloxymethyl)-2-[(4S)-2-oxo-pyrrolidin-4-ylmethyl]-2-penem-3-carbonsäureallylester der (5R,6S)-6-Hydroxymethyl-2-[(4S)-2-oxo-pyrrolidin-4-ylmethyl)-2-penem-3-carbonsäureallylester,
IR (3 % $CH_2$ $Cl_2$ ): 2750-3610, 1777, 1688 cm $^{-1}$.

Beispiel 26: Analog Beispiel 4 werden die folgenden Penem-3-carbonsäuren als Natriumsalz dargestellt:
aus (5R,6S)-6-[(1'R)-1'-Allyloxycarbonyloxyäthyl]-2-[(2S)-1-methyl-5-oxo-pyrrolidin-2-ylmethyl]-2-penem-3-carbonsäureallylester die (5R,6S)-6-[(1'R)-1'-Hydroxyäthyl]-2-[(2S)-1-methyl-5-oxo-pyrrolidin-2-ylmethyl]-2-penem-3-carbonsäure,
$\alpha_D$ (0,597 % $H_2O$): +36,4°;
UV ($10^{-4}$ % $H_2O$) $\lambda_{max}$ = 260; 305 nm;
IR (3 % DMSO-$d_6$ ): 3100-3650, 2800-3050, 1769, 1680, 1610, 1360, 990 cm $^{-1}$;
$^1$H-NMR ($D_2O$): $\delta$ 5,62 (d, 1, J = 1,5), 4,25 (dt, 1, J = 6,5 und 7,8), 3,99 (m, 1), 3,87 (dd, 1, J = 1,5 und 7,9), 3,32 (dd, 1, J = 4,5 und 14,0) 3,06 (dd, 1, J = 7,5 und 14,0) 2,83 (s, 3), 2,44 (m, 1), 2,35 (m, 1), 2,22 (m, 1), 1,90 (m, 1), 1,30 (d, 3, J = 6,5);
aus (5R,6S)-6-[(1'R)-1'-Allyloxycarbonyloxyäthyl]-2-[(2R)-1-methyl-5-oxo-pyrrolidin-2-ylmethyl]-2-penem-3-carbonsäureallylester die (5R,6S)-6-[(1'R)-1'-Hydroxyäthyl]-2-[(2R)-1-methyl-5-oxo-pyrrolidin-2-ylmethyl]-2-penem-3-carbonsäure,
$\alpha_D$ (0,646 % $H_2O$): + 219,8°;
UV ($10^{-4}$ % $H_2O$) $\lambda_{max}$ = 261; 305 nm;

IR (3 % DMSO-$d_6$): 3100-3650, 2800-3050, 1770, 1680, 1610, 1360, 985 $cm^{-1}$;
$^1$H-NMR ($D_2O$): $\delta$ 5,61 (d, 1, J = 1,4), 4,24 (q, 1, J = 7,0), 3,99 (m, 1), 3,86 (dd, 1, J = 1,4 und 5,9), 3,59 (dd, 1, J = 4,8 und 15,1), 2,85 (s, 3), 2,73 (dd, 1, J = 5,5 und 15,1), 2,52 (m, 1), 2,39 (m, 1), 2,25 (m, 1), 1,87 (m, 1), 1.30 (d, 1, J = 6,0);
aus (5R,6S)-6-Hydroxymethyl-2-[(2S)-6-oxo-piperidin-2-ylmethyl]-2-penem-3-carbonsäureallylester die (5R,6S)-6-Hydroxymethyl]-2-[(2S)-6-oxo-piperidin-2-ylmethyl]-2-penem-3-carbonsäure,
UV ($10^{-4}$ % $H_2O$) $\lambda_{max}$ = 258; 303 nm;
IR (3 % DMSO-$d_6$): 2800-3700, 1774, 1675 $cm^{-1}$;
aus (5R,6S)-6-Hydroxymethyl-2-[(2R)-6-oxo-piperidin-2-ylmethyl]-2-penem-3-carbonsäureallylester die (5R,6S)-6-Hydroxymethyl-2-[(2R)-6-oxo-piperidin-2-ylmethyl]-2-penem-3-carbonsäure,
UV ($10^{-4}$ % $H_2O$) $\lambda_{max}$ = 261; 302 nm;
IR (3 % DMSO-$d_6$): 2800-3700, 1773, 1675 $cm^{-1}$;
aus (5R,6S)-6-[(1'R)-1'-Allyloxycarbonyloxyäthyl]-2-[(2S)-6-oxo-piperidin-2-ylmethyl]-2-penem-3-carbonsäureallylester die (5R,6S)-6-[1'R)-1'-Hydroxyäthyl]-2-[(2S)-6-oxo-piperidin-2-ylmethyl]-2-penem-3-carbonsäure,
UV ($10^{-4}$ % $H_2O$) $\lambda_{max}$ = 259; 304 nm;
IR (3 % DMSO-$d_6$): 2750-3710, 1772, 1660 $cm^{-1}$;
$\alpha_D$ (0,12 % $H_2O$): +56,4°;
aus (5R,6S)-6-[(1'R)-1'-Allyloxycarbonyloxyäthyl]-2-[(2R)-6-oxo-piperidin-2-ylmethyl]-2-penem-3-carbonsäureallylester die (5R,6S)-6-[(1'R)-1'-Hydroxyäthyl]-2-[(2R)-6-oxo-piperidin-2-ylmethyl]-2-penem-3-carbonsäure,
UV ($10^{-4}$ % $H_2O$) $\lambda_{max}$ = 262; 302 nm;
IR (3 % DMSO-$d_6$): 2810-3690, 1772, 1675 $cm^{-1}$;
aus (5R,6S)-6-Hydroxymethyl-2-[(2S)-4-oxo-azetidin-2-ylmethyl]-2-penem-3-carbonsäureallylester die (5R,6S)-6-Hydroxymethyl-2-[(2S)-4-oxo-azetidin-2-ylmethyl]-2-penem-3-carbonsäure,
UV ($10^{-4}$ % $H_2O$) $\lambda_{max}$ = 260; 305 nm;
IR (3 % DMSO-$d_6$): 2800-3600, 1760, 1745 $cm^{-1}$;
aus (5R,6S)-6-Hydroxymethyl-2-[(2R)-4-oxo-azetidin-2-ylmethyl]-2-penem-3-carbonsäureallylester die (5R,6S)-6-Hydroxymethyl-2-[(2R)-4-oxo-azetidin-2-ylmethyl]-2-penem-3-carbonsäure,
UV ($10^{-4}$ % $H_2O$) $\lambda_{max}$ = 260; 306 nm;
IR (3 % DMSO-$d_6$): 2800-3600, 1765, 1751 $cm^{-1}$;
aus (5R,6S)-6-[(1'R)-1'-Allyloxycarbonyloxyäthyl]-2-[(2S)-4-oxo-azetidin-2-ylmethyl]-2-penem-3-carbonsäureallylester die (5R,6S)-6-[1'R)-1'-Hydroxyäthyl]-2-[(2S)-4-oxo-azetidin-2-ylmethyl]-2-penem-3-carbonsäure,
$\alpha_D$ (0,619 % $H_2O$): +132,9°;
UV ($10^{-4}$ % $H_2O$) $\lambda_{max}$ = 260; 304 nm;
IR (3 % DMSO-$d_6$): 2800-3600, 1760, 1745, 1610, 1591, 1362, 990 $cm^{-1}$;
$^1$H-NMR ($D_2O$): $\delta$ 5,7 (d, 1, J = 1,6), 4,25 (dt, 1, J = 5,4 und 6,5), 4,00 (dd, 1, J = 1,5 und 5,5), 3,30 (dd, 1, J = 5,7 und 15,0), 3,14 (m, 3), 2,77 (dd, 1, J = 2,2 und 15,0), 1,30 (d, 3, J = 6,4);
aus (5R,6S)-6-[(1'R)-1'-Allyloxycarbonyloxyäthyl]-2-[(2R)-4-oxo-azetidin-2-ylmethyl]-2-penem-3-carbonsäureallylester die (5R,6S)-6-[(1'R)-1'-Hydroxyäthyl]-2-[(2R)-4-oxo-azetidin-2-ylmethyl]-2-penem-3-carbonsäure,
UV ($10^{-4}$ % $H_2O$) $\lambda_{max}$ = 260; 306 nm;
IR (3 % DMSO-$d_6$): 2800-3600, 1762, 1750 $cm^{-1}$;
aus (5R,6S)-6-Hydroxymethyl-2-[2-[(2S)-5-oxo-pyrrolidin-2-yl)-äthyl]-2-penem-3-carbonsäureallylester die (5R,6S)-6-Hydroxymethyl-2-[2-[(2S)-5-oxo-pyrrolidin-2-yl]-äthyl]-2-penem-3-carbonsäure, UV ($10^{-4}$ % $H_2O$) $\lambda_{max}$ = 257; 302 nm;
IR (3 % DMSO-$d_6$): 2600-3650, 1770, 1691 $cm^{-1}$;
aus (5R,6S)-6-[(1'R)-1'-Allyloxycarbonyloxyäthyl]-2-[2-[(2S)-5-oxo-pyrrolidin-2-yl]-äthyl]-2-penem-3-carbonsäureallylester die (5R,6S)-6-[1'R)-1'-Hydroxyäthyl]-2-[2-[(2S)-5-oxo-pyrrolidin-2-yl]-äthyl]-2-penem-3-carbonsäure,
$\alpha_D$ (0,108 % $H_2O$) +194,5°;
UV ($10^{-4}$ % $H_2O$) $\lambda_{max}$ = 259; 302 nm;
IR (3 % DMSO-$d_6$): 2600=3650, 1770, 1690, 1610, 1588, 1365 $cm^{-1}$;
$^1$H-NMR ($D_2O$): $\delta$ 5,63 (d, 1, J = 1,4), 4,24 (q, 1, J = 6,4), 3,84 (dd, 1, J = 1,4 und 6,2), 3,78 (m, 1), 2,87 (t, 2, J = 7,5), 2,39 (m, 2), 2,30 (m, 1), 1,82 (m, 1), 1,80 (m, 2), 1,31 (d, 1, J = 6,5);
aus (5R,6S)-6-[(1'R)-1'-Allyloxycarbonyloxyäthyl]-2-[(3S)-2-oxo-pyrrolidin-3-ylmethyl)-2-penem-3-carbonsäureallylester die (5R,6S)-6-[(1'R)-1'-Hydroxyäthyl]-2-[(3S)-2-oxo-pyrrolidin-3-ylmethyl]-2-penem-3-carbonsäure,
UV ($10^{-4}$ % $H_2O$) $\lambda_{max}$ = 262; 303 nm;
IR (3 % DMSO-$d_6$): 2780-3700, 1768, 1691 $cm^{-1}$;
aus (5R,6S)-6-Hydroxymethyl-2-[(3S)-2-oxo-pyrrolidin-3-ylmethyl]-2-penem-3-carbonsäureallylester die (5R,6S)-6-Hydroxymethyl-2-[(3S)-2-oxo-pyrrolidin-3-ylmethyl]-2-penem-3-carbonsäure,
UV ($10^{-4}$ % $H_2O$) $\lambda_{max}$ = 262; 303 nm;
IR (3 % DMSO-$d_6$): 2800-3700, 1772, 1690 $cm^{-1}$;
aus (5R,6S)-6-Hydroxymethyl-2-[(4S)-2-oxo-pyrrolidin-4-ylmethyl]-2-penem-3-carbonsäureallylester die (5R,6S)-6-Hydroxymethyl-2-[(4S)-2-oxo-pyrrolidin-4-ylmethyl]-2-penem-3-carbonsäure,
UV ($10^{-4}$ % $H_2O$) $\lambda_{max}$ = 257; 302 nm;

IR (3 % DMSO-$d_6$): 2800-3700, 1775, 1691 $cm^{-1}$;
aus (5R,6S)-6-[(1'R)-1'-Allyloxycarbonyloxyäthyl]-2-[(4S)-2-oxo-pyrrolidin-4-ylmethyl]-2-penem-3-carbonsäureallylester die (5R,6S)-6-[(1'R)-1-Hydroxyäthyl)-2-[(4S)-2-oxo-pyrrolidin-4-ylmethyl]-2-penem-3-carbonsäure,
UV ($10^{-4}$ % $H_2O$) $\lambda_{max}$ = 258; 302 nm;
IR (3 % DMSO-$d_6$): 2800-3700, 1776, 1691 $cm^{-1}$.
aus (5R,6S)-6-[(1'R)-1'-Allyloxycarbonyloxyäthyl]-2-[(2S)-1-allyl-5-oxo-pyrrolidin-2-ylmethyl]-2-penem-3-carbonsäureallylester die (5R,6S)-6-[(1'R)-1-Hydroxyäthyl)-2-[(2S)-1-allyl-5-oxo-pyrrolidin-2-ylmethyl]-2-penem-3-carbonsäure,
$\alpha_D$ (0,616 % $H_2O$) +101,7°;
UV ($10^{-4}$ % $H_2O$) $\lambda_{max}$ = 260; 304 nm;
IR (3 % DMSO-$d_6$): 2800-3140, 1770, 1680, 1610, 1580, 1415, 1365 $cm^{-1}$;
$^1$H-NMR ($D_2O$): $\delta$ 5,79 (m, 1), 5,62 (d, 1, J = 1,4), 5,23 (dd, 1, J = 1,4 und 7,2), 5,19 (dd, 1, J = 1,5 und 13,8), 4,24 (q, 1, J = 6,4), 4,14 (ddm, 1, J = 4,5 und 16,2), 4,06 (m, 1), 3,85 (dd, 1, J = 1,4 und 5,9), 3,73 (dd, 1, J = 5,9 und 16,2), 3,22 (dd, 1, J = 4,4 und 14,3), 3,09 (dd, 1, J = 7,4 und 14,3), 2,50 (m, 1), 2,43 (m, 1), 2,27 (m, 1), 1,92 (m, 1), 1,30 (d, 1, J = 6,4);
aus (5R,6S)-6-[(1'R)-1'Allyloxycarbonyloxyäthyl]-4-[(2S)-2,5-dihydro-5-oxo-pyrrol-2-ylmethyl]-2-penem-3-carbonsäureallylester die (5R,6S)-6-[(1'R)-1'-Hydroxyäthyl]-2-[(2S)-5-oxo-2,5-dihydropyrrol-2-ylmethyl]-2-penem-3-carbonsäure,
UV ($10^{-4}$ % $H_2O$): $\lambda_{max}$ 260, 305 nm;
IR (3 % DMSO-$d_6$): 2700-3700, 1770, 1700, 1610 $cm^{-1}$;
aus (5R,6S)-6-[1'R)-1'-Allyloxycarbonyloxyäthyl]-2-[(2S)-1-(2-allyloxycarbonyläthyl)-5-oxo-pyrrolidin-2-ylmethyl]-2-penem-3-carbonsäureallylester die (5R,6S)-6-[(1'R)-1'-Hydroxyäthyl]-2-[(2S)-1-(2-carboxyäthyl)-5-oxo-pyrrolidin-2-ylmethyl]-2-penem-3-carbonsäure (Dinatriumsalz),
UV ($10^{-4}$ % $H_2O$) $\lambda_{max}$ 257, 305 nm,
IR (3 % in DMSO-$d_6$): 2700-3700, 1770, 1670, 1610, 1583, 1370, 990 $cm^{-1}$;
$^1$H-NMR ($D_2O$): $\delta$ 5,63 (d, 1, J = 1,5), 4,23 (q, 1, J = 6,0), 4,09 (m, 1), 3,87 (dd, 1, J = 1,5 und 5,0), 3,78 (m, 1), 3,28 (m, 1), 3,25 (m,1), 3,11 (dd, 1, J = 6,5 und 14,2), 2,39 (m, 4), 2,18 (m, 1), 1,90 (m, 1), 1,28 (d, 1, J = 6,2);
aus (5R,6S)-6-[(1'R)-1'-Allyloxycarbonyloxyäthyl]-2-[(2S)-1-(2-allyloxycarbonylaminoäthyl)-5-oxo-pyrrolidin-2-ylmethyl]-2-penem-3-carbonsäureallylester die (5R,6S)-6-[(1'R)-1'-Hydroxyäthyl]-2-[(2S)-1-(2-aminoäthyl)-5-oxo-pyrrolidin-2-ylmethyl]-2-penem-3-carbonsäure (freie Säure),
UV ($10^{-4}$ % $H_2O$) $\lambda_{max}$ 259, 305 nm,
IR (3 % DMSO-$d_6$): 2800-3650, 1770, 1675, 1610, 1582, 1370 $cm^{-1}$;
aus (5R,6S)-6-[(1'R)-1'-Allyloxycarbonyloxyäthyl]-2-[(2S)-1-(2-allyloxycarbonyloxyäthyl)-5-oxo-pyrrolidin-2-ylmethyl]-2-penem-3-carbonsäureallylester die (5R,6S)-6-[(1'R)-1'-Hydroxyäthyl]-2-[(2S)-1-(2-hydroxyäthyl)-5-oxo-pyrrolidin-2-ylmethyl]-2-penem-3-carbonsäure,
UV ($10^{-4}$ % $H_2O$) $\lambda_{max}$ 256, 304 nm,
IR (3 % DMSO-$d_6$): 2800-3650, 1771, 1672, 1610, 1583 $cm^{-1}$;
aus (5R,6S)-6-[(1'R)-1'-Allyloxycarbonyloxyäthyl]-2-[(2S)-1-(2-cyanoäthyl)-5-oxo-pyrrolidin-2-ylmethyl]-2-penem-3-carbonsäureallylester die (5R,6S)-6-[(1'R)-1'-Hydroxyäthyl]-2-[(2S)-1-(2-cyanoäthyl)-5-oxo-pyrrolidin-2-ylmethyl]-2-penem-3-carbonsäure,
UV ($10^{-4}$ % $H_2O$) $\lambda_{max}$ 256, 305 nm,
IR (3 % DMSO-$d_6$): 2800-3150, 2250, 1770, 1671, 1610 $cm^{-1}$.

Beispiel 27:
(5R,6S)-6-[(1'R)-1'-Hydroxyäthyl]-2-[(2S)-5-oxo-pyrrolidin-2-ylmethyl]-2-penem-3-carbonsäure-pivaloyloxymethylester

0,75 g (5 mMol) Natriumjodid werden in 3 ml Aceton gelöst und mit 0,19 ml (1,3 mMol) Pivalinsäurechlormethylester versetzt. Das Gemisch wird bei Raumtemperatur während 3 Std. gerührt und anschliessend auf 9 ml Methylenchlorid getropft. Die ausgefallenen anorganischen Salze werden abfiltriert. Die Methylenchloridlösung wird bis auf ca. 1 ml eingeengt und bei 0° zu einer Lösung von 167 mg (0,5 mMol) (5R,6S)-6-[(1'R)-1'-Hydroxyäthyl]-2-[(2S)-5-oxo-pyrrolidin-2-ylmethyl]-2-penem-3-carbonsäure (Natriumsalz) in 5 ml abs. DMF und 0,5 ml DMSO gegeben. Nach 30 Min. Rühren bei 0° und weiteren 30 Min. bei Raumtemperatur wird das Reaktionsgemisch in Aethylacetat aufgenommen und mit Sole gewaschen. Nach Trocknen über Natriumsulfat und Einengen am Rotationsverdampfer wird das Rohprodukt an Silicagel gereinigt (Laufmittel Toluol/Aethylacetat).
IR (Methylenchlorid): 3590, 1770, 1690 $cm^{-1}$.

Beispiel 28:
(5R,6S)-6-[(1'R)-1'-Hydroxyäthyl]-2-[(2S)-5-oxo-pyrrolidin-2-ylmethyl]-2-penem-3-carbonsäure-acetoxymethylester

133 mg (0,398 mMol) (5R,6S)-6-[1'R)-1'-Hydroxyäthyl]-2-[(2S)-5-oxo-pyrrolidin-2-ylmethyl]-2-penem-3-carbonsäure (Natriumsalz) werden in 5 ml abs. DMF und 0,5 ml abs. DMSO gelöst und bei 0° tropfenweise mit einer Lösung von 70 mg (0,46 mMol) Acetoxybrommethan in 0,5 ml abs. DMF versetzt. Nach 30 Minuten Rühren bei 0° und anschliessend weiteren 30 Minuten bei Raumtemperatur wird das Reaktionsgemisch in

Aethylacetat aufgenommen und mit Sole gewaschen. Nach Trocknen der organischen Phase über Natriumsulfat und Einengen am Rotationsverdampfer wird das Rohprodukt an Silicagel gereinigt (Laufmittel Toluol/Aethylacetat).
IR (Methylenchlorid): 3580, 1770, 1690 cm $^{-1}$.

Beispiel 29:
(5R,6S)-6-[(1'R)-1'-Hydroxyäthyl]-2-[(2S)-5-oxo-pyrrolidin-2-ylmethyl]-2-penem-3-carbonsäure-äthoxycarbonyloxyäthylester

0,6 g (4 mMol) Natriumjodid werden in 2 ml Aceton gelöst und mit 0,14 ml Aethyl-1-chloräthylcarbonat versetzt. Das Gemisch wird bei Raumtemperatur während 3 Stunden gerührt, auf 8 ml Methylenchlorid getropft und von den ausgefallenen anorganischen Salzen abfiltriert. Die Methylenchloridlösung wird bis ca. 1 ml eingeengt und bei 0° zu einer Lösung von 167 mg (0,5 mMol) (5R,6S)-6-[(1'R)-1'-Hydroxyäthyl]-2-[(2S)-5-oxo-pyrrolidin-2-ylmethyl]-2-penem-3-carbonsäure (Natriumsalz) in 3 ml DMF gegeben. Nach 3 Std. Rühren bei 0° nimmt man das Reaktionsgemisch in Aethylacetat auf und wäscht die organische Phase mit Wasser. Nach Trocknen über Natriumsulfat und Einengen am Rotationsverdampfer reinigt man das Rohprodukt an Silicagel (Laufmittel: Toluol/Aethylacetat).
IR (Methylenchlorid): 3585, 1770, 1690 cm $^{-1}$.

Beispiel 30:
(5R,6S)-6-[(1'R)-1'-Allyloxycarbonyloxyäthyl]-2-[(2S)-5-oxo-pyrrolidin-2-ylmethyl]-2-penem-3-carbonsäureallylester

2 g (4,26 mMol) 2-[(3S,4R)-3-[(1'R)-1'-Allyloxycarbonyloxyäthyl]-4-[(2S)-5-oxo-pyrrolidin-2-ylacetylthio]-2-oxo-azetidin-1-yl]-2-oxo-essigsäureallylester und 1,78 ml (10,5 mMol) Triäthylphosphit in 200 ml Toluol werden bei 105° unter Argonatmosphäre 2 Stunden gerührt. Die Aufarbeitung erfolgt, wie in Beispiel 2 beschrieben.
IR (3 % $CH_2Cl_2$ ): 3420, 2820-3460, 1791, 1701 cm $^{-1}$.
Die Verbindung ist mit der in Beispiel 10 beschriebenen Verbindung identisch.
Das Ausgangsmaterial kann wie folgt hergestellt werden:

a. (3S,4R)-3-[(1'R)-1'-Allyloxycarbonyloxyäthyl]-4-[(2S)-5-oxo-pyrrolidin-2-ylacetylthio]-2-oxo-azetidin

Zu einer Lösung von 2,57 g (10 mMol) (3S,4R)-3-[(1'R)-1'-Allyloxycarbonyloxyäthyl]-4-acetoxy-2-oxo-azetidin (vgl. Europäische Patentanmeldung Nr. 126709) in 25 ml Aceton wird bei 5° langsam 16 ml (16 mMol) 1N NaOH und 2,23 g (14 mMol) (2S)-5-Oxo-pyrrolidin-2-thiocarbonsäure zugegeben. Es wird 30 Min. gerührt, danach wird das Aceton durch Eindampfen entfernt und der wässrige Rückstand dreimal mit je 25 ml Aethylacetat extrahiert. Die vereinigten Extrakte werden mit 25 ml $NaHCO_3$ -Lösung und dann mit 25 ml konzentrierter NaCl-Lösung gewaschen, über $Na_2SO_4$ getrocknet und eingedampft. Der Rückstand wird durch Chromatographie an Kieselgel (Laufmittel Toluol/Aethylacetat) gereinigt.
IR (4 % $CH_2Cl_2$ ): 3410, 2800-3650, 1775, 1720, 1500, 1220, 840 cm $^{-1}$.

b.
2-[(3S,4R)-3-[(1'R)-1'-Allyloxycarbonyloxyäthyl]-4-[(2S)-5-oxo-pyrrolidin-2-ylacetylthio]-2-oxo-azetidin--1-yl]-2-oxo-essigsäureallylester

Zu einer Lösung von 1,78 g (50 mMol) (3S,4R)-3-[(1'R)-1'-Allyloxycarbonyloxyäthyl)-4-[(2S)-5-oxo-pyrrolidin-2-ylacetylthio]-2-oxo-azetidin in 25 ml ($CH_2Cl_2$ werden bei -15° unter Argonatmosphäre 0,84 ml (7,5 mMol) Allyloxyoxalylchlorid und 1,27 ml (7,5 mMol) Hünig-Base hinzugetropft. Die Mischung wird 1 Stunde bei gleicher Temperatur gerührt, nacheinander mit 25 ml 0,1N HCl, zweimal 25 ml $NaHCO_3$ -Lösung und 25 ml konzentrierter NaCl-Lösung gewaschen, über $Na_2SO_4$ getrocknet und eingedampft. Das Rohprodukt wird ohne weitere Reinigung weiter umgesetzt.
IR (5 % $CH_2Cl_2$ ): 3410, 2800-3700, 1815, 1720, 1210 cm $^{-1}$.

Der (5R,6S)-6-[(1'R)-1'-Allyloxycarbonyloxyäthyl]-2-[(2S)-5-oxo-pyrrolidin-2-ylmethyl]-2-penem-3-carbonsäureallylester wird in analoger Weise, wie im Beispiel 11 bzw. 4 beschrieben, in das Natriumsalz der (5R,6S)-6-[(1'R)-1'-Hydroxyäthyl]-2-[(2S)-5-oxo-pyrrolidin-2-ylmethyl]-2-penem-3-carbonsäure überführt.

In analoger Weise, wie im Beispiel 30 beschrieben, können alle in den vorangehenden Beispielen genannten Penem-Verbindungen hergestellt werden.

Beispiel 31:
2-[(3S,4R)-3-(tert.Butyldimethylsilyloxymethyl)-4-(5-oxo-tetrahydrofuran-2-acetylthio)-2-oxo-azetidin-1-yl]-2-triphenylphosphoranylidenessigsäureallylester (Diastereomere A und B)

Eine Lösung von 4,2 g racemischem 5-Oxo-tetrahydrofuran-2-acetylchlorid [aus der Säure nach R.D. Allan et al., Austr. J. Chem. 36, 977 (1983) hergestellt] in 100 ml $CH_2Cl_2$ wird bei 0° unter Rühren tropfenweise mit einem Gemisch bestehend aus 10,23 g Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters (Europäische Patentanmeldung Nr. 125207), 2,18 ml Pyridin und 50 ml Methylenchlorid versetzt. Nach beendigtem Zutropfen entfernt man das Kältebad und rührt noch 1 Stunde bei Raumtemperatur nach. Der sich gebildete Niederschlag wird über Hyflo abgenutscht und das Filtrat je einmal mit ges. $NaHCO_3$ - und Kochsalz-Lösung gewaschen, mit $Na_2SO_4$

getrocknet und eingedampft. Das Rohgemisch wird an Kieselgel chromatographisch in die Diastereomeren aufgetrennt (Laufmittel: Hexan/Aethylacetat 1:1).

2-[(3S,4R)-3-(tert.Butyldimethylsilyloxymethyl)-4-[(2R ?)-5-oxo-tetrahydrofuran-2-acetylthio]-2-oxo-azetidin-1-yl]-2-triphenylphosphoranylidenessigsäureallylester (Diastereomer A)

DC (Silicagel): Hexan-Aethylacetat (1:1) $R_f = 0{,}22$

IR (Methylenchlorid): 5,64; 5,68; 5,93 μm.

2-[(3S,4R)-3-(tert.Butyldimethylsilyloxymethyl)-4-[(2S ?)-5-oxo-tetrahydrofuran-2-acetylthio]-2-oxo-azetidin-1-yl]-2-triphenylphosphoranylidenessigsäureallylester (Diastereomer B)

DC (Silicagel): Hexan-Aethylacetat 1:1 $R_f = 0{,}16$

IR ($CH_2Cl_2$): 5,63; 5,71; 5,79; 5,91 μm.

Beispiel 32: (5R,6S)-6-(tert.Butyldimethylsilyloxymethyl)-2-[(2R ?)-5-oxo-tetrahydrofuran-2-ylmethyl]-2-penem-3-carbonsäureallylester (Diastereomer A)

730 mg 2-[(3S,4R)-3-(tert.Butyldimethylsilyloxymethyl)-4-[(2R ?)-5-oxo-tetrahydrofuran-2-acetylthio]-2-oxo-azetidin-1-yl)-2-triphenylphosphoranylidenessigsäureallylester (Diastereomer A) werden in 100 ml Toluol gelöst, mit einigen Kristallen 2,6-Di-tert.butyl-p-kresol (BHT) versetzt und während 7,5 Stunden unter Stickstoffatmosphäre bei 90° gerührt. Eindampfen des Lösungsmittels und Chromatographie des Rückstandes an Kieselgel mit dem Laufmittel Aether/Hexan (3:1) ergibt das reine Produkt.

Smp.: 88-90°

IR: (Methylenchlorid): 5,63; 5,87; 6,34 μm

Beispiel 33: (5R,6S)-6-(tert.Butyldimethylsilyloxymethyl)-2-[(2S ?)-5-oxo-tetrahydrofuran-2-ylmethyl]-2-penem-3-carbonsäureallylester (Diastereomer B)

Analaog Beispiel 32 erhält man ausgehend von 690 mg 2-[(3S,4R)-3-(tert.Butyldimethylsilyloxymethyl)-4-[(2S ?)-5-oxo-tetrahydrofuran-2-acetylthio[-2-oxo-azetidin-1-yl]-2-triphenylphosphoranyliden-essigsäureallylester (Diastereomer B) die Titelverbindung.

Smp. 105-108°.

IR (Methylenchlorid): 5,64; 5,88; 6,34 μm.

Beispiel 34: (5R,6S)-6-Hydroxymethyl-2-[(2R ?)-5-oxo-tetrahydrofuran-2-ylmethyl]-2-penem-3-carbonsäureallylester (Diastereomer A)

327 mg (5R,6S)-6-(tert.Butyldimethylsilyloxymethyl)-2-[(2R ?)-5-oxo-tetrahydrofuran-2-ylmethyl]-2-penem-3-carbonsäureallylester (Diastereomer A) werden in 6 ml abs. Tetrahydrofuran (THF) gelöst und unter Argonatmosphäre nach Abkühlen auf -80° mit 0,495 ml Essigsäure versetzt. Dann gibt man tropfenweise 18 ml einer 0,2 M Tetrabutylammoniumfluorid-Lösung in THF dazu, lässt das Gemisch auf Raumtemperatur erwärmen und rührt 3 Stunden bei dieser Temperatur. Das Reaktionsgemisch wird auf 5 ml am Rotationsverdampfer eingeengt und der Rückstand in 50 ml Aethylacetat aufgenommen. Die organische Phase wird je einmal mit ges. $NaHCO_3$ - und ges. Kochsalzlösung gewaschen, über Natriumsulfat getrocknet und eingeengt. Eindampfen am Rotationsverdampfer gibt das Rohprodukt, das an Silicagel mit Hexan/Aethylacetat (1:2,5) gereinigt wird.

NMR ($CDCl_3$): $\delta$ = 5,66 (d); 3,36 (dd); 3,11 (dd) ppm

UV (Aethanol): $\lambda_{max}$ = 319; 248 nm

Beispiel 35: (5R,6S)-6-Hydroxymethyl-2-[(2S ?)-5-oxo-tetrahydrofuran-2-ylmethyl]-2-penem-3-carbonsäureallylester (Diastereomer B)

Analog Beispiel 34 erhält man ausgehend von 300 mg (5R,6S)-6-(tert.Butyldimethylsilyloxymethyl)-2-[(2S ?)-5-oxo-tetrahydrofuran-2-ylmethyl]-2-penem-3-carbonsäureallylester (Diastereomer B) die Titelverbindung

NMR ($CDCl_3$): $\delta$ = 5,64 (d); 3,43 (dd); 3,07 (dd) ppm.

UV (Aethanol): $\lambda_{max}$: 320; 245 nm.

Beispiel 36: (5R,6S)-6-Hydroxymethyl-2-[(2R ?)-5-oxo-tetrahydrofuran-2-ylmethyl]-2-penem-3-carbonsäure, Natriumsalz (Diastereomer A)

367 mg (5R,6S)-6-Hydroxymethyl-2-[(2R ?)-5-oxo-tetrahydrofuran-2-ylmethyl]-2-penem-3-carbonsäureallylester (Diastereomer A) werden in 8 ml abs. THF gelöst und auf 0° abgekühlt. Nach Zugabe von 15 mg Palladium-tetrakis(triphenylphosphin) und 0,86 ml Tributylzinnhydrid lässt man das Reaktionsgemisch auf Raumtemperatur erwärmen und rührt während 1,5 Stunden. Dann wird mit 0,19 ml Essigsäure versetzt und weitere 30 Minuten bei Raumtemperatur gerührt. Danach giesst man das Reaktionsgemisch auf Aethylacetat und extrahiert das Produkt mit ges. $NaHCO_3$ -Lösung. Die Reinigung erfolgt durch Chromatographie an XAD-2 (Laufmittel:Wasser).

NMR ($D_2O$): $\delta$ = 5,62 (d); 3,50 (dd); 2,97 (dd) ppm

UV ($H_2O$): $\lambda_{max}$: 303; 258 nm.

Beispiel 37: (5R,6S)-6-Hydroxymethyl-2-[(2S ?)-5-oxo-tetrahyrofuran-2-ylmethyl]-2-penem-3-carbonsäure, Natriumsalz (Diastereomer B)

Analog zu Beispiel 36 erhält man ausgehend von 83 mg (5R,6S)-6-Hydroxymethyl-2-[(2S ?)-5-oxo-tetrahydrofuran-2-ylmethyl]-2-penem-3-carbonsäureallylester (Diastereomer B) die Titelverbindung.
NMR ($D_2O$): $\delta$ = 5,63; 3,31; 3,23 ppm.
UV ($H_2O$); $\lambda_{max}$: 300; 263 nm.

Beispiel 38:
2-[(3S,4R)-3-[(1'R)-1'-Allyloxycarbonyloxyäthyl]-4-(5-oxo-tetrahydrofuran-2-acetylthio)-2-oxo-acetidin--1-yl]-2-triphenylphosphoranylidenessigsäureallylester

Eine Lösung von 2,8 g racemischem 5-Oxo-tetrahydrofuran-2-ylacetylchlorid [aus der Säure nach R.D. Allan et al, Austr. J. Chem. 36, 977 (1983) hergestellt] in 30 ml $CH_2Cl_2$ wird auf 0° abgekühlt und tropfenweise mit einem Gemisch bestehend aus 8,0 g Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters (siehe Beispiel 17), 1,4 ml Pyridin und 30 ml $CH_2Cl_2$ versetzt. Nach beendigtem Zutropfen entfernt man das Kältebad und rührt noch 1 Std. bei Raumtemperature nach. Der sich gebildete Niederschlag wird über Hyflo abgenutscht und das Filtrat je einmal mit gesättigter $NaHCO_3$ -und NaCl- Lösung gewaschen, mit $Na\ SO_4$ getrocknet und eingedampft. Nach mehrmaliger Reinigung an Kieselgel (Laufmittel Toluol/Aethylacetat 1:1 und Hexan/Aethylacetat 1:2) erhält man 2-[(3S,4R)-3-[(1'R)-1'-Allyloxycarbonyloxyäthyl]-4-(5-oxo-tetrahydrofuran-2-acetylthio)-2-oxo-azetidin-1-yl]-2-triphenylphosphoranylidenessigsäureallylester (Diastereomerengemisch) als farbloses Oel, das beim Trocknen zu einem Schaum erstarrt.
IR (Methylenchlorid): 5,68; 5,90; 6,47 µm.
In analoger Weise erhält man ausgehend von optisch aktivem (2R)-bzw. (2S)-5-Oxo-tetrahydrofuran-2-ylacetylchlorid und dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters die diastereomeren Phosphorane 2-[(3S,4R)-3-[(1'R)-1'-Allyloxycarbonyloxyäthyl]-4-[(2R)-5-oxo-tetrahydrofuran-2-acetylthio]-2-oxo-azetidin-1-yl]-2-triphenylphosphoranylidenessigsäureallylester:
IR (Methylenchlorid): 5,67; 5,90; 6,17 µm;
bzw. 2-[(3S,4R)-3-[(1'R)-1'-Allyloxycarbonyloxyäthyl]-4-[(2S)-5-oxo-tetrahydrofuran-2-acetylthio]-2-oxo-azetidin-1-yl]-2-triphenylphosphoranylidenessigsäureallylester:
IR (Methylenchlorid): 5,76; 5,89; 6,11 µm.

Beispiel 39:
(5R,6S)-6-[(1'R)-1'-Allyloxycarbonyloxyäthyl]-2-(5-oxo-tetrahydrofuran-2-ylmethyl)-2-penem-3-carbonsäureallylester (Diastereomerengemisch)

Der 2-[(3S,4R)-3-[(1'R)-1'-Allyloxycarbonyloxyäthyl]-4-(5-oxo-tetrahydrofuran-2-acetylthio)-2-oxo-azetidin-1-yl]-2-triphenylphosphoranylidenessigsäureallylester (Diastereomerengemisch, 212 mg) wird in 15 ml abs. Toluol gelöst, mit einigen Kristallen 2,6-Di-tert.butyl-p-kresol (BHT) versetzt und unter einer Stickstoff-Atmosphäre während 24 Std. bei 90° gerührt. Eindampfen des Lösungsmittels und Chromatographie des Rückstandes an Kieselgel mit dem Laufmittel Toluol/Aethylacetat (2:1) ergibt das reine Produkt als viskoses Oel.
IR (Methylenchlorid): 5,61; 5,72; 5,84 µm.

Beispiel 40: Diastereomere von
(5R,6S)-6-[(1'R)-1'-Allyloxycarbonyloxyäthyl]-2-(5-oxo-tetrahydrofuran-2-ylmethyl)-2-penem-3-carbonsäureallylester

Analog Beispiel 39 erhält man aus 290 mg 2-[(3S,4R)-3-[(1'R)-1'-Allyloxycarbonyloxyäthyl]-4-[(2R)-5-oxo-tetrahydrofuran-2-acetylthio]-2-oxo-azetidin-1-yl]-2-triphenylphosphoranylidenessigsäureallylester bzw. 2-[(3S,4R)-3-[(1'R)-1'-Allyloxycarbonyloxyäthyl]-4-[(2S)-5-oxo-tetrahydrofuran-2-acetylthio]-2-oxo-azetidin-1-yl]-2-triphenylphosphoranylidenessigsäureallylester nach Reinigung an Silicagel (Laufmittel: Toluol/Aethylacetat 2:1) die diastereomeren Penem-Ester
(5R,6S)-6-[(1'R)-1'-Allyloxycarbonyloxyäthyl]-2-[(2R)-5-oxo-tetrahydrofuran-2-ylmethyl]-2-penem-3-carbonsäureallylester,
IR (Methylenchlorid): 5,62; 5,73; 5,85 µm;
bzw.
(5R,6S)-6-[(1'R)-1'-Allyloxycarbonyloxyäthyl]-2-[(2S)-5-oxo-tetrahydrofuran-2-ylmethyl]-2-penem-3-carbonsäureallylester,
IR(Methylenchlorid): 5,61; 5,73; 5,88 µm.

Beispiel 41: (5R,6S)-6-[(1'R)-1'-Hydroxyäthyl]-2-(5-oxo-tetrahydrofuran-2-ylmethyl)-2-penem-3-carbonsäure, Natriumsalz (Diastereomerengemisch)

260 mg (5R,6S)-6-[(1'R)-1'-Allyloxycarbonyloxyäthyl]-2-(5-oxo-tetrahydrofuran-2-ylmethyl)]-2-penem-3-carbonsäureallylester (Diastereomerengemisch) werden bei 0° und unter Argon in 10 ml abs. THF gelöst und mit 20 mg Palladiumtetrakis-(triphenylphosphin) versetzt. Mit der Spritze wird tropfenweise 630 µl Tributylzinnhydrid zugegeben. Danach wird das Kältebad entfernt und das Reaktionsgemisch 1,5 Std. bei Raumtemperatur nachgerührt. Nach Zugabe von 139 µl Essigsäure rührt man weitere 30 Minuten bei Raumtemperatur, verdünnt dann das Reaktionsgemisch mit Aethylacetat und extrahiert die Lösung zweimal mit Soda-Lösung (0,9 g Soda in 50 ml) sowie viermal mit gesättigter $NaHCO_3$ -Lösung. Der pH der

anorganischen Phase wird mit Essigsäure auf 8,5 eingestellt, das Gemisch am Rotationsverdampfer aufkonzentriert und an XAD-2 gereinigt. Man erhält die Titelverbindung als farbloses Lyophilisat.
IR ($DMSO-d_6$): 5,66; 6,19; 6,32 µm
UV ($H_2O$): $\lambda_{max}$: 304; 258 nm.

Beispiel 42: Diastereomere von (5R,6S)-6-[(1′R)-1′-Hydroxyäthyl]-2-(5-oxo-tetrahydrofuran-2-ylmethyl)-2-penem-3-carbonsäure, Natriumsalz

In analoger Weise, wie in Beispiel 41 beschrieben, werden 150 mg (5R,6S)-6-[(1′R)-1′-Allyloxycarbonyloxyäthyl]-2-[(2R)-5-oxo-tetrahydrofuran-2-ylmethyl]-2-penem-3-carbonsäureallylester, bzw. 150 mg (5R,6S)-6-[(1′R)-1′-Allyloxycarbonyloxyäthyl]-2-[(2S)-5-oxo-tetrahydrofuran-2-ylmethyl]-2-penem-3-carbonsäureallylester zu den folgenden diastereomeren Penemen umgesetzt, die über eine XAD-2 Säule gereinigt werden.
(5R,6S)-6-[(1′R)-1′-Hydroxyäthyl]-2-[(2R)-5-oxo-tetrahydrofuran-2-ylmethyl]-2-penem-3-carbonsäure, Natriumsalz,
UV($H_2O$): $\lambda_{max}$ = 304; 260 nm;
(5R,6S)-6-[(1′R)-1′-Hydroxyäthyl]-2-[(2S)-5-oxo-tetrahydrofuran-2-ylmethyl]-2-penem-3-carbonsäure, Natriumsalz,
UV($H_2O$): $\lambda_{max}$= 304; 260 nm.

Beispiel 43: Diastereomere von 2-[(3S,4R)-3-(tert.Butyldimethylsilyloxymethyl)-4-(3-oxo-phthalan-1-acetylthio)-2-oxo-azetidin-1-yl]-2-triphenylphosphoranylidenessigsäureallylester

Eine Lösung von 3,28 g 3-Oxo-phthalan-1-acetylchlorid (hergestellt aus der freien Säure mit $SOCl_2$) in 150 ml Methylenchlorid, wird bei 0° unter Rühren tropfenweise mit einem Gemisch bestehend aus 8,6 g Silbersalz des 2-[(3S,4R)-3-(tert.Butyldimethylsilyloxymethyl)-4-mercapto-2-oxo-acetidin-1-yl]-2-triphenylphosphoranylidenessigsäureallylester, 1,28 ml Pyridin und 100 ml Methylenchlorid versetzt. Nach beendigtem Zutropfen entfernt man das Kältebad und rührt noch 1 Stunde bei Raumtemperatur nach. Der sich gebildete Niederschlag wird über Hyflo abgenutscht und das Filtrat je einmal mit ges. $NaHCO_3$ - und Kochsalzlösung gewaschen, mit $Na_2SO_4$ getrocknet und eingedampft. Das Rohgemisch wird über Silicagel filtriert (Laufmittel Hexan/Aethylacetat 1:1) und an einer Merck-Fertigsäule in die Diastereomeren aufgetrennt (Laufmittel Hexan/Aethylacetat 2:1 und 1:1)
2-[(3S,4R)-3-(tert.Butyldimethylsilyloxymethyl)-4-[(1S ?)-3-oxo-phthalan-1-acetylthio]-2-oxo-azetidin-1-yl]-2-triphenylphosphoranylidenessigsäureallylester (Diastereomer A):
DC (Silicagel, Hexan/Aethylacetat 1:1): $R_f$ = 0,19
IR (Methylendichlorid): 5,68; 5,93; 6,18 µm
2-[(3S,4R)-3-(tert.Butyldimethylsilyloxymethyl)-4-[(1R ?)-3-oxo-phthalan-1-acetylthio]-2-oxo-azetidin-1-yl]-2-triphenylphosphoranylidenessigsäureallylester (Diastereomer B):
DC (Silicagel, Hexan/Aethylacetat 1:1): $R_f$ = 0,148
IR (Methylenchlorid): 5,66; 5,91; 6,18 µm.

Beispiel 44: (5R,6S)-6-(tert.Butyldimethylsilyloxymethyl)-2-[(1S ?)-3-oxo-phthalan-1-ylmethyl]-2-penem-3-carbonsäureallylester (Diastereomer A)

915 mg 2-[(3S,4R)-3-(tert.Butyldimethylsilyloxymethyl)-4-[(1S ?)-3-oxo-phthalan-1-acetylthio]-2-oxo-azetidin-1-yl]-2-triphenylphosphoranylidenessigsäureallylester (Diastereomer A) werden in 100 ml Toluol gelöst, mit einigen Kristallen 2,6-Di-tert.butyl-p-kresol (BHT) versetzt und während 4 Std. unter einer Stickstoffatmosphäre bei 100° gerührt. Eindampfen des Lösungsmittels und Chromatographie des Rückstandes an Kieselgel mit dem Laufmittel Toluol/Aethylacetat (2:1) ergibt das reine Produkt.
DC: (Silicagel, Toluol/Aethylacetat 1:1): $R_f$ = 0,75
IR (Methylenchlorid): 5,59; 5,65; 5,86; 6,32 µm.
NMR ($CDCl_3$): $\delta$ = 5,49 (d), 3,52 (dd); 3,45 (dd) ppm.

Beispiel 45: (5R,6S)-6-(tert.Butyldimethylsilyloxymethyl)-2-(1R ?)-3-oxo-phthalan-1-ylmethyl]-2-penem-3-carbonsäureallylester (Diastereomer B)

Analog Beispiel 44 erhält man ausgehend von 810 mg 2-[(3S,4R)-3-(tert.Butyldimethylsilyloxymethyl)-4-[(1R ?)-3-oxo-phthalan-1-acetylthio]-2-oxo-azetidin-1-yl]-2-triphenylphosphoranylidenessigsäureallylester (Diastereomer B) nach 2 Stunden Heizen bei 100° die reine Titelverbindung Smp.: 124-125°.
IR (Methylenchlorid): 5,58; 5,65; 5,86; 6,32 µm
NMR ($CDCl_3$): $\delta$ = 5,57 (d); 3,75 (dd); 3,24 (dd) ppm.

Beispiel 46: (5R,6S)-6-Hydroxymethyl-2-[(1S ?)-3-oxo-phthalan-1-ylmethyl]-2-penem-3-carbonsäureallylester (Diastereomer A)

590 mg (5R,6S)-6-(tert.Butyldimethylsilyloxymethyl)-2-[(1S ?)-3-oxo-phthalan-1-ylmethyl]-2-penem-3-carbonäureallylester (Diastereomer A) werden in 15 ml abs. Tetrahydrofuran gelöst und unter Argonatmosphäre nach abkühlen auf -80° mit 0,81 ml Essigsäure versetzt. Danach gibt man tropfenweise 13,24 ml einer 0,44 M Tetrabutylammoniumfluorid-Lösung in THF dazu, lässt das Gemisch auf Raumtemperatur kommen und rührt 3

Stunden bei dieser Temperatur nach. Das Reaktionsgemisch wird am Rotationsverdampfer eingeengt, und der Rückstand in 50 ml Aethylacetat aufgenommen. Die organische Phase wird je einmal mit ges. $NaHCO_3$ -und ges. Kochsalz-Lösung gewaschen, über Natriumsulfat getrocknet und eingeengt. Eindampfen am Rotationsverdampfer gibt das Rohprodukt, das an Silicagel mit Toluol/Aethylacetat 1:2 gereinigt wird.
IR (Methylenchlorid): 5,59; 5,65; 5,86; 6,33 µm.
NMR ($CDCl_3$): $\delta$ = 5,51 (d); 3,60 (dd); 3,46 (dd) ppm.

Beispiel 47: (5R,6S)-6-Hydroxymethyl-2-[(1R ?)-3-oxo-phthalan-1-ylmethyl]-2-penem-3-carbonsäureallylester (Diastereomer B)

Analog Beispiel 46 erhält man ausgehend von 485 mg (5R,6S)-6-(tert.Butyldimethylsilyloxymethyl)-2-(1R ?)-3-oxo-phthalan-1-ylmethyl]-2-penem-3-carbonsäureallylester (Diastereomer B) die Titelverbindung.
IR: (Methylenchlorid): 5,59; 5,65; 5,86; 6,33 µm.
NMR ($CDCl_3$): $\delta$ = 5,62 (d); 3,76 (dd); 3,28 (dd) ppm.

Beispiel 48: (5R,6S)-6-Hydroxymethyl-2-[(1S ?)-3-oxo-phthalan-1-ylmethyl]-2-penem-3-carbonsäure, Natriumsalz (Diastereomer A)

172 mg (5R,6S)-6-Hydroxymethyl-2-[(1S ?)-3-oxo-phthalan-1-ylmethyl]-2-penem-3-carbonsäureallylester (Diastereomer A) werden in 5 ml abs. THF gelöst und auf 0° abgekühlt. Nach Zugabe von 10 mg Palladiumtetrakis (triphenylphosphin) und 353 µl Tributylzinnhydrid, lässt man das Reaktionsgemisch auf Raumtemperatur erwärmen und rührt während 1 Stunde. Danach wird mit 79 µl Essigsäure versetzt und weitere 30 Minuten bei Raumtemperatur gerührt. Das Reaktionsgemisch wird auf Aethylacetat gegossen, einmal mit 10 ml $Na_3CO_3$ -Lösung (0,9 g/50 ml $H_2O$) und dreimal mit je 10 ml ges. $NaHOC_3$ -Lösung extrahiert. Die vereinigten wässerigen Phasen werden einmal mit Aethylacetat gewaschen. Die Reinigung erfolgt durch Chromatographie an XAD-2 (Laufmittel: Wasser und Wasser/Isopropanol 9:1).
NMR ($D_2O$): $\delta$ = 5,22 (s); 4,05 (dd); 3,26 (dd) ppm
UV ($H_2O$): $\lambda_{max}$ 305; 284; 275; 231 nm.

Beispiel 49: (5R,6S)-6-Hydroxymethyl-2-[(1R ?)-3-oxo-phthalan-1-ylmethyl]-2-penem-3-carbonsäure, Natriumsalz (Diastereomer B)

In analoger Weise, wie in Beispiel 48 beschrieben, erhält man ausgehend von (5R,6S)-6-Hydroxymethyl--2-[(1R ?)-3-oxo-phthalan-1-ylmethyl]-2-penem-3-carbonsäureallylester (Diastereomer B) die Titelverbindung.
NMR ($D_2O$): $\delta$ = 5,43 (d); 4,09 (dd); 3,48 (dd) ppm.
UV ($H_2O$): $\lambda_{max}$ = 304; 284; 275; 231 nm.

Beispiel 50: (5R,6S)-6-[(1'R)-1'-Hydroxyäthyl]-2-(3-oxo-phthalan-1-ylmethyl)-2-penem-3-carbonsäure, Natriumsalz (Diastereomere A und B

In analoger Weise, wie in den Beispielen 43-49 beschrieben, erhält man ausgehend vom Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und 3-Oxo-phthalan-1-acetylchlorid die diastereomeren Peneme (5R,6S)-6-[(1'R)-1'-Hydroxyäthyl]-2-[(1S ?)-3-oxo-phthalan-1-ylmethyl]-2-penem-3-carbonsäure, Natriumsalz (Diastereomer A)
[UV ($H_2O$): $\lambda_{max}$ 305; 285; 274; 231 nm]
und (5R,6S)-6-[(1'R)-1'-Hydroxyäthyl]-2-[(1R ?)-3-oxo-phthalan-1-ylmethyl]-2-penem-3-carbonsäure, Natriumsalz (Diastereomer B)
[UV ($H_2O$): $\lambda_{max}$ 304; 283; 275; 230 nm].

Beispiel 51: (5R,6S)-6-Hydroxymethyl-2-[(2S ?)-5-oxo-tetrahydrofuran-2-ylmethyl]-2-penem-3-carbonsäure-pivaloyloxymethylester

0,75 g (5 mMol) Natriumjodid werden in 3 ml Aceton gelöst und mit 0,19 ml (1,3 mMol) Pivalinsäurechlormethylester versetzt. Das Gemisch wird bei Raumtemperatur während 3 Std. gerührt und anschliessend auf 9 ml Methylenchlorid getropft. Die ausgefallenen anorganischen Salze werden abfiltriert. Die Methylenchloridlösung wird bis auf ca. 1 ml eingeengt und bei 0° zu einer Lösung von 160 mg (0,5 mMol) (5R,6S)-6-Hydroxymethyl-2-[(2S ?)-5-oxo-tetrahydrofuran-2-ylmethyl]-2-penem-3-carbonsäure, Natriumsalz (Diastereomer B) in 5 ml abs. DMF und 0,5 ml DMSO gegeben. Nach 30 Min. Rühren bei 0° und weiteren 30 Min. bei Raumtemperatur wird das Reaktionsgemisch in Aethylacetat aufgenommen und mit Sole gewaschen. Nach Trocknen über Natriumsulfat und Einengen am Rotationsverdampfer wird das Rohprodukt an Silicagel gereinigt (Laufmittel Toluol/Aethylacetat).
IR (Methylenchlorid): 2,79; 5,63; 5,89 µm.

Beispiel 52: (5R,6S)-6-Hydroxymethyl-2-[(2R ?)-5-oxo-tetrahydrofuran-2-ylmethyl]-2-penem-3-carbonsäure-acetoxymethylester

128 mg (0,398 mMol) (5R,6S)-6-Hydroxymethyl-2-[(2R ?)-5-oxo-tetrahydrofuran-2-ylmethyl]-2-penem-3-carbonsäure, Natriumsalz (Diastereomer A) werden in 5 ml abs. DMF und 0,5 ml abs. DMSO gelöst und bei 0° tropfenweise mit einer Lösung von 70 mg (0,46 mMol) Acetoxybrommethan in 0,5 ml abs. DMF versetzt. Nach 30 Minuten Rühren bei 0° und anschliessend weiteren 30 Minuten bei Raumtemperatur wird das

Reaktionsgemisch in Aethylacetat aufgenommen und mit Sole gewaschen. Nach Trocknen der organischen Phase über Natriumsulfat und Einengen am Rotationsverdampfer wird das Rohprodukt an Silicagel gereinigt (Laufmittel Toluol/Aethylacetat).
IR (Methylenchlorid): 2,79; 5,62; 5,88 μm.

Beispiel 53:
2-[(3S,4R)-3-[(1'R)-1'-Allyloxycarbonyloxyäthyl]-4-[3-(2-oxo-tetrahydrofuran-4-yl)-propionylthio]-2-oxo-azetidin-1-yl]-2-triphenylphosphoranylidenessigsäureallylester (Diastereomerengemisch)

293 mg Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und 47 μl Pyridin werden in 2 ml $CH_2Cl_2$ gelöst und bei 0° unter Argon mit 111 mg racemischem 3-(2-Oxo-tetrahydrofuran-4-yl)-propionylchlorid (erhalten auf übliche Art mit $SOCl_2$ aus der entsprechenden Säure, die nach O. Ceder et al., Acta Chem. Scand. 24, 2693 (1970) hergestellt wurde) tropfenweise versetzt. Nach 75 min. Rühren bei Raumtemperatur wird der entstandene Niederschlag über Hyflo abgenutscht und das Filtrat eingedampft. Der Rückstand wird in Aethylacetat aufgenommen, zweimal mit ges. $NaHCO_3$ -und zweimal mit ges. NaCl-Lösung gewaschen. Nach Trocknen über $Na_2SO_4$ engt man die organische Phase ein und reinigt den Rückstand an Kieselgel (Laufmittel Toluol/Aethylacetat 1:2). Man erhält die Titelverbindung als viskoses Oel, das beim Trocknen zu einem Schaum erstarrt.
IR ($CH_2Cl_2$): 5,68; 5,91; 6,18 μm.

Beispiel 54:
(5R,6S)-6-[(1'R)-1'-Allyloxycarbonyloxyäthyl]-2-[2-(2-oxo-tetrahydrofuran-4-yl)-äthyl]-2-penem-3-carbonsäureallylester (Diastereomerengemisch)

Analog zu dem in Beispiel 39 beschriebenen Verfahren erhält man ausgehend von 3,1 g 2-[(3S,4R)-3-[(1'R)-1'-Allyloxycarbonyloxyäthyl]-4-[3-(2-oxo-tetrahydrofuran-4-yl)-propionylthio]-2-oxo-azetidin-1-yl]-2-triphenylphosphoranylidenessigsäureallylester (Diastereomerengemisch) nach 5 Std. Rühren auf dem Oelbad bei einer Badtemperatur von 110° und anschliessender chromatographischer Reinigung an Silicagel (Laufmittel:Hexan/Aethylacetat 1:1) die Titelverbindung als gelbliches Harz.
IR ($CH_2Cl_2$): 5,60; 5,72; 5,85; 6,32 μm.

Beispiel 55:
(5R,6S)-6-[(1'R)-1'-Hydroxyäthyl]-2-[2-(2-oxo-tetrahydrofuran-4-yl)-äthyl]-2-penem-3-carbonsäure Natriumsalz (Diastereomerengemisch)

140 mg (5R,6S)-6-[(1'R)-1'-Allyloxycarbonyloxyäthyl]-2-[2-(2-oxo-tetrahydrofuran-4-yl)-äthyl]-2-penem-3-carbonsäureallylester (Diastereomerengemisch) werden unter Argon in 1,5 ml abs. Tetrahydrofuran gelöst und mit 20 mg Palladium-tetrakis-(triphenylphosphin) versetzt. Zu diesem Gemisch tropft man 63 μl Acetylaceton und rührt 3 Std. bei Raumtemperatur nach. Das Reaktionsgemisch wird dananch am Rotationsverdampfer eingeengt, mit 0,82 ml ges. $NaHCO_3$ -Lösung versetzt und direkt über eine XAD-2-Säule filtriert. Die Produkte enthaltenden Fraktionen werden eingeengt und nochmals an Opti-up Silicagel gereinigt (Laufmittel in beiden Fällen Wasser). Man erhält die Titelverbindung als farbloses Lyophilisat.
IR (DMSO-$d_6$): 5,66; 6,20; 6,32 μm.
UV ($H_2O$): $\lambda_{max}$ = 303, 260 nm.

Beispiel 56:
[(3S,4R)-3-(tert.-Butyldimethylsilyloxymethyl)-4-(2,5-dihydro-5-oxo-furan-2-ylacetylthio)-2-oxo-azetidin--1-yl]-2-triphenylphosphoranylidenessigsäureallylester (Diastereomerengemisch)

580 mg 2,5-Dihydro-5-oxo-furan-2-ylacetylchlorid (erhältlich mit $SOCl_2$ aus der entsprechenden Säure, die nach der Methode von J.A. Elridge et. al., J. Chem. Soc. (1950) 2228 hergestellt wurde) werden in 25 ml $CH_2Cl_2$ gelöst und bei 0° langsam mit einer Lösung von 1,71 g Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und 285 mg Pyridin in 25 ml $CH_2Cl_2$ versetzt. Nach beendigtem Zutropfen wird das Kältebad entfernt und noch 1 Std. bei Raumtemperatur nachgerührt. Die Aufarbeitung erfolgt analog zu dem in Beispiel 38 beschriebenen Verfahren. Die Reinigung des Phosphorans (Diastereomerengemisch) erfolgt durch Chromatographie an Silicagel (Laufmittel Hexan/Aethylacetat 1:1).
IR ($CH_2Cl_2$): 5,67, 5,89; 6,17 μm.

Beispiel 57:
(5R,6S)-6-(tert.Butyldimethylsilyloxymethyl)-2-(2,5-dihydro-5-oxo-furan-2-ylmethyl)-2-penem-3-carbonsäureallylester (Diastereomerengemisch)

645 g 2-[(3S,4R)-3-(tert.Butyldimethylsilyloxymethyl)-4-(2,5-dihydro-5-oxo-furan-2-ylacetylthio)-2-oxo-azetidin-1-yl]-2-triphenylphosphoranylidenessigsäureallylester (Diastereomerengemisch) werden in 50 ml abs. Toluol gelöst, mit einigen Kristallen BHT versetzt und unter einer Stickstoff-Atmosphäre während 3 Std. bei einer Oelbadtemperatur von 95-100° gerührt. Eindampfen des Lösungsmittels und Chromatographie des Rückstandes an Kieselgel in Hexan/Aethylacetat (2,5:1) ergibt das reine Produkt.
IR ($CH_2Cl_2$): 5,58; 5,67; 5,85 μm.

Beispiel 58:
(5R,6S)-6-Hydroxymethyl-2-(2,5-dihydro-5-oxo-furan-2-ylmethyl)-2-penem-3-carbonsäureallylester (Diastereomerengemisch)

190 mg (5R,6S)-6-(tert.Butyldimethylsilyloxymethyl)-2-(2,5-dihydro-5-oxo-furan-2-ylmethyl)-2-penem-3-carbonsäureallylester (Diastereomerengemisch) werden in 5 ml abs. Tetrahydrofuran gelöst und auf -78° abgekühlt. Zu dieser Lösung tropft man mit einer Spritze 288 µl Essigsäure, gefolgt von 2,1 ml einer 1 M Tetrabutylammoniumfluorid-Lösung in THF. Nach beendigtem Zutropfen wird das Kältebad entfernt und noch 1,5 Std. weiter gerührt. Das Reaktionsgemisch wird in Aethylacetat aufgenommen und mit ges. $NaHCO_3$ -Lösung und ges. NaCl-Lösung neutral gewaschen. Nach Trocknen über $Na_2SO_4$ wird die Lösung eingeengt und der Rückstand an Silicagel chromatographiert (Laufmittel:Hexan/Aethylacetat 1:1).
IR ($CH_2Cl_2$): 5,58; 5,66; 6,31 µm.

Beispiel 59: (5R,6S)-6-Hydroxymethyl-2-(2,5-dihydro-5-oxo-furan-2-ylmethyl)-2-penem-3-carbonsäure, Natriumsalz (Diastereomerengemisch)

Analog zu dem in Beispiel 41 beschriebenen Verfahren erhält man ausgehend von 50 mg (5R,6S)-6-Hydroxymethyl-2-(2,5-dihydro-5-oxo-furan-2-ylmethyl)-2-penem-3-carbonsäureallylester (Diastereomerengemisch) nach Reinigung an XAD-2 die Titelverbindung als farbloses Lyophilisat.
IR (KBr): 5,71 µm.
UV ($H_2O$): $\lambda_{max}$ 305, 257 nm.

Beispiel 60: Diastereomeren des
2-[(3S,4R)-3-(tert.Butyldimethylsilyloxymethyl)-4-(2,5-dihydro-5-oxo-furan-2-ylacetylthio)-2-oxo-azetidin-1-yl]-2-triphenylphosphoranylidenessigsäureallylester

Das gemäss Beispiel 56 erhältliche Diastereomerengemisch wird durch mehrmaliges Chromatographieren an Kieselgel (Laufmittel Hexan/Aethylacetat 2,5:1) in die einzelnen Diastereomeren aufgetrennt:
2-[(3S,4R)-3-(tert.Butyldimethylsilyloxymethyl)-4-[(2S)-2,5-dihydro-5-oxo-furan-2-ylacetylthio]-2-oxo-azetidin-1-yl]-2-triphenylphosphoranylidenessigsäureallylester:
IR ($CH_2Cl_2$): 5,68; 5,89; 6,17 µm.
2-[(3S,4R)-3-(tert.Butyldimethylsilyloxymethyl)-4-[(2R)-2,5-dihydro-5-oxo-furan-2-ylacetylthio]-2-oxo-azetidin-1-yl]-2-triphenylphosphoranylidenessigsäureallylester
IR ($CH_2Cl_2$): 5,68; 5,91; 6,18 µm.

Beispiel 61:
(5R,6S)-6-(tert.Butyldimethylsilyloxymethyl)-2-[(2S)-2,5-dihydro-5-oxo-furan-2-ylmethyl]-2-penem-3-carbonsäureallylester

Analog zu dem in Beispiel 57 beschriebenen Verfahren erhält man ausgehend von 744 mg 2-[(3S,4R)-3-(tert.Butyldimethylsilyloxymethyl)-4-[(2S)-2,5-dihydro-5-oxo-furan-2-ylacetylthio]-2-oxo-azetidin-1-yl]-2-triphenylphosphoranylidenessigsäureallylester die Titelverbindung in kristalliner Form.
IR ($Ch_2Cl_2$): 5,58; 5,67; 5,85; 6,32 µm.

Beispiel 62:
(5R,6S)-6-Hydroxymethyl-2-[(2S)-2,5-dihydro-5-oxo-furan-2-ylmethyl]-2-penem-3-carbonsäureallylester

Analog zu dem in Beispiel 58 beschriebenen Verfahren erhält man ausgehend von 315 mg (5R,6S)-6-(tert.Butyldimethylsilyloxymethyl)-2-[(2S)-2,5-dihydro-5-oxo-furan-2-ylmethyl]-2-penem-3-carbonsäureallylester nach chromatographischer Reinigung an Kieselgel die Titelverbindung als viskoses Oel.
IR ($CH_2Cl_2$): 2,84; 5,59; 5,83; 5,85; 6,32 µm.

Beispiel 63: (5R,6S)-6-Hydroxmethyl-2-[(2S)-2,5-dihydro-5-oxo-furan-2-ylmethyl]-2-penem-3-carbonsäure, Natriumsalz

Analog zu dem in Beispiel 59 beschriebenen Verfahren erhält man ausgehend von 170 mg (5R,6S)-6-Hydroxymethyl-2-[(2S)-2,5-dihydro-5-oxo-furan-2-ylmethyl]-2-penem-3-carbonsäureallylester nach Reinigung an XAD-2 und Lyophilisation die Titelverbindung als farbloses Lyophilisat.
IR (DMSO-$d_6$): 5,71; 6,19; 6,31 µm.
UV ($H_2O$): $\lambda_{max} = 306, 259$ nm.

Beispiel 64:
(5R,6S)-6-(tert.Butyldimethylsilyloxymethyl)-2-[(2R)-2,5-dihydro-5-oxo-furan-2-ylmethyl]-2-penem-3-carbonsäureallylester

Analog zu dem in Beispiel 57 beschriebenen Verfahren erhält man ausgehend von 702 mg 2-[(3S,4R)-3-(tert.Butyldimethylsilyloxymethyl)-4-[(2R)-2,5-dihydro-5-oxo-furan-2-ylacetylthio]-2-oxo-azetidin-1-yl]-2-triphenylphosphoranylidenessigsäureallylester nach chromatographischer Reinigung an Silicagel (Laufmittel Hexan/Aethylacetat 2,5:1) die Titelverbindung in kristalliner Form.
IR ($CH_2Cl_2$): 5,58; 5,67; 5,85; 6,32 µm.

Die Verbindung wurde einer Röntgenstrukturanalyse unterworfen, welche die angegebene absolute

Konfiguration bestätigen.

Beispiel 65:
(5R,6S)-6-Hydroxymethyl-2-[(2R)-2,5-dihydro-5-oxo-furan-2-ylmethyl]-2-penem-3-carbonsäureallylester

Analog zu dem in Beispiel 58 beschriebenen Verfahren erhält man ausgehend von 193 mg (5R,6S)-6-(tert.Butyldimethylsilyloxymethyl)-2-[(2R)-2,5-dihydro-5-oxo-furan-2-ylmethyl]-2-penem-3-carbonsäureallylester nach chromatographischer Reinigung an Kieselgel die Titelverbindung als farbloses kristalline Verbindung.
IR ($CH_2Cl_2$): 5,58; 5,67; 5,85; 6,32 μm.

Beispiel 66: (5R,6S)-6-Hydroxymethyl-2-[(2R)-2,5-dihydro-5-oxo-furan-2-ylmethyl]-2-penem-3-carbonsäure, Natriumsalz

Analog zu dem in Beispiel 59 beschriebenen Verfahren erhält man ausgehend von 357 mg (5R,6S)-6-Hydroxymethyl-2-[(2R)-2,5-dihydro-5-oxo-furan-2-ylmethyl]-2-penem-3-carbonsäureallylester nach Reinigung an XAD-2 und Lyophilisation die Titelverbindung als farbloses Lyophilisat.
IR (DMSO-$d_6$): 5,65; 5,71; 6,19 μm.
UV ($H_2O$): $\lambda_{max}$ = 305, 256 nm

Beispiel 67:
2-[(3S,4R)-3-[(1'R)-1'-Allyloxycarbonyloxyäthyl]-4-(2,5-dihydro-5-oxo-furan-2-ylacetylthio)-2-oxo-azetidin-1-yl]-2-triphenylphosphoranylidenessigsäureallylester (Diastereomerengemisch)

Analog zu dem in Beispiel 38 beschriebenen Verfahren erhält man ausgehend von 1,3 g racemischem 2,5-Dihydro-5-oxo-furan-2-ylacetylchlorid, 3,8 g Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und 676 μl Pyridin nach Reinigung an Kieselgel die Titelverbindung, die beim Trocknen zu einem Schaum erstarrt.
IR ($CH_2Cl_2$): 5,68; 5,91; 6,16 μm.

Beispiel 68:
(5R,6S)-6-[(1'R)-1'-Allyloxycarbonyloxyäthyl)-2-(2,5-dihydro-5-oxo-furan-2-ylmethyl)-2-penem-3-carbonsäureallylester (Diastereomerengemisch)

Analog zu dem in Beispiel 39 beschriebenen Verfahren erhält man ausgehend von 1,97 g 2-[(3S,4R)-3-[(1'R)-1'-Allyoxycarbonyloxyäthyl]-4-(2,5-dihydro-5-oxo-furan-2-ylacetylthio)-2-oxo-azetidin-1-yl]-2-triphenylphosphoranylidenessigsäureallylester (Diastereomerengemisch) die Titelverbindung als viskoses Oel.
IR ($CH_2Cl_2$): 5,57; 5,68; 5,84; 6,31 μm.

Beispiel 69:
(5R,6S)-6-[(1'R)-1'-Hydroxyäthyl]-2-(2,5-dihydro-5-oxo-furan-2-ylmethyl)-2-penem-3-carbonsäure, Natriumsalz (Diastereomerengemisch)

Analog zu dem in Beispiel 55 beschriebenen Verfahren erhält man ausgehend von 962 mg (5R,6S)-6-[(1'R)-1'-Allyloxycarbonyloxyäthyl)-2-(2,5-dihydro-5-oxo-furan-2-ylmethyl)-2-penem-3-carbonsäureallylester (Diastereomerengemisch) nach Reinigung über eine XAD-2 Säule die Titelverbindung.
IR (DMSO-$d_6$): 5,63; 5,70; 6,19; 6,31 μm.
UV ($H_2O$): 306, 259 nm.

Beispiel 70: Diastereomere von
2-[(3S,4R)-3-[(1'R)-1'-Allyloxycarbonyloxyäthyl]-4-(2,5-dihydro-3-methyl-5-oxo-furan-2-ylacetylthio)-2-oxo-azetidin-1-yl]-2-triphenylphosphoranylidenessigsäureallylester

Analog zu dem in Beispiel 38 beschriebenen Verfahren erhält man ausgehend von 3,02 g racemischem 2,5-Dihydro-3-methyl-5-oxo-furan-2-ylacetylchlorid (erhältlich mit $SOCl_2$ aus der entsprechenden Säure, die nach der Methode von J.A. Elridge et al., J. Chem. Soc. (1950) 2228 hergestellt wurde) ein Gemisch der diastereomeren Phosphorane. Diese können durch mehrmaliges Chromatographieren an Kieselgel (Laufmittel Toluol/Aethylacetat 1:1 sowie Hexan/Aethylacetat 1:1) getrennt werden.
2-[(3S,4R)-3-[(1'R)-1'-Allyloxycarbonyloxyäthyl]-4-(2,5-dihydro-3-methyl-5-oxo-furan-2-ylacetylthio)-2-oxo-azetidin-1-yl]-2-triphenylphosphoranylidenessigsäureallylester (Diastereomer A):
IR ($CH_2Cl_2$): 5,68; 5,91; 6,17 μm.
2-[(3S,4R)-3-[(1'R)-1'-Allyloxycarbonyloxyäthyl]-4-(2,5-dihydro-3-methyl-5-oxo-furan-2-ylacetylthio)-2-oxo-azetidin-1-yl]-2-triphenylphosphoranylidenessigsäureallylester (Diastereomer B):
IR ($CH_2Cl_2$): 5,67; 5,89; 6,16 μm.

Beispiel 71:
(5R,6S)-6-[(1'R)-1'-Allyloxycarbonyloxyäthyl]-2-(2,5-dihydro-3-methyl-5-oxo-furan-2-ylmethyl)-2-penem-3-carbonsäureallylester (Diastereomer A)

Analog zu dem in Beispiel 39 beschriebenen Verfahren erhält man ausgehend von 878 mg 2-[(3S,4R)-3-[(1'R)-1'-Allyloxycarbonyloxyäthyl]-4-(2,5-dihydro-3-methyl-5-oxo-furan-2-ylacetylthio)-2-oxo-azetidin-1-yl]-2-triphenylphosphoranylidenessigsäureallylester (Diastereomer A) nach Reinigung an Kieselgel (Laufmittel Hexan/Aethylacetat 1:1) die Titelverbindung.
IR ($CH_2Cl_2$): 5,58; 5,66; 5,71; 5,85; 6,07; 6,33 µm.

Beispiel 72:
(5R,6S)-6-[(1'R)-1'-Hydroxyäthyl]-2-(2,5-dihydro-3-methyl-5-oxo-furan-2-ylmethyl)-2-penem-3-carbonsäure, Natriumsalz (Diastereomer A)

Analog zu dem in Beispiel 55 beschriebenen Verfahren erhält man ausgehend von 119 mg (5R,6S)-6-[(1'R)-1'-Allyloxycarbonyloxyäthyl]-2-(2,5-dihydro-3-methyl-5-oxo-furan-2-ylmethyl)-2-penem-3-carbonsäureallylester (Diastereomer A) nach Reinigung über XAD-2 und Lyophilisation die Titelverbindung als farbloses Lyophilisat. IR (DMSO-$d_6$): 5,65; 5,70; 6,09; 6,19; 6,32 µm.
UV ($H_2O$): $\lambda_{max}$: 307, 258 nm.

Beispiel 73:
(5R,6S)-6-[(1'R)-1'-Allyloxycarbonyloxyäthyl]-2-(2,5-dihydro-3-methyl-5-oxo-furan-2-ylmethyl)-2-penem-3-carbonsäureallylester (Diastereomer B)

Analog zu dem in Beispiel 39 beschriebenen Verfahren erhält man ausgehend von 626 mg 2-[(3S,4R)-3-[(1'R)-1'-Allyloxycarbonyloxyäthyl]-4-(2,5-dihydro-3-methyl-5-oxo-furan-2-ylacetylthio)-2-oxo-azetidin-1-yl]-2-triphenylphosphoranylidenessigsäureallylester (Diastereomer B) nach Reinigung an Kieselgel (Laufmittel Hexan/Aethylacetat 2,5:1) die Titelverbindung.
IR ($CH_2Cl_2$): 5,58; 5,67; 5,71; 5,85; 6,07; 6,32 µm.

Beispiel 74:
(5R,6S)-6-[(1'R)-1'-Hydroxyäthyl]-2-(2,5-dihydro-3-methyl-5-oxo-furan-2-ylmethyl)-2-penem-3-carbonsäure, Natriumsalz (Diastereomer B)

Analog zu dem in Beispiel 55 beschriebenen Verfahren erhält man ausgehend von 558 mg (5R,6S)-6-[(1'R)-1'-Allyloxycarbonyloxyäthyl]-2-(2,5-dihydro-3-methyl-5-oxo-furan-2-ylmethyl)-2-penem-3-carbonsäureallylester (Diastereomer B) nach Reinigung an XAD-2 und Lyophilisation die Titelverbindung als farbloses Lyophilisat. IR (DMSO-$d_6$): 5,65; 5,69; 6,08; 6,19; 6,32 µm.
UV ($H_2O$): $\lambda_{max}$: 305, 258 nm.

Beispiel 75:
2-[(3S,4R)-3-[(1'R)-1'-Allyloxycarbonyloxyäthyl]-4-[3-(5-oxo-tetrahydrofuran-2-yl)-propionylthio]-2-oxo-azetidin-1-yl]-2-triphenylphosphoranylidenessigsäureallylester (Diastereomerengemisch)

Analog zu dem in Beispiel 38 beschriebenen Verfahren erhält man ausgehend von 380 mg racemischem 3-(5-Oxo-tetrahydrofuran-2-yl)-propionylchlorid die Titelverbindung als Diastereomerengemisch.
IR ($CH_2Cl_2$): 5,66; 5,89; 6,17 µm.

Beispiel 76:
(5R,6S)-6-[(1'R)-1'-Allyloxycarbonyloxyäthyl]-2-[2-(5-oxo-tetrahydrofuran-2-yl)-äthyl]-2-penem-3-carbonsäureallylester (Diastereomerengemisch)

Analog zu dem in Beispiel 39 beschriebenen Verfahren erhält man ausgehend von 418 mg 2-[(3S,4R)-3-[(1'R)-1'-Allyloxycarbonyloxyäthyl]-4-[3-(5-oxo-tetrahydrofuran-2-yl)-propionylthio]-2-oxo-azetidin-1-yl]-2-triphenylphosphoranylidenessigsäureallylester (Diastereomerengemisch) nach Reinigung an Kieselgel (Laufmittel Hexan/Aethylacetat 1:1) die Titelverbindung.
IR ($CH_2Cl_2$): 5,58; 5,62; 5,72; 5,84; 6,32 µm.

Beispiel 77:
(5R,6S)-6-[(1'R)-1'-Hydroxyäthyl]-2-[2-(5-oxo-tetrahydrofuran-2-yl)-äthyl]-2-penem-3-carbonsäure, Natriumsalz (Diastereomerengemisch)

Analog zu den in Beispiel 55 beschriebenen Verfahren erhält man ausgehend von 149 mg (5R,6S)-6-[(1'R)-1'-Allyloxycarbonyloxyäthyl]-2-[2-(5-oxo-tetrahydrofuran-2-yl)-äthyl]-2-penem-3-carbonsäureallylester (Diastereomerengemisch) nach Reinigung über XAD-2 und Lyophilisation die Titelverbindung als farbloses Lyophilisat.
IR (DMSO-$d_6$): 5,65; 6,19; 6,30 µm.
UV ($H_2O$): $\lambda_{max}$ = 302, 259 nm.

Beispiel 78: In analoger Weise, wie in den Beispielen 43-49 beschrieben, werden ausgehend von Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters und racem. 5-Methoxy-3-oxo-phthalan-1-ylacetylchlorid (erhältlich aus der freien Säure mit $SOCl_2$) die folgenden diastereomeren Peneme erhalten:
(5R,6S)-6-Hydroxymethyl-2-(5-methoxy-3-oxo-phthalan-1-ylmethyl)-2-penem-3-carbonsäure, Natriumsalz
Diastereomer A: UV ($H_2O$): $\lambda_{max}$: 302; 229 (Schulter); 212 nm;
Diastereomer B: UV ($H_2O$): $\lambda_{max}$: 303, 230 (Schulter); 212 nm.

Beispiel 79: In analoger Weise, wie in den Beispielen 31-78 beschrieben, erhält man ausgehend vom Silbersalz des 2-[(3S,4R)-3-(tert.Butyldimethylsilyloxymethyl)-4-mercapto-2-oxo-azetidin-1-yl]-2-triphenylphosphoranylidenessigsäureallylesters bzw. vom Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters mit (4R)- bzw. (4S)-3-(2-Oxo-tetrahydrofuran-4-yl)-propionylchlorid (hergestellt jeweils aus der freien Säure mit $SOCl_2$ )
(5R,6S)-6-Hydroxymethyl-2-[(4R)-2-(2-oxo-tetrahydrofuran-4-yl)-äthyl]-2-penem-3-carbonsäure, Natriumsalz
UV ($H_2O$): $\lambda_{max}$: 302, 257 nm;
(5R,6S)-6-Hydroxymethyl-2-[(4S)-2-(2-oxo-tetrahydrofuran-4-yl)-äthyl]-2-penem-3-carbonsäure, Natriumsalz
UV ($H_2O$): $\lambda_{max}$: 303, 257 nm;
bzw.
(5R,6S)-6-[(1'R)-1'-Hydroxyäthyl]-2-[(4R)-2-(2-oxo-tetrahydrofuran-4-yl)-äthyl]-2-penem-3-carbonsäure, Natriumsalz
UV ($H_2O$): $\lambda_{max}$: 301, 256 nm;
(5R,6S)-6-[(1'R)-1'-Hydroxyäthyl]-2-[(4S)-2-(2-oxo-tetrahydrofuran-4-yl)-äthyl]-2-penem-3-carbonsäure, Natriumsalz
UV ($H_2O$): $\lambda_{max}$: 302, 258 nm.

Beispiel 80: In analoger Weise, wie in den Beispielen 31-78 beschrieben, werden ausgehend vom Silbersalz des 2-[(3S,4R)-3-(tert. Butyldimethylsilyloxymethyl)-4-mercapto-2-oxo-azetidin-1-yl]-2-triphenylphosphoranylidenessigsäureallylesters bzw. vom Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und racemischem bzw. optisch aktivem [(2R) oder (2S)] 2,5-Dihydro-5-oxo-furan-2-ylacetylchlorid, 2,5-Dihydro-3-methyl-5-oxo-furan-2-ylacetylchlorid bzw. 2,5-Dihydro-3,4-dimethyl-5-oxo-furan-2-ylacetylchlorid (jeweils hergestellt aus der freien Säure mit $SOCl_2$ ) die folgenden Peneme hergestellt:
(5R,6S)-6-[(1'R)-1'-Hydroxyäthyl]-2-(2,5-dihydro-5-oxo-furan-2-yl-methyl)-2-penem-3-carbonsäure, Natriumsalz
Diastereomer A: UV ($H_2O$): $\lambda_{max}$= 302; 257 nm
Diastereomer B: UV ($H_2O$): $\lambda_{max}$= 303; 257 nm
(5R,6S)-6-Hydroxymethyl-2-(2,5-dihydro-3-methyl-5-oxo-furan-2-yl-methyl)-2-penem-3-carbonsäure, Natriumsalz
Diastereomer A: UV ($H_2O$): $\lambda_{max}$= 305; 258 nm
Diastereomer B: UV ($H_2O$): $\lambda_{max}$= 304; 258 nm
(5R,6S)-6-Hydroxymethyl-2-(2,5-dihydro-3,4-dimethyl-5-oxo-furan-2-ylmethyl)-2-penem-3-carbonsäure, Natriumsalz
Diastereomer A: UV ($H_2O$): $\lambda_{max}$= 304; 258 nm
Diastereomer B: UV ($H_2O$): $\lambda_{max}$= 305; 258 nm
(5R,6S)-6-[1'R)-1'-Hydroxyäthyl]-2-(2,5-dihydro-3,4-dimethyl-5-oxo-furan-2-ylmethyl)-2-penem-3-carbonsäure, Natriumsalz
Diastereomer A: UV ($H_2O$): $\lambda_{max}$= 304; 259 nm
Diastereomer B: UV ($H_2O$): $\lambda_{max}$= 306; 257 nm.

Beispiel 81: Analog zu dem in Beispiel 55 beschriebenen Verfahren erhält man ausgehend von 358 mg (5R,6S)-6-[(1'R)-1'-Allyloxycarbonyloxyäthyl]-2-[3-(5-oxo-tetrahydrofuran-2-yl)-propyl]-2-penem-3-carbonsäureallylester das Natriumsalz der (5R,6S)-6-[(1'R)-1'-Hydroxyäthyl]-2-[3-(5-oxo-tetrahydrofuran-2-yl)-propyl]-2-penem-3-carbonsäure.
UV (Wasser): $\lambda_{max}$ = 302, 260 nm.

Das Ausgangsmaterial kann wie folgt hergestellt werden:

a) 1-Pyrrolidinocyclopentan

84,1 g Cyclopentanon und 106,7 g Pyrrolidin werden in 300 ml Benzol vorgelegt und über Nacht am Wasserabscheider gekocht. Nach Entfernung des Lösungsmittels sowie des überschüssigen Reagenzes setzt man das Enamin ohne weitere Reinigung weiter um.

b) 3-(2-Oxo-cyclopent-1-yl)-propionsäureäthylester

40 g Enamin (vgl. Beispiel 81a) und 43,8 g Acrylsäureäthylester werden in 300 ml Dioxan während 4 Std. zum Sieden erhitzt. Danach gibt man 25 ml Wasser zum Reaktionsgemisch hinzu und erhitzt weitere 30 Min. unter Rückfluss. Die Lösung wird eingeengt, mit 1N HCl und gesättigter $NaHCO_3$ -Lösung gewaschen und mit $Na_2SO_4$ getrocknet. Nach Entfernung des Lösungsmittels destilliert man den Rückstand (Sdp. 71°C/200 Pa). Zurück bleibt das Produkt als farblose Flüssigkeit. IR ($CH_2Cl_2$): 5,78 μm.

c) 3-(6-Oxo-tetrahydropyran-2-yl)-propionsäureäthylester

1 g des Esters aus Beispiel 81b in 10 ml $CH_2Cl_2$ werden mit 1,3 g m-Chlorperbenzoesäure und 0,5 g $NaHCO_3$ versetzt und 15 Std. bei ca. 0° gerührt. Die Reaktionsmischung wird über eine Fritte abgenutscht, mit gesättigter $NaHCO_3$ -Lösung, 1N Natronlauge und wieder mit gesättigter $NaHCO_3$ -Lösung gewaschen, über $Na_2SO_4$ getrocknet und eingeengt. Filtration an Kieselgel in Diäthyläther/Hexan (1:1) sowie Destillation im Kugelrohr (115°/200 Pa) ergibt die Titelverbindung. IR ($CH_2Cl_2$): 5,78 μm.

d) 4-(5-Oxo-tetrahydrofuran-2-yl)-buttersäure

8,5 g des Esters aus Beispiel 81c werden in 27 ml 2N HCl und 30 ml THF über Nacht bei 0° gerührt. Danach erhitzt man das Reaktionsgemisch noch während 1 Std. auf 50°, verdünnt mit 100 ml Wasser und sättigt die Lösung mit Kochsalz. Diese Lösung extrahiert man während 15 Std. mit Diäthyläther. Reinigung der so erhaltenen Substanz an Kieselgel in $CH_2Cl_2$ /Methanol (98:2) ergibt die Titelverbindung. IR ($CH_2Cl_2$): 5,64; 5,84 µm.

Die 4-(5-Oxo-tetrahydrofuran-2-yl)-buttersäure wird in üblicher Weise mit Thionylchlorid in das Säurechlorid überführt, welches in analoger Weise, wie in den Beispielen 56 und 57 beschrieben, in die Ausgangsverbindung umgewandelt wird.

Beispiel 82:

2-[(3S,4R)-3-[(1'R)-1'-Allyloxycarbonyloxyäthyl]-4-[(2S)-1-methoxycarbonylmethyl-5-oxo-pyrrolidin-2-ylacetylthio]-2-oxo-azetidin-1-yl]-2-triphenylphosphoranylidenessigsäureallylester

Analog Beispiel 1 wird aus dem Silbersalz des 2-[(3S,4R)-3-[(1'R)-1'-Allyloxycarbonyloxyäthyl]-4-mercapto-2-oxo-azetidin-1-yl]-2-triphenylphosphoranylidenessigsäureallylesters und (2S)-1-Methoxycarbonylmethyl-5-oxo-pyrrolidin-2-ylacetylchlorid (hergestellt aus der Säure mit $SOCl_2$) die Titelverbindung hergestellt.

IR (3 % $CH_2Cl_2$): 1750, 1690; 1620 $cm^{-1}$.

DC (Silicagel, Aethylacetat), $R_f$ = 0,45.

Beispiel 83:

(5R,6S)-6-[(1'R)-1'-Allyloxycarbonyloxyäthyl]-2-[(2S)-1-methoxycarbonylmethyl-5-oxo-pyrrolidin-2-ylmethyl]-2-penem-3-carbonsäureallylester

Analog Beispiel 2 wird der 2-[(3S,4R)-3-[(1'R)-1'-Allyloxycarbonyloxyäthyl]-4-[(2S)-1-methoxycarbonylmethyl-5-oxo-pyrrolidin-2-yl-acetylthio]-2-oxo-azetidin-1-yl]-2-triphenylphosphoranylidenessigsäureallylester zur Titelverbindung umgesetzt.

IR (3 % $CH_2Cl_2$): 1785, 1745, 1695, 1580 $cm^{-1}$.

DC (Silicagel, Aethylacetat), $R_f$ = 0,52.

Beispiel 84:

(5R,6S)-6-[(1'R)-1'-Hydroxyäthyl]-2-[(2S)-1-methoxycarbonylmethyl-5-oxo-pyrrolidin-2-ylmethyl]-2-penem-3-carbonsäure Natriumsalz

230 mg (0,45 mMol) (5R,6S)-6-[(1'R)-1'-Allyloxycarbonyloxyäthyl]-2-[(2S)-1-methoxycarbonylmethyl-5-oxo-pyrrolidin-2-ylmethyl]-2-penem-3-carbonsäureallylester und 155 mg (0,99 mMol) Dimethylbarbitursäure werden in 5 ml Tetrahydrofuran bei Raumtemperatur unter Stickstoffatmosphäre gelöst. Nach Zugabe von 61 mg Palladiumtetrakis-(triphenylphosphin) wird die Reaktionsmischung 45 Minuten bei Raumtemperatur gerührt. Das Produkt wird als Natriumsalz bei pH 7,1 über XAD2 filtriert und danach lyophilisiert.

UV (Wasser) $\lambda_{max}$ = 305 nm.

IR (3 % DMSO-$d_6$) 1769, 1687, 1612 $cm^{-1}$.

DC ($UPC_{12}$, Wasser), $R_f$ = 0,3.

Beispiel 85: Trockenampullen oder Vials, enthaltend 0,5 g (5R,6S)-6-[(1'R)-1'-Hydroxyäthyl]-2-[(2S)-5-oxo-pyrrolidin-2-yl-methyl]-2-penem-3-carbonsäure, Natriumsalz als Wirksubstanz, werden wie folgt hergestellt:

Zusammensetzung (für 1 Ampulle oder Vial):

Wirksubstanz 0,5 g

Mannit 0,5 g

Eine sterile wässerige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele verwendet werden.

**Patentansprüche**

1. Verbindungen der Formel

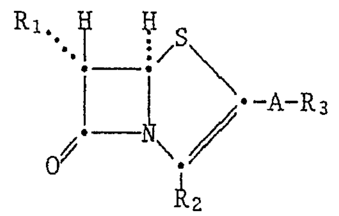

(I),

worin $R_1$ durch Hydroxy oder geschütztes Hydroxy substituiertes Niederalkyl ist, $R_2$ Carboxyl oder funktionell abgewandeltes Carboxyl bedeutet, A für Niederalkylen steht und $R_3$ einen über ein Ringkohlenstoffatom gebundenen, gegebenenfalls partiell ungesättigten Lactamylrest oder Lactonylrest darstellt, optische Isomere von Verbindungen der Formel I, Mischungen dieser optischen Isomere und Salze von Verbindungen der Formel I, die einen salzbildende Gruppe aufweisen.

2. Verbindungen der Formel I gemäss Anspruch 1, worin $R_1$ durch Hydroxy oder geschütztes Hydroxy substituiertes Niederalkyl ist, $R_2$ Carboxyl, unter physiologischen Bedingungen spaltbares verestertes Carboxyl oder geschütztes Carboxyl $R_2'$ darstellt, A Niederalkylen bedeutet und $R_3$ einen über ein Ringkohlenstoffatom gebundenen, monocyclischen 4- bis 6-gliedrigen, von einer $\omega$-Amino-($C_3$-$C_5$)-niederalkancarbonsäure oder $\omega$-Amino-($C_4$-$C_5$)-niederalkencarbonsäure abgeleiteten Lactamylrest, einen 5- oder 6-gliedrigen, von einer $\omega$-Amino-($C_4$-$C_5$)-niederalkencarbonsäure abgeleiteten Benzolactamylrest, einen monocyclischen 4- bis 6-gliedrigen, von einer $\omega$-Hydroxy-($C_3$-$C_5$)-niederalkancarbonsäure oder $\omega$-Hydroxy-($C_4$-$C_5$)-niederalkencarbonsäure abgeleiteten Lactonylrest oder einen 5- oder 6-gliedrigen, von einer $\omega$-Hydroxy-($C_4$-$C_5$)-niederalkencarbonsäure abgeleiteten Benzolactonylrest darstellt, welcher Rest unsubstituiert oder durch Hydroxy, Niederalkoxy, Niederalkanoyloxy, Halogen, Mercapto, Niederalkylthio, Niederalkyl, durch Hydroxy, Halogen, Niederalkanoyloxy, Amino, Niederalkylamino, Diniederalkylamino, Niederalkanoylamino, Carboxy, Niederalkoxycarbonyl, Cyano, Carbamoyl oder Carbamoyloxy substituiertes Niederalkyl, Niederalkenyl, Amino, Niederalkylamino, Diniederalkylamino, Niederalknoylamino, Carboxyl, Niederalkoxycarbonyl, Cyano und/oder gegebenenfalls durch Niederalkyl, Hydroxy, Niederalkoxy, Halogen und/oder Nitro substituiertes Phenyl substituiert ist, optische Isomere von solchen Verbindungen der Formel I, welche im Rest $R_1$, $R_3$ und/oder A zusätzliche Chiralitätszentren aufweisen, Mischungen dieser optischen Isomere und Salze von Verbindungen der Formel I, die eine salzbildende Gruppe enthalten.

3. Verbindungen der Formel I gemäss Anspruch 1, worin $R_1$ durch Hydroxy substituiertes Niederalkyl ist, $R_2$ Carboxyl oder unter physiologischen Bedingungen spaltbares verestertes Carboxyl bedeutet, A Niederalkylen ist und $R_3$ einen Rest der Formel

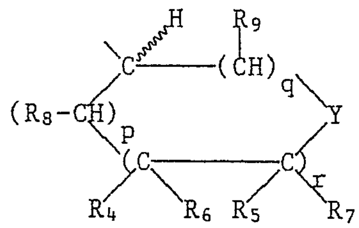

(II),

darstellt, worin Y für die Gruppe $-NR_{10}-C(=O)$-steht und $R_{10}$ Wasserstoff, Niederalkenyl, oder gegebenenfalls durch Amino, Hydroxy, Carboxy oder Cyano substituiertes Niederalkyl bedeutet, $R_4$ und $R_5$ für Wasserstoff stehen oder gemeinsam eine Bindung darstellen, $R_6$ und $R_7$ unabhängig voneinander für Wasserstoff, Niederalkyl, durch Hydroxy, Halogen, Niederalkoxy, Amino, Niederalkylamino, Diniederalkylamino, Niederalkanoylamino oder Carbamoyloxy substituiertes Niederalkyl oder gegebenenfalls durch Niederalkyl, Hydroxy, Niederalkoxy und/oder Halogen substituiertes Phenyl stehen, $R_8$ und $R_9$ unabhängig voneinander für Wasserstoff oder Niederalkyl stehen, r 0 oder 1 ist und p und q unabhängig voneinander für eine ganze Zahl von 0 bis 3 stehen, mit der Massgabe, dass wenn r 0 ist, die Summe von p und q 1, 2 oder 3 ist, oder wenn r 1 ist, die Summe von p und q 0 oder 1 ist, und mit der weiteren Massgabe, dass das dem Lactam-Ringstickstoffatom benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optische Isomere von solchen Verbindungen der Formel I, welche im Reste $R_1$, $R_3$ und/oder A zusätzliche Chiralitätszentren aufweisen, Mischungen dieser optischen Isomere und Salze von Verbindungen der Formel I, die eine salzbildende Gruppe enthalten.

4. Verbindungen der Formel I gemäss Anspruch 1, worin $R_1$ durch Hydroxy substituiertes Niederalkyl ist, $R_2$ Carboxyl oder unter physiologischen Bedingungen spaltbares verestertes Carboxyl bedeutet, A Niederalkylen ist und $R_3$ einen Rest der Formel

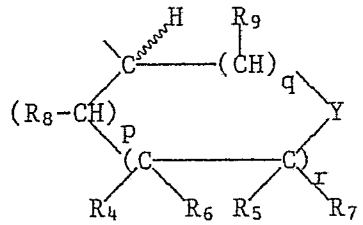 (II),

darstellt, worin Y für die Gruppe -O-C(=O)- steht, $R_4$ und $R_5$ Wasserstoff bedeuten oder gemeinsam eine Bindung darstellen, $R_6$ und $R_7$ unabhängig voneinander für Wasserstoff, Niederalkyl, durch Hydroxy, Halogen, Niederalkanoyloxy, Amino, Niederalkylamino, Diniederalkylamino, Niederalkanoylamino oder Carbamoyloxy substituiertes Niederalkyl, Hydroxy, Halogen, Amino, Niederalkylamino, Diniederalkylamino, Niederalkanoylamino oder gegebenenfalls durch Niederalkyl, Hydroxy, Niederalkoxy und/oder Halogen substituiertes Phenyl stehen oder $R_4$ und $R_5$ gemeinsam eine Bindung darstellen und $R_6$ und $R_7$ gemeinsam für einen gegebenenfalls durch Hydroxy, Niederalkoxy, Niederalkyl und/oder Halogen substituierten 1,4-Butadien-1,4-ylen-Rest stehen, $R_8$ und $R_9$ unabhängig voneinander Wasserstoff, Niederalkyl, Amino oder Hydroxy bedeuten, r 0 ist, die Summe von p und q 2 oder 3 ist, oder r 1, q 0 und p 0 oder 1 ist, mit der Massgabe, dass das dem Lacton-Ringsauerstoff benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optische Isomere von solchen Verbindungen der Formel I, welche im Reste $R_1$, $R_3$ und/oder A zusätzliche Chiralitätszentren aufweisen, Mischungen solcher optischen Isomere und Salze von Verbindungen der Formel I, die eine salzbildende Gruppe enthalten.

5. Verbindungen der Formel I gemäss Anspruch 1, worin $R_1$ in $\alpha$-Stellung durch Hydroxy substituiertes Niederalkyl ist, $R_2$ Carboxyl oder unter physiologischen Bedingungen spaltbares verestertes Carboxy bedeutet, A Niederalkylen ist, und $R_3$ einen Rest der Formel II darstellt, worin Y die Gruppe $-NR_{10}$ -C(=O)- ist und $R_{10}$ Wasserstoff oder Niederalkyl bedeutet, $R_8$ und $R_9$ Wasserstoff bedeuten, r 0 ist, p eine ganze Zahl von 1 bis 3 ist und q 0 oder 1 ist, mit der Massgabe, dass die Summe von p und q höchstens 3 beträgt, optische Isomere von solchen Verbindungen der Formel I, welche im Rest $R_1$, $R_3$ und/oder A zusätzliche Chiralitätszentren aufweisen, Mischungen dieser optischen Isomere und Salze von Verbindungen der Formel I, die eine salzbildende Gruppe enthalten.

6. Verbindungen der Formel I gemäss Anspruch 1, worin $R_1$ in $\alpha$-Stellung durch Hydroxy substituiertes Niederalkyl ist, $R_2$ Carboxyl oder unter physiologischen Bedingungen spaltbares verestertes Carboxyl bedeutet, A Niederalkylen ist, und $R_3$ einen Rest der Formel II darstellt, worin Y eine Gruppe -O-C(=O)-ist, r 1 ist, p und q 0 sind, $R_4$, $R_5$, $R_6$ und $R_7$ Wasserstoff darstellen oder $R_4$ und $R_5$ gemeinsam eine Bindung darstellen und $R_6$ und $R_7$ unabhängig voneinander für Wasserstoff, Niederalkyl oder durch Halogen, Amino, Diniederalkylamino oder Niederalkanoylamino substituiertes Niederalkyl stehen, oder r 0 ist, p und q jeweils 1 sind und $R_8$ und $R_9$ für Wasserstoff stehen, optische Isomere von solchen Verbindungen der Formel I, welche im Rest $R_1$, $R_3$ und/oder A zusätzliche Chiralitätszentren aufweisen, Mischungen solcher optischen Isomere und Salze von Verbindungen der Formel I, die eine salzbildende Gruppe enthalten.

7. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet, A für Methylen oder Aethylen steht und $R_3$ 5-Oxo-tetrahydrofuran-2-yl, gegebenenfalls durch Niederalkyl substituiertes 2,5-Dihydro-5-oxo-furan-2-yl, 5-Oxo-pyrrolidin-2-yl oder 4-Oxo-azetidin-2-yl ist, optische Isomere von Verbindungen der Formel I, Mischungen dieser optischen Isomere und Salze von Verbindungen der Formel I, die eine salzbildende Gruppe aufweisen.

8. (5R,6S)-6-[(1'R)-1'-Hydroxyäthyl]-2-(5-oxo-pyrrolidin-2-yl-methyl)-2-penem-3-carbonsäure, die reinen Diastereomeren davon und Mischungen dieser Diastereomeren und pharmazeutisch annehmbare Salze davon gemäss Anspruch 1.

9. (5R,6S)-6-[(1'R)-1'-Hydroxyäthyl]-2-[(2S)-5-oxo-pyrrolidin-2-yl-methyl]-2-penem-3-carbonsäure und pharmazeutisch annehmbare Salze davon gemäss Anspruch 8.

10. (5R,6S)-6-[(1'R)-1'-Hydroxyäthyl]-2-(4-oxo-azetidin-2-ylmethyl)-2-penem-3-carbonsäure, die reinen Diastereomeren davon und Mischungen dieser Diastereomeren und pharmazeutisch annehmbare Salze davon gemäss Anspruch 1.

11. (5R,6S)-6-[(1'R)-1'-Hydroxyäthyl]-2-[(2S)-4-oxo-azetidin-2-yl-methyl]-2-penem-3-carbonsäure und pharmazeutisch annehmbare Salze davon gemäss Anspruch 10.

12. (5R,6S)-6-[(1'R)-1'-Hydroxyäthyl]-2-[(2S)-1-methyl-5-oxo-pyrrolidin-2-ylmethyl]-2-penem-3-carbonsäure, die reinen Diastereomeren davon und Mischungen dieser Diastereomeren und pharmazeutisch annehmbare Salze davon gemäss Anspruch 1.

13. (5R,6S)-6-[(1'R)-1'-Hydroxyäthyl]-2-(5-oxo-tetrahydrofuran-2-yl-methyl)-2-penem-3-carbonsäure, die reinen Diastereomeren davon und Gemische dieser Diastereomeren und pharmazeutisch annehmbare Salze davon gemäss Anspruch 1.

14. (5R,6S)-6-[(1'R)-1'-Hydroxyäthyl]-2-[(2S)-5-oxo-tetrahydrofuran-2-ylmethyl]-2-penem-3-carbonsäure und pharmazeutisch annehmbare Salze davon gemäss Anspruch 13.

15. (5R,6S)-6-[(1'R)-1'-Hydroxyäthyl]-2-[(2R)-5-oxo-tetrahydrofuran-2-ylmethyl]-2-penem-3-carbonsäure und pharmazeutisch annehmbare Salze davon gemäss Anspruch 13.

16. (5R,6S)-6-[(1'R)-1'-Hydroxyäthyl]-2-(2,5-dihydro-3-methyl-5-oxo-furan-2-ylmethyl)-2-penem-3-carbonsäure, die reinen Diastereomeren davon und Gemische dieser Diastereomeren und pharmazeutisch annehmbare Salze davon gemäss Anspruch 1.

17. (5R,6S)-6-[(1'R)-1'-Hydroxyäthyl]-2-[2-(2-oxo-tetrahydrofuran-4-yl)-äthyl]-2-penem-3-carbonsäure, die reinen Diastereomeren davon und Gemische dieser Diastereomeren und pharmazeutisch annehmbare Salze davon gemäss Anspruch 1.

18. (5R,6S)-6-[(1'R)-1'-Hydroxyäthyl]-2-[2-(5-oxo-tetrahydrofuran-2-yl)-äthyl]-2-penem-3-carbonsäure, die reinen Diastereomeren davon und Gemische dieser Diastereomeren und pharmazeutisch annehmbare Salze davon gemäss Anspruch 1.

19. (5R,6S)-6-[(1'R)-1'-Hydroxyäthyl]-2-(2,5-dihydro-5-oxo-furan-2-ylmethyl)-2-penem-3-carbonsäure, die reinen Diastereomeren davon und Gemische dieser Diastereomeren und pharmazeutisch annehmbare Salze davon gemäss Anspruch 1.

20. Pharmazeutisches Präparat enthaltend eine Verbindung der Formel I gemäss Anspruch 1 oder ein pharmazeutisch annehmbares Salz einer solchen Verbindung.

21. Verwendung von Verbindungen der Formel I gemäss Anspruch 1 oder deren pharmazeutisch annehmbaren Salzen zur Herstellung von pharmazeutischen Präparaten.

22. Eine Verbindung der Formel I gemäss Anspruch 1 oder ein pharmazeutisch annehmbares Salz einer solchen Verbindung zur Anwendung in einem Verfahren zur therapeutischen Behandlung des menschlichen oder tierischen Körpers.

23. Verfahren zur Herstellung von Verbindungen der Formel I gemäss Anspruch 1, dadurch gekennzeichnet, dass man eine Ylid-Verbindung der Formel

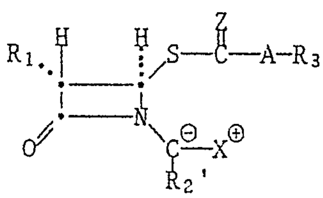

(III),

worin $R_1$, A und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

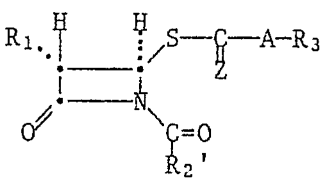

(IV),

worin $R_1$, A und $R_3$ die unter Formel I und $R_2'$ und Z die unter Formel III angegebenen Bedeutungen haben, mit einer organischen Verbindung des dreiwertigen Phosphors behandelt, und, wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine Carboxylgruppe $R_2$ in eine unter physiolo gischen Bedingungen spaltbare veresterte Carboxylgruppe überführt, und/oder, wenn erwunscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

24. Die nach dem Verfahren gemäss Anspruch 23 erhältlichen Verbindungen.

Patentansprüche für die folgenden Vertragsstaaten: AT, ES, GR

1. Verfahren zur Herstellung von Verbindungen der Formel

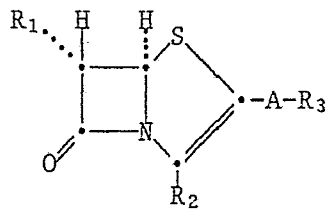

(I),

worin $R_1$ durch Hydroxy oder geschütztes Hydroxy substituiertes Niederalkyl ist, $R_2$ Carboxyl oder funktionell abgewandeltes Carboxyl bedeutet, A für Niederalkylen steht und $R_3$ einen über ein Ringkohlenstoffatom gebundenen, gegebenenfalls partiell ungesättigten Lactamylrest oder Lactonylrest darstellt, optischen Isomeren von Verbindungen der Formel I, Mischungen dieser optischen Isomere und Salzen von Verbindungen der Formel I, die einen salzbildende Gruppe aufweisen, dadurch gekennzeichnet, dass man eine Ylid-Verbindung der Formel

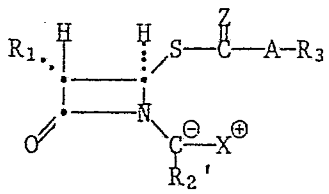

(III),

worin $R_1$, A und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

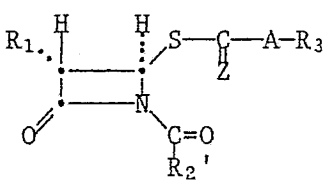

(IV),

worin $R_1$, A und $R_3$ die unter Formel I und $R_2'$ und Z die unter Formel III angegebenen Bedeutungen haben, mit einer organischen Verbindung des dreiwertigen Phosphors behandelt, und, wenn erwünscht oder notwendig, in einer erhältlichen Verbindung der Formel I eine geschützte funktionelle Gruppe in die freie funktionelle Gruppe überführt, und/oder, wenn erwünscht, in einer erhältlichen Verbindung der Formel I eine Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt, und/oder, wenn erwünscht, ein erhältliches Gemisch von isomeren Verbindungen in die einzelnen Isomere auftrennt.

2. Verfahren gemäss Anspruch 1 zur Herstellung von Verbindungen der Formel I, worin $R_1$ durch Hydroxy oder geschütztes Hydroxy substituiertes Niederalkyl ist, $R_2$ Carboxyl, unter physiologischen Bedingungen spaltbares verestertes Carboxyl oder geschütztes Carboxyl $R_2'$ darstellt, A Niederalkylen bedeutet und $R_3$ einen über ein Ringkohlenstoffatom gebundenen, monocyclischen 4- bis 6-gliedrigen, von einer $\omega$-Amino-($C_3$-$C_5$)-niederalkancarbonsäure oder $\omega$-Amino-($C_4$-$C_5$)-niederalkencarbonsäure abgeleiteten Lactamylrest, einen 5- oder 6-gliedrigen, von einer $\omega$-Amino-($C_4$-$C_5$)-niederalkencarbonsäure abgeleiteten Benzolactamylrest, einen monocyclischen 4-bis 6-gliedrigen, von einer $\omega$-Hydroxy-($C_3$-$C_5$)-niederalkancarbonsäure oder $\omega$-Hydroxy-($C_4$-$C_5$)-niederalkencarbonsäure abgeleiteten Lactonylrest oder einen 5- oder 6-gliedrigen, von einer $\omega$-Hydroxy-($C_4$-$C_5$)-niederalkencarbonsäure abgeleiteten Benzolactonylrest darstellt, welcher Rest unsubstituiert oder durch Hydroxy, Nieder alkoxy, Niederalkanoyloxy, Halogen, Mercapto, Niederalkylthio, Niederalkyl, durch Hydroxy, Halogen, Niederalkanoyloxy, Amino, Niederalkylamino, Diniederalkylamino, Niederalkanoylamino, Carboxyl, Niederalkoxycarbonyl, Cyano, Carbamoyl oder Carbamoyloxy substituiertes Niederalkyl, Niederalkenyl, Amino, Niederalkylamino, Diniederalkylamino, Niederalknoylamino, Carboxyl, Niederalkoxycarbonyl, Cyano und/oder gegebenenfalls durch Niederalkyl, Hydroxy, Niederalkoxy, Halogen und/oder Nitro substituiertes Phenyl substituiert ist, optischen Isomeren von solchen Verbindungen der Formel I, welche im Rest $R_1$, $R_3$ und/oder A zusätzliche Chiralitätszentren aufweisen, Mischungen dieser optischen Isomere und Salzen von Verbindungen der Formel I, die eine salzbildende Gruppe enthalten.

3. Verfahren gemäss Anspruch 1 zur Herstellung von Verbindungen der Formel I, worin $R_1$ durch Hydroxy substituiertes Niederalkyl ist, $R_2$ Carboxyl oder unter physiologischen Bedingungen spaltbares verestertes Carboxyl bedeutet, A Niederalkylen ist und $R_3$ einen Rest der Formel

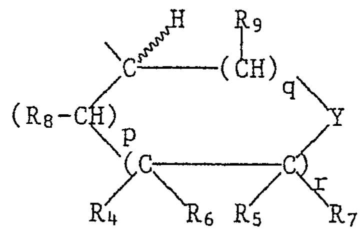

(II),

darstellt, worin Y für die Gruppe $-NR_{10}$ -C(=O)-steht und $R_{10}$ Wasserstoff, Niederalkenyl, oder gegebenenfalls durch Amino, Hydroxy, Carboxy oder Cyano substituiertes Niederalkyl bedeutet, $R_4$ und $R_5$ für Wasserstoff stehen oder gemeinsam eine Bindung darstellen, $R_6$ und $R_7$ unabhängig voneinander für Wasserstoff, Niederalkyl, durch Hydroxy, Halogen, Niederalkoxy, Amino, Niederalkylamino, Diniederalkylamino, Niederalkanoylamino oder Carbamoyloxy substituiertes Niederalkyl oder gegebenenfalls durch Niederalkyl, Hydroxy, Niederalkoxy und/oder Halogen substituiertes Phenyl stehen, $R_8$ und $R_9$ unabhängig voneinander für Wasserstoff oder Niederalkyl stehen, r 0 oder 1 ist und p und q unabhängig voneinander für eine ganze Zahl von 0 bis 3 stehen, mit der Massgabe, dass wenn r 0 ist, die Summe von p und q 1, 2 oder 3 ist, oder wenn r 1 ist, die Summe von p und q 0 oder 1 ist, und mit der weiteren Massgabe, dass das dem Lactam-Ringstickstoffatom benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optischen Isomeren von solchen Verbindungen der Formel I, welche im Reste $R_1$, $R_3$ und/oder A zusätzliche Chiralitätszentren aufweisen, Mischungen dieser optischen Isomere und Salzen von Verbindungen der Formel I, die eine salzbildende Gruppe enthalten.

4. Verfahren gemäss Anspruch 1 zur Herstellung von Verbindungen der Formel I, worin $R_1$ durch Hydroxy substituiertes Niederalkyl ist, $R_2$ Carboxyl oder unter physiologischen Bedingungen spaltbares verestertes Carboxyl bedeutet, A Niederalkylen ist und $R_3$ einen Rest der Formel

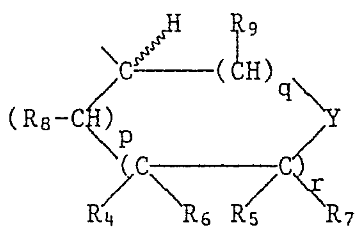

(II),

darstellt, worin Y für die Gruppe -O-C(=O)- steht, $R_4$ und $R_5$ Wasserstoff bedeuten oder gemeinsam eine Bindung darstellen, $R_6$ und $R_7$ unabhängig voneinander für Wasserstoff, Niederalkyl, durch Hydroxy, Halogen, Niederalkanoyloxy, Amino, Niederalkylamino, Diniederalkylamino, Niederalkanoylamino oder Carbamoyloxy substituiertes Niederalkyl, Hydroxy, Halogen, Amino, Niederalkylamino, Diniederalkylamino, Niederalkanoylamino oder gegebenenfalls durch Niederalkyl, Hydroxy, Niederalkoxy und/oder Halogen substituiertes Phenyl stehen oder $R_4$ und $R_5$ gemeinsam eine Bindung darstellen und $R_6$ und $R_7$ gemeinsam für einen gegebenenfalls durch Hydroxy, Niederalkoxy, Niederalkyl und/oder Halogen substituierten 1,4-Butadien-1,4-ylen-Rest stehen, $R_8$ und $R_9$ unabhängig voneinander Wasserstoff, Niederalkyl, Amino oder Hydroxy bedeuten, r 0 ist, die Summe von p und q 2 oder 3 ist, oder r 1, q 0 und p 0 oder 1 ist, mit der Massgabe, dass das dem Lacton-Ringsauerstoff benachbarte Kohlenstoffatom in den Resten der Formel II keinen Substituenten aus der Gruppe Hydroxy, Halogen, Amino, Niederalkylamino, Diniederalkylamino oder Niederalkanoylamino trägt, optischen Isomeren von solchen Verbindungen der Formel I, welche im Reste $R_1$, $R_3$ und/oder A zusätzliche Chiralitätszentren aufweisen, Mischungen solcher optischen Isomere und Salzen von Verbindungen der Formel I, die eine salzbildende Gruppe enthalten.

5. Verfahren gemäss Anspruch 1 zur Herstellung von Verbindungen der Formel I, worin $R_1$ in $\alpha$-Stellung durch Hydroxy substituiertes Niederalkyl ist, $R_2$ Carboxyl oder unter physiologischen Bedingungen spaltbares verestertes Carboxyl bedeutet, A Niederalkylen ist, und $R_3$ einen Rest der Formel II darstellt, worin Y die Gruppe $-NR_{10}$ -C(=O)- ist und $R_{10}$ Wasserstoff oder Niederalkyl bedeutet, $R_8$ und $R_9$ Wasserstoff bedeuten, r 0 ist, p eine ganze Zahl von 1 bis 3 ist und q 0 oder 1 ist, mit der Massgabe, dass die Summe von p und q höchstens 3 beträgt, optischen Isomeren von solchen Verbindungen der Formel I, welche im Rest $R_1$, $R_3$ und/oder A zusätzliche Chiralitätszentren aufweisen, Mischungen dieser optischen Isomere und Salzen von Verbindungen der Formel I, die eine salzbildende Gruppe enthalten.

6. Verfahren gemäss Anspruch 1 zur Herstellung von Verbindungen der Formel I, worin $R_1$ in $\alpha$-Stellung durch Hydroxy substituiertes Niederalkyl ist, $R_2$ Carboxyl oder unter physiologischen Bedingungen spaltbares verestertes Carboxyl bedeutet, A Niederalkylen ist, und $R_3$ einen Rest der Formel II darstellt, worin Y eine Gruppe -O-C(=O)-ist, r 1 ist, p und q 0 sind, $R_4$, $R_5$, $R_6$ und $R_7$ Wasserstoff darstellen oder $R_4$ und $R_5$ gemeinsam eine Bindung darstellen und $R_6$ und $R_7$ unabhängig voneinander für Wasserstoff, Niederalkyl oder durch Halogen, Amino, Diniederalkylamino oder Niederalkanoylamino substituiertes Niederalkyl stehen, oder r 0 ist, p und q jeweils 1 sind und $R_8$ und $R_9$ für Wasserstoff stehen, optischen Isomeren von solchen Verbindungen der Formel I, welche im Rest $R_1$, $R_3$ und/oder A zusätzliche Chiralitätszentren aufweisen, Mischungen solcher optischen Isomere und Salzen von Verbindungen der Formel I, die eine salzbildende Gruppe enthalten.

7. Verfahren gemäss Anspruch 1 zur Herstellung von Verbindung der Formel I, worin $R_1$ Hydroxymethyl oder 1-Hydroxyäthyl ist, $R_2$ Carboxyl oder unter physiologischen Bedingungen spaltbares verestertes Carboxyl bedeutet, A für Methylen oder Aethylen steht und $R_3$ 5-Oxo-tetrahydrofuran-2-yl, gegebenenfalls durch Niederalkyl substituiertes 2,5-Dihydro-5-oxo-furan-2-yl, 5-Oxo-pyrrolidin-2-yl oder 4-Oxo-azetidin-2-yl ist, optischen Isomeren von Verbindungen der Formel I, Mischungen dieser optischen Isomere und Salzen von Verbindungen der Formel I, die eine salzbildende Gruppe aufweisen.

8. Verfahren gemäss Anspruch 1 zur Herstellung von (5R,6S)-6-[(1'R)-1'-Hydroxyäthyl]-2-(5-oxo-pyrrolidin-2-ylmethyl)-2-penem-3-carbonsäure, der reinen Diastereomeren davon und Mischungen dieser Diastereomeren und pharmazeutisch annehmbaren Salzen davon.

9. Verfahren gemäss Anspruch 8 zur Herstellung von (5R,6S)-6-[(1'R)-1'-Hydroxyäthyl]-2-[(2S)-5-oxo-pyrrolidin-2-ylmethyl]-2-penem-3-carbonsäure und pharmazeutisch annehmbaren Salzen davon.

10. Verfahren gemäss Anspruch 1 zur Herstellung von (5R,6S)-6-[(1'R)-1'-Hydroxyäthyl]-2-(4-oxo-azetidin-2-ylmethyl)2-penem-3-carbonsäure, der reinen Diastereomeren davon und Mischungen dieser Diastereomeren und pharmazeutisch annehmbaren Salzen davon.

11. Verfahren gemäss Anspruch 10 zur Herstellung von (5R,6S)-6-[(1'R)-1'-Hydroxyäthyl]-2-[(2S)-4-oxo-azetidin-2-ylmethyl]-2-penem-3-carbonsäure und pharmazeutisch annehmbaren Salzen davon.

12. Verfahren gemäss Anspruch 1 zur Herstellung von (5R,6S)-6-[(1'R)-1'-Hydroxyäthyl]-2-[(2S)-1-methyl-5-oxo-pyrrolidin-2-yl-methyl]-2-penem-3-carbonsäure, der reinen Diastereomeren davon und Mischungen dieser Diastereomeren und pharmazeutisch annehmbaren Salzen davon.

13. Verfahren gemäss Anspruch 1 zur Herstellung von (5R,6S)-6-[(1'R)-1'-Hydroxyäthyl]-2-(5-oxo-tetrahydrofuran-2-ylmethyl)-2-penem-3-carbonsäure, der reinen Diastereomeren davon und Gemische dieser Diastereomeren und pharmazeutisch annehmbaren Salzen davon.

14. Verfahren gemäss Anspruch 13 zur Herstellung von (5R,6S)-6-[(1'R)-1'-Hydroxyäthyl]-2-[(2S)-5-oxo-tetrahydrofuran-2-ylmethyl]-2-penem-3-carbonsäure und pharmazeutisch annehmbaren Salzen davon.

15. Verfahren gemäss Anspruch 13 zur Herstellung von (5R,6S)-6-[(1'R)-1'-Hydroxyäthyl]-2-[(2R)-5-oxo-tetrahydrofuran-2-ylmethyl]-2-penem-3-carbonsäure und pharmazeutisch annehmbaren Salzen davon.

16. Verfahren gemäss Anspruch 1 zur Herstellung von (5R,6S)-6-[(1'R)-1'-Hydroxyäthyl]-2-(2,5-dihydro-3-methyl-5-oxo-furan-2-yl-methyl)-2-penem-3-carbonsäure, der reinen Diastereomeren davon und Gemische dieser Diastereomeren und pharmazeutisch annehmbaren Salze davon.

17. Verfahren gemäss Anspruch 1 zur Herstellung von (5R,6S)-6-[(1'R)-1'-Hydroxyäthyl]-2-[2-(2-oxo-tetrahydrofuran-4-yl)-äthyl]-2-penem-3-carbonsäure, der reinen Diastereomeren davon und Gemische dieser Diastereomeren und pharmazeutisch annehmbaren Salzen davon.

18. Verfahren gemäss Anspruch 1 zur Herstellung von (5R,6S)-6-[(1'R)-1'-Hydroxyäthyl]-2-[2-(5-oxo-tetrahydrofuran-2-yl)-äthyl]-2-penem-3-carbonsäure, der reinen Diastereomeren davon und Gemische dieser Diastereomeren und pharmazeutisch annehmbaren Salzen davon.

19. Verfahren gemäss Anspruch 1 zur Herstellung von (5R,6S)-6-[(1'R)-1'-Hydroxyäthyl]-2-(2,5-dihydro-5-oxo-furan-2-ylmethyl)-2-penem-3-carbonsäure, der reinen Diastereomeren davon und Gemische dieser Diastereomeren und pharmazeutisch annehmbaren Salzen davon.

20. Verfahren zur Herstellung von pharmazeutischen Präparaten, dadurch gekennzeichnet, dass man eine nach dem Verfahren gemäss Anspruch 1 erhältliche Verbindung der Formel I oder ein pharmazeutisch annehmbares Salz einer solchen Verbindung mit einem pharmazeutisch verwendbaren Trägermaterial mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 87 81 0386

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 138 539 (PFIZER)<br>* Ansprüche *<br>--- | 1,20 | C 07 D 499/00<br>A 61 K 31/43//<br>C 07 F 9/65<br>C 07 F 7/18<br>C 07 D 205/08<br>C 07 D 403/12 |
| A | EP-A-0 002 210 (MERCK)<br>* Ansprüche *<br>--- | 1,20, 23 | |
| P,A | CHEMICAL ABSTRACTS, Band 106, Nr. 13, 30. März 1987, Seite 625, Ref. Nr. 101960a; Columbus, Ohio, US & JP-A-61 207 387 (SUNTORY LTD) 13-09-1986.<br>* Zusammenfassung *<br>--------------- | 1,20, 23 | |

RECHERCHIERTE SACHGEBIETE (Int. Cl.4)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-21,23,24

Unvollständig recherchierte Patentansprüche:

Nicht recherchierte Patentansprüche: 22

Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 15-09-1987 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument